# EUROPEAN PATENT SPECIFICATION

(11) **EP 3 354 728 B1**
(45) Date of publication and mention of the grant of the patent: **22.04.2020**
(21) Application number: 18160297.0
(22) Date of filing: 11.12.2013
(51) Int. Cl.: C12N 9/28, C11D 3/386

(54) **ALPHA-AMYLASE VARIANTS**
ALPHA-AMYLASE-VARIANTEN
VARIANTES D'ALPHA-AMYLASE

(30) Priority: 21.12.2012 US 201261740852 P
(43) Date of publication of application: 01.08.2018
(62) Divisional of application: 13814346.6
(73) Proprietor: Danisco US Inc., Palo Alto, CA 94304 (US)
(72) Inventor: CASCAO-PEREIRA, Luis G., Palo Alto, CA 94304 (US); ESTELL, David A., Palo Alto, CA 94304 (US); FINAN, Dina, Palo Alto, CA 94304 (US); KOLMAN, Marc, Palo Alto, CA 94304 (US); RAMER, Sandra W., Palo Alto, CA 94304 (US)
(74) Representative: Mewburn Ellis LLP

(56) References cited:
- WO-A1-2015/077126
- WO-A2-00/60058
- WO-A2-2010/115021
- DATABASE UniProt [Online] 2 November 2010 (2010-11-02), "SubName: Full=Glucan 1,4-alpha-maltohexaosidase; EC=3.2.1.98; Flags: Precursor;", XP002721592, retrieved from EBI accession no. UNIPROT:E0I4J1 Database accession no. E0I4J1
- DATABASE UniProt [Online] 13 June 2012 (2012-06-13), "SubName: Full=Cytochrome C oxidase subunit II;", XP002721593, retrieved from EBI accession no. UNIPROT:I0BPU4 Database accession no. I0BPU4
- KEIJI TSUSAKI ET AL: "Purification and characterization of highly branched alpha -glucan-producing enzymes from Paenibacillus sp. PP710.", BIOSCIENCE, BIOTECHNOLOGY, AND BIOCHEMISTRY, vol. 76, no. 4, 7 April 2012 (2012-04-07), pages 721-731, XP055106811, ISSN: 0916-8451
- ZENG Li-juan,YANG Jian,CHEN Ying,WANG Qing-yan,HUANG Ri-bo: "solation of raw starch-digesting amylase producing strain Paenibacillus sp. and its purification and characterization", Chinese Journal of Bioprocess Engineering , vol. 8, no. 4 2010, pages 62-68, XP002721596, Retrieved from the Internet: URL:http://lib.cqvip.com/qk/87046X/20104/3 4667285.html [retrieved on 2014-03-11]
- Ikram-Ul-Haq ET AL: "SOLID STATE FERMENTATION FOR THE PRODUCTION OF [Alpha]-AMYLASE BY PAENIBACILLUS AMYLOLYTICUS", Pak. J. Bot., 1 March 2012 (2012-03-01), pages 341-346, XP055106853, Retrieved from the Internet: URL:http://www.pakbs.org/pjbot/PDFs/44(SI1 )/51.pdf [retrieved on 2014-03-11]
- P. PASON ET AL: "Paenibacillus curdlanolyticus Strain B-6 Xylanolytic-Cellulolytic Enzyme System That Degrades Insoluble Polysaccharides", APPLIED AND ENVIRONMENTAL MICROBIOLOGY, vol. 72, no. 4, 1 April 2006 (2006-04-01), pages 2483-2490, XP055106867, ISSN: 0099-2240, DOI: 10.1128/AEM.72.4.2483-2490.2006

### SEQUENCE LISTING

<110> DANISCO US INC.
<120> ALPHA-AMYLASE VARIANTS
<130> GRF/FP7346018
<140> Divisional of EP 13814346.6
   <141> 2013-12-11
<150> EP 13814346.6
   <151> 2013-12-11
<150> PCT/US2013/074282
   <151> 2013-12-11
<150> US 61/740,852
   <151> 2012-12-21
<160> 5
<170> PatentIn version 3.5
<210> 1
   <211> 1512
   <212> DNA
   <213> Artificial Sequence
<220>
   <223> Synthetic: codon-modified nucleic acid sequence encoding the mature form of PcuAmy1-v1 fused to a sequence encoding the Bacillus licheniformis Epr signal peptide
<400> 1
   atgaaaaaac tgtggaaaat cgcggtcagc gcagccatgt ttgtcggctt ttttgcgaac      60
   agcccgagag ctagcgcagc cgacaacggc acaatcatgc agtatttcga gtggtacctg     120
   ccgaacgacg gagcgcactg gaacagactt aataacgacg cacaaaacct gaaaaatgtg     180
   ggcatcacgg cagtgtggat tcctccggca tacaagggcg gcagctcagc agatgttggc     240
   tacggagttt acgatacata cgacctgggc gagttcaatc agaaaggcac ggtcagaaca     300
   aagtacggaa cgaagagcga actgatttca gcggtcaaca atcttcacgc aaagggcatt     360
   gcggtttacg gcgacgtggt cctgaaccat agaatgaatg cggatgcaac ggagcttgtg     420
   gatgcggttg aggtggatcc gaacaacaga aacgtcgaga cgacaagcac gtatcagatc     480
   caggcatgga cgcaatacga tttcccgggc agaggcaaca cgtacagcag ctttaaatgg     540
   agatggtatc acttcgacgg cgtcgactgg gaccagagca gaggcctgaa cagaatctat     600
   aagctggatg gcaaggattg ggactgggag gtcgacagcg agtacggcaa ctacgattac     660
   ctgatgggag cggacctgga cttcaaccac ccggatgtgg ttaacgaaac aaagacatgg     720
   ggcaaatggt ttgtgaacac ggtgaacctg gatggcgtca gactggacgc ggttaagcac     780
   atcaagttcg acttcatgag agactgggtg aacaacgtga gaagcacgac gggcaagaac     840
   cttttcgcag ttggcgagta ttggcactac gacgtgaaca aactgaacag ctacatcacg     900
   aagacgaatg gcacgatgag cctgttcgac gtgccgctgc actttagatt ttatgatgca     960
   agcaacggcg gaggcggcta cgacatgaga aacctgctga ataacacgct gatgagcagc    1020
   aacccgatga aggcggttac attcgttgag aaccatgaca cacaaccgac gcaggccctg    1080
   caatcaacgg tccaaagctg gtttaagccg cttgcgtatg ctacaatcct gacgagagag    1140
   caaggctacc cgtgcgtttt ctacggcgac tattatggaa caagcgacgg caaaattagc    1200
   agctacaagc cgatcatgga taaactgctt aacgcgagaa aggtgtacgc ctacggcacg    1260
   cagagagatt acttcgatca tccggacatc gttggctgga caagagaagg cgatgcagca    1320
   catgctggct caggactggc aacgcttatc acagatggcc ctggcggaag caagtggatg    1380
   tatgttggaa cgtcaaaggc aggccaggtc tggacggata aaacaggaaa cagaagcgga    1440
   acggtgacga ttgatgccaa tggctgggga aacttttggg ttaatggcgg atcagttagc    1500
   gtttgggcaa aa                                                        1512
<210> 2
   <211> 506
   <212> PRT
   <213> Artificial Sequence
<220>
   <223> Synthetic: precursor form of PcuAmy1 alpha-amylase, which includes the B. licheniformis Epr signal peptide
<400> 2
   Met Lys Lys Leu Trp Lys Ile Ala Val Ser Ala Ala Met Phe Val Gly
   1               5                   10                  15
   Phe Phe Ala Asn Ser Pro Arg Ala Ser Ala Ala Asp Asn Gly Thr Ile
               20                  25                  30
   Met Gln Tyr Phe Glu Trp Tyr Leu Pro Asn Asp Gly Ala His Trp Asn
           35                  40                  45
   Arg Leu Asn Asn Asp Ala Gln Asn Leu Lys Asn Val Gly Ile Thr Ala
       50                  55                  60
   Val Trp Ile Pro Pro Ala Tyr Lys Gly Gly Ser Ser Ala Asp Val Gly
   65                  70                  75                  80
   Tyr Gly Val Tyr Asp Thr Tyr Asp Leu Gly Glu Phe Asn Gln Lys Gly
                   85                  90                  95
   Thr Val Arg Thr Lys Tyr Gly Thr Lys Ser Glu Leu Ile Ser Ala Val
               100                 105                 110
   Asn Asn Leu His Ala Lys Gly Ile Ala Val Tyr Gly Asp Val Val Leu
   115 120 125
   Asn His Arg Met Asn Ala Asp Ala Thr Glu Leu Val Asp Ala Val Glu
   130 135 140
   Val Asp Pro Asn Asn Arg Asn Val Glu Thr Thr Ser Thr Tyr Gln Ile
   145 150 155 160
   Gln Ala Trp Thr Gln Tyr Asp Phe Pro Gly Arg Gly Asn Thr Tyr Ser
   165 170 175
   Ser Phe Lys Trp Arg Trp Tyr His Phe Asp Gly Val Asp Trp Asp Gln
   180 185 190
   Ser Arg Gly Leu Asn Arg Ile Tyr Lys Leu Arg Gly Asp Gly Lys Asp
   195 200 205
   Trp Asp Trp Glu Val Asp Ser Glu Tyr Gly Asn Tyr Asp Tyr Leu Met
   210 215 220
   Gly Ala Asp Leu Asp Phe Asn His Pro Asp Val Val Asn Glu Thr Lys
   225 230 235 240
   Thr Trp Gly Lys Trp Phe Val Asn Thr Val Asn Leu Asp Gly Val Arg
   245 250 255
   Leu Asp Ala Val Lys His Ile Lys Phe Asp Phe Met Arg Asp Trp Val
   260 265 270
   Asn Asn Val Arg Ser Thr Thr Gly Lys Asn Leu Phe Ala Val Gly Glu
   275 280 285
   Tyr Trp His Tyr Asp Val Asn Lys Leu Asn Ser Tyr Ile Thr Lys Thr
   290 295 300
   Asn Gly Thr Met Ser Leu Phe Asp Val Pro Leu His Phe Arg Phe Tyr
   305 310 315 320
   Asp Ala Ser Asn Gly Gly Gly Gly Tyr Asp Met Arg Asn Leu Leu Asn
   325 330 335
   Asn Thr Leu Met Ser Ser Asn Pro Met Lys Ala Val Thr Phe Val Glu
   340 345 350
   Asn His Asp Thr Gln Pro Thr Gln Ala Leu Gln Ser Thr Val Gln Ser
   355 360 365
   Trp Phe Lys Pro Leu Ala Tyr Ala Thr Ile Leu Thr Arg Glu Gln Gly
       370                 375                 380
   Tyr Pro Cys Val Phe Tyr Gly Asp Tyr Tyr Gly Thr Ser Asp Gly Lys
   385                 390                 395                 400
   Ile Ser Ser Tyr Lys Pro Ile Met Asp Lys Leu Leu Asn Ala Arg Lys
                   405                 410                 415
   Val Tyr Ala Tyr Gly Thr Gln Arg Asp Tyr Phe Asp His Pro Asp Ile
               420                 425                 430
   Val Gly Trp Thr Arg Glu Gly Asp Ala Ala His Ala Gly Ser Gly Leu
           435                 440                 445
   Ala Thr Leu Ile Thr Asp Gly Pro Gly Gly Ser Lys Trp Met Tyr Val
       450                 455                 460
   Gly Thr Ser Lys Ala Gly Gln Val Trp Thr Asp Lys Thr Gly Asn Arg
   465                 470                 475                 480
   Ser Gly Thr Val Thr Ile Asp Ala Asn Gly Trp Gly Asn Phe Trp Val
                   485                 490                 495
   Asn Gly Gly Ser Val Ser Val Trp Ala Lys
               500                 505
<210> 3
   <211> 480
   <212> PRT
   <213> Paenibacillus curdlanolyticus
<220>
   <221> misc_feature
   <223> mature form of PcuAmy1 alpha-amylase
<400> 3
   Ala Asp Asn Gly Thr Ile Met Gln Tyr Phe Glu Trp Tyr Leu Pro Asn
   1               5                   10                  15
   Asp Gly Ala His Trp Asn Arg Leu Asn Asn Asp Ala Gln Asn Leu Lys
               20                  25                  30
   Asn Val Gly Ile Thr Ala Val Trp Ile Pro Pro Ala Tyr Lys Gly Gly
           35                  40                  45
   Ser Ser Ala Asp Val Gly Tyr Gly Val Tyr Asp Thr Tyr Asp Leu Gly
       50                  55                  60
   Glu Phe Asn Gln Lys Gly Thr Val Arg Thr Lys Tyr Gly Thr Lys Ser
   65                  70                  75                  80
   Glu Leu Ile Ser Ala Val Asn Asn Leu His Ala Lys Gly Ile Ala Val
                   85                  90                  95
   Tyr Gly Asp Val Val Leu Asn His Arg Met Asn Ala Asp Ala Thr Glu
               100                 105                 110
   Leu Val Asp Ala Val Glu Val Asp Pro Asn Asn Arg Asn Val Glu Thr
           115                 120                 125
   Thr Ser Thr Tyr Gln Ile Gln Ala Trp Thr Gln Tyr Asp Phe Pro Gly
       130                 135                 140
   Arg Gly Asn Thr Tyr Ser Ser Phe Lys Trp Arg Trp Tyr His Phe Asp
   145                 150                 155                 160
   Gly Val Asp Trp Asp Gln Ser Arg Gly Leu Asn Arg Ile Tyr Lys Leu
                   165                 170                 175
   Arg Gly Asp Gly Lys Asp Trp Asp Trp Glu Val Asp Ser Glu Tyr Gly
               180                 185                 190
   Asn Tyr Asp Tyr Leu Met Gly Ala Asp Leu Asp Phe Asn His Pro Asp
           195                 200                 205
   Val Val Asn Glu Thr Lys Thr Trp Gly Lys Trp Phe Val Asn Thr Val
       210                 215                 220
   Asn Leu Asp Gly Val Arg Leu Asp Ala Val Lys His Ile Lys Phe Asp
   225                 230                 235                 240
   Phe Met Arg Asp Trp Val Asn Asn Val Arg Ser Thr Thr Gly Lys Asn
                   245                 250                 255
   Leu Phe Ala Val Gly Glu Tyr Trp His Tyr Asp Val Asn Lys Leu Asn
               260                 265                 270
   Ser Tyr Ile Thr Lys Thr Asn Gly Thr Met Ser Leu Phe Asp Val Pro
           275                 280                 285
   Leu His Phe Arg Phe Tyr Asp Ala Ser Asn Gly Gly Gly Gly Tyr Asp
       290                 295                 300
   Met Arg Asn Leu Leu Asn Asn Thr Leu Met Ser Ser Asn Pro Met Lys
   305                 310                 315                 320
   Ala Val Thr Phe Val Glu Asn His Asp Thr Gln Pro Thr Gln Ala Leu
                   325                 330                 335
   Gln Ser Thr Val Gln Ser Trp Phe Lys Pro Leu Ala Tyr Ala Thr Ile
               340                 345                 350
   Leu Thr Arg Glu Gln Gly Tyr Pro Cys Val Phe Tyr Gly Asp Tyr Tyr
           355                 360                 365
   Gly Thr Ser Asp Gly Lys Ile Ser Ser Tyr Lys Pro Ile Met Asp Lys
       370                 375                 380
   Leu Leu Asn Ala Arg Lys Val Tyr Ala Tyr Gly Thr Gln Arg Asp Tyr
   385                 390                 395                 400
   Phe Asp His Pro Asp Ile Val Gly Trp Thr Arg Glu Gly Asp Ala Ala
                   405                 410                 415
   His Ala Gly Ser Gly Leu Ala Thr Leu Ile Thr Asp Gly Pro Gly Gly
               420                 425                 430
   Ser Lys Trp Met Tyr Val Gly Thr Ser Lys Ala Gly Gln Val Trp Thr
           435                 440                 445
   Asp Lys Thr Gly Asn Arg Ser Gly Thr Val Thr Ile Asp Ala Asn Gly
       450                 455                 460
   Trp Gly Asn Phe Trp Val Asn Gly Gly Ser Val Ser Val Trp Ala Lys
   465                 470                 475                 480
<210> 4
   <211> 478
   <212> PRT
   <213> Artificial Sequence
<220>
   <223> Synthetic: mature form of variant PcuAmy1 amylase (PcuAmyl-vl) having deletions of residues Arg-177 and Gly-178
<400> 4
   Ala Asp Asn Gly Thr Ile Met Gln Tyr Phe Glu Trp Tyr Leu Pro Asn
   1               5                   10                  15
   Asp Gly Ala His Trp Asn Arg Leu Asn Asn Asp Ala Gln Asn Leu Lys
               20                  25                  30
   Asn Val Gly Ile Thr Ala Val Trp Ile Pro Pro Ala Tyr Lys Gly Gly
           35                  40                  45
   Ser Ser Ala Asp Val Gly Tyr Gly Val Tyr Asp Thr Tyr Asp Leu Gly
       50                  55                  60
   Glu Phe Asn Gln Lys Gly Thr Val Arg Thr Lys Tyr Gly Thr Lys Ser
   65                  70                  75                  80
   Glu Leu Ile Ser Ala Val Asn Asn Leu His Ala Lys Gly Ile Ala Val
                   85                  90                  95
   Tyr Gly Asp Val Val Leu Asn His Arg Met Asn Ala Asp Ala Thr Glu
               100                 105                 110
   Leu Val Asp Ala Val Glu Val Asp Pro Asn Asn Arg Asn Val Glu Thr
           115                 120                 125
   Thr Ser Thr Tyr Gln Ile Gln Ala Trp Thr Gln Tyr Asp Phe Pro Gly
       130                 135                 140
   Arg Gly Asn Thr Tyr Ser Ser Phe Lys Trp Arg Trp Tyr His Phe Asp
   145                 150                 155                 160
   Gly Val Asp Trp Asp Gln Ser Arg Gly Leu Asn Arg Ile Tyr Lys Leu
                   165                 170                 175
   Asp Gly Lys Asp Trp Asp Trp Glu Val Asp Ser Glu Tyr Gly Asn Tyr
               180                 185                 190
   Asp Tyr Leu Met Gly Ala Asp Leu Asp Phe Asn His Pro Asp Val Val
           195                 200                 205
   Asn Glu Thr Lys Thr Trp Gly Lys Trp Phe Val Asn Thr Val Asn Leu
       210                 215                 220
   Asp Gly Val Arg Leu Asp Ala Val Lys His Ile Lys Phe Asp Phe Met
   225                 230                 235                 240
   Arg Asp Trp Val Asn Asn Val Arg Ser Thr Thr Gly Lys Asn Leu Phe
                   245                 250                 255
   Ala Val Gly Glu Tyr Trp His Tyr Asp Val Asn Lys Leu Asn Ser Tyr
               260                 265                 270
   Ile Thr Lys Thr Asn Gly Thr Met Ser Leu Phe Asp Val Pro Leu His
           275                 280                 285
   Phe Arg Phe Tyr Asp Ala Ser Asn Gly Gly Gly Gly Tyr Asp Met Arg
   290 295 300
   Asn Leu Leu Asn Asn Thr Leu Met Ser Ser Asn Pro Met Lys Ala Val
   305 310 315 320
   Thr Phe Val Glu Asn His Asp Thr Gln Pro Thr Gln Ala Leu Gln Ser
   325 330 335
   Thr Val Gln Ser Trp Phe Lys Pro Leu Ala Tyr Ala Thr Ile Leu Thr
   340 345 350
   Arg Glu Gln Gly Tyr Pro Cys Val Phe Tyr Gly Asp Tyr Tyr Gly Thr
   355 360 365
   Ser Asp Gly Lys Ile Ser Ser Tyr Lys Pro Ile Met Asp Lys Leu Leu
   370 375 380
   Asn Ala Arg Lys Val Tyr Ala Tyr Gly Thr Gln Arg Asp Tyr Phe Asp
   385 390 395 400
   His Pro Asp Ile Val Gly Trp Thr Arg Glu Gly Asp Ala Ala His Ala
   405 410 415
   Gly Ser Gly Leu Ala Thr Leu Ile Thr Asp Gly Pro Gly Gly Ser Lys
   420 425 430
   Trp Met Tyr Val Gly Thr Ser Lys Ala Gly Gln Val Trp Thr Asp Lys
   435 440 445
   Thr Gly Asn Arg Ser Gly Thr Val Thr Ile Asp Ala Asn Gly Trp Gly
   450 455 460
   Asn Phe Trp Val Asn Gly Gly Ser Val Ser Val Trp Ala Lys
   465 470 475
<210> 5
   <211> 26
   <212> PRT
   <213> Bacillus licheniformis
<220>
   <221> misc_feature
   <223> B. licheniformis Epr signal peptide
<400> 5
   Met Lys Lys Leu Trp Lys Ile Ala Val Ser Ala Ala Met Phe Val Gly
   1 5 10 15
   Phe Phe Ala Asn Ser Pro Arg Ala Ser Ala
               20                  25

## Description

### FIELD OF THE INVENTION

Disclosed are compositions and methods relating to variant α-amylases. The variant α-amylases are useful, for example, for starch liquefaction and saccharification, cleaning starchy stains, textile desizing, baking, and brewing.

### BACKGROUND

α-Amylases hydrolyze starch, glycogen, and related polysaccharides by cleaving internal α-1,4-glucosidic bonds. α-Amylases, particularly from *Bacilli,* have been used for a variety of different purposes, including but not limited to starch liquefaction and saccharification, textile desizing, starch modification in the paper and pulp industry, brewing, baking, production of syrups for the food industry, production of feedstocks for fermentation processes, and in animal feed to increase digestibility. These enzymes can also be used to remove starchy soils and stains during dishwashing and laundry washing. There is continued interest in the identification and/or engineering of α-amylases for industrial and commercial use.

WO 00/60058 discloses α-amylase enzymes from *Bacillus* and variants thereof.

The sequence of a putative α-amylase from *Paenibacillus curdanolyticus* YK9 is deposited under EBI accession number UNIPROT:E0I4J1, entry version 12, 28 November 2012 (E0I4d1_9BACL).

### BRIEF SUMMARY

The invention provides a method of preparing a variant α-amylase polypeptide from a parental α-amylase polypeptide, comprising introducing at least one combinable mutation into said parental α-amylase polypeptide at a productive amino acid position; wherein:
(a) the combinable mutation produces a variant amylase wherein the minimum performance indices (PI) relative to the parental amylase for:
   (i) protein expression,
   (ii) activity, and
   (iii) detergent stability or thermostability are either
      (aa) greater than or equal to 0.9, and the PI for any one of (i), (ii), or (iii) is greater than 1.0,
      (ab) greater than or equal to 0.8, and the PI for any one of (i), (ii), or (iii) is greater than or equal to 1.2, or
      (ac) greater than or equal to 0.5, and the PI for any one of (i), (ii), or (iii) is greater than or equal to 1.5;
      wherein protein expression, activity, detergent stability and thermostability are each determined using an assay described in Example 1;
(b) the productive position is an amino acid position that can be substituted with a plurality of different amino acid residues, each of which substitutions result in a variant α-amylase that meets the requirements of (i),
(c) the productive position is T333, using SEQ ID NO: 3 for numbering, and
(d) the combinable mutation is T333F, T333Q, T333Y, T333N, T333S, T333A, T333D, T333E or T333G,
wherein the parental α-amylase and the variant α-amylase have at least 80% amino acid sequence identity to the amino acid sequence of SEQ ID NO: 3 or SEQ ID NO: 4.

The method may comprise introducing a plurality of combinable mutations into said parental α-amylase, wherein each said combinable substitution is at a productive position selected from 472, 280, 191, 1, 2, 3, 4, 5, 6, 9, 10, 14, 15, 16, 17, 19, 20, 22, 23, 24, 25, 26, 27, 28, 29, 30, 31, 32, 33, 34, 35, 36, 37, 38, 39, 41, 44, 45, 47, 48, 49, 50, 51, 53, 57, 60, 63, 65, 67, 68, 69, 70, 71, 72, 73, 74, 76, 78, 79, 80, 81, 82, 83, 84, 85, 86, 87, 88, 89, 90, 91, 92, 93, 94, 95, 96, 97, 98, 100, 101, 102, 105, 107, 110, 111, 112, 113, 114, 115, 116, 117, 120, 121, 122, 125, 126, 127, 128, 129, 130, 131, 132, 133, 134, 135, 136, 137, 138, 139, 140, 141, 142, 143, 144, 146, 147, 148, 149, 150, 151, 152, 153, 155, 156, 157, 162, 164, 166, 167, 168, 169, 170, 171, 172, 173, 174, 175, 176, 179, 180, 181, 182, 189, 190, 192, 200, 202, 203, 204, 205, 206, 207, 208, 210, 211, 212, 213, 214, 215, 217, 218, 220, 221, 222, 223, 224, 225, 226, 227, 229, 231, 234, 237, 238, 239, 241, 242, 243, 244, 246, 247, 248, 249, 250, 251, 252, 253, 254, 255, 256, 257, 258, 259, 260, 261, 264, 265, 266, 267, 268, 269, 270, 271, 272, 273, 274, 275, 276, 278, 279, 281, 282, 283, 285, 287, 289, 291, 292, 293, 294, 295, 297, 298, 299, 300, 302, 303, 306, 307, 309, 310, 311, 313, 314, 315, 316, 317, 318, 319, 320, 322, 331, 337, 338, 339, 340, 341, 342, 345, 347, 348, 356, 357, 359, 361, 362, 364, 367, 368, 369, 370, 371, 373, 374, 376, 377, 378, 379, 380, 381, 382, 383, 384, 385, 386, 387, 388, 390, 391, 392, 396, 398, 401, 402, 403, 404, 405, 406, 407, 412, 414, 415, 416, 418, 419, 420, 425, 426, 427, 430, 431, 432, 433, 435, 440, 441, 442, 443, 444, 446, 448, 450, 451, 452, 453, 454, 455, 456, 457, 459, 461, 462, 463, 465, 467, 469, 471, 473, 474, 475, 477, and 480, using SEQ ID NO: 3 for numbering.

The method may further comprise introducing a deletion corresponding to a residue selected from the group consisting of Arg-177, Gly-178, Asp-179, and Gly-180, using SEQ ID NO: 3 for numbering, e.g. introducing deletions corresponding to residues Arg-177 and Gly-178.

The parental α-amylase and the variant α-amylase may have at least 90%, preferably at least 95%, amino acid sequence identity to the amino acid sequence of SEQ ID NO: 3 or SEQ ID NO: 4.,

The method may further comprise the step of formulating the variant α-amylase into a composition.

The composition may further comprise a surfactant.

The composition may be a detergent composition, a composition for liquefying starch, a composition for saccharifying a composition comprising starch, a composition for SSF post liquefaction, a composition for direct SSF without prior liquefaction, a composition for producing a fermented beverage, a composition for producing a baked food product, a composition for textile desizing, or is a composition effective for removing starchy stains from laundry, dishes, or textiles.

Specific examples include a laundry detergent, a laundry detergent additive, a manual or automatic dishwashing detergent, and a dishwashing machine cleaning composition.

The composition may further comprise one or more additional enzymes selected from the group consisting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase and laccase.

Throughout this specification, it will be understood that references to "the present α-amylase" or the like refer to α-amylases produced by the methods of the invention.

These and other aspects and embodiments of the present compositions and methods are further described, below.

### BRIEF DESCRIPTION OF THE DRAWINGS

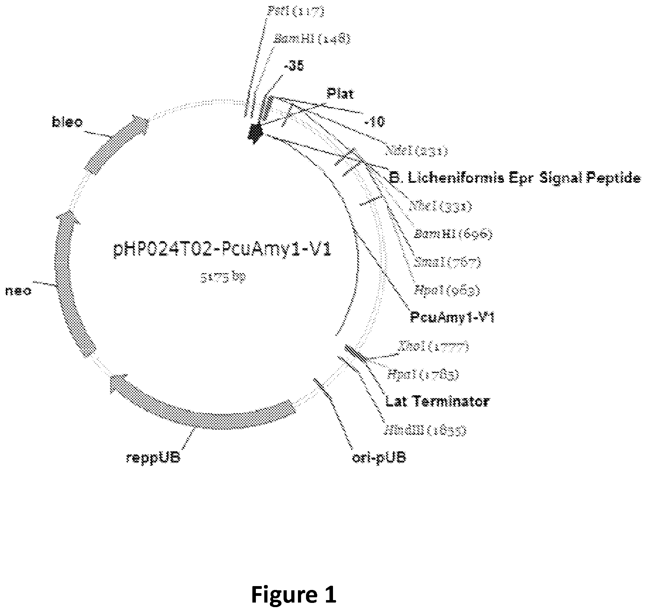

Figure 1 is a plasmid map of pHP024T02-PcuAmyl-vl, which produces the variant PcuAmyl amylase, PcuAmyl-vl.

### BRIEF DESCRIPTION OF THE SEQUENCES

SEQ ID NO:1 is a codon-modified nucleic acid sequence encoding the mature form of PcuAmyl-vl (SEQ ID NO: 4) attached to the *Bacillus licheniformis* Epr signal peptide (SEQ ID NO: 5).
SEQ ID NO: 2 sets forth the amino acid sequence of the precursor form of PcuAmyl α-amylase, which includes the *B. licheniformis* Epr signal peptide.
SEQ ID NO: 3 sets forth the amino acid sequence of the mature form of PcuAmyl α-amylase.
SEQ ID NO: 4 sets forth the amino acid sequence of the mature form of PcuAmyl-vl variant amylase, having a deletion of residues Arg-177 and Gly-178.
SEQ ID NO:5 sets forth the amino acid sequence of the *B. licheniformis* Epr signal peptide.

### DETAILED DESCRIPTION

As summarized above, described herein are compositions and methods relating to PcuAmyl α-amylase, and variants, thereof, which find use in numerous applications including starch liquefaction and saccharification, for cleaning starchy stains in laundry, dishwashing, and other applications, for textile processing (*e.g.,* desizing), in animal feed for improving digestibility, and for baking and brewing. These and other aspects of the compositions and methods are described in detail, below.

Prior to describing the various aspects and embodiments of the present compositions and methods, the following definitions and abbreviations are described.

### 1. Definitions and Abbreviations

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art. Abbreviations and definitions for certain terms are set forth below.

### 1.1. Abbreviations and Acronyms

The following abbreviations/acronyms, when and if present, have the following meanings unless otherwise specified:
- ABTS: 2,2-azino-bis-3-ethylbenzothiazoline-6-sulfonic acid
- AE or AEO: alcohol ethoxylate
- AES or AEOS: alcohol ethoxysulfate
- AkAA: *Aspergillus kawachii* α-amylase
- AnGA: *Aspergillus niger* glucoamylase
- AOS: α-olefinsulfonate
- AS: alkyl sulfate
- cDNA: complementary DNA
- CMC: carboxymethylcellulose
- DE: dextrose equivalent
- DNA: deoxyribonucleic acid
- DPn: degree of saccharide polymerization having n subunits
- ds or DS: dry solids
- DTMPA: diethylenetriaminepentaacetic acid
- EC: Enzyme Commission
- EDTA: ethylenediaminetetraacetic acid
- EO: ethylene oxide (polymer fragment)
- EOF: End of Fermentation
- FH: French hardness
- GA: glucoamylase
- GAU/g ds: glucoamylase activity unit/gram dry solids
- GH: general hardness
- HFCS: high fructose corn syrup
- HgGA: *Humicola grisea* glucoamylase
- IPTG: isopropyl β-D-thiogalactoside
- IRS: insoluble residual starch
- kDa: kiloDalton
- LAS: linear alkylbenzenesulfonate
- LAT, BLA: *B. licheniformis* amylase
- MW: molecular weight
- MWU: modified Wohlgemuth unit; 1.6x10⁻⁵ mg/MWU = unit of activity
- NCBI: National Center for Biotechnology Information
- NOBS: nonanoyloxybenzenesulfonate
- NTA: nitriloacetic acid
- OxAm: Purastar HPAM 5000L (Danisco US Inc.)
- PAHBAH: p-hydroxybenzoic acid hydrazide
- PEG: polyethyleneglycol
- pI: isoelectric point
- PI: performance index
- ppm: parts per million, *e.g.,* µg protein per gram dry solid
- PVA: poly(vinyl alcohol)
- PVP: poly(vinylpyrrolidone)
- RCF: relative centrifugal/centripetal force (*i.e.,* x gravity)
- RNA: ribonucleic acid
- SAS: alkanesulfonate
- SDS-PAGE: sodium dodecyl sulfate polyacrylamide gel electrophoresis
- SSF: simultaneous saccharification and fermentation
- SSU/g solid: soluble starch unit/gram dry solids
- sp.: species
- TAED: tetraacetylethylenediamine
- Tm: melting temperature
- TrGA: *Trichoderma reesei* glucoamylase
- w/v: weight/volume
- w/w: weight/weight
- v/v: volume/volume
- wt%: weight percent
- °C: degrees Centigrade
- H₂O: water
- dH₂O or DI: deionized water
- dIH₂O: deionized water, Milli-Q filtration
- g or gm: grams
- µg: micrograms
- mg: milligrams
- kg: kilograms
- µL and µl: microliters
- mL and ml: milliliters
- mm: millimeters
- µm: micrometer
- M: molar
- mM: millimolar
- µM: micromolar
- U: units
- sec: seconds
- min(s): minute/minutes
- hr(s): hour/hours
- DO: dissolved oxygen
- Ncm: Newton centimeter
- ETOH: ethanol
- eq.: equivalents
- N: normal
- uPWA: variant α-amylase derived from *Pyrococcus woesei*
- PWA: α-amylase from *Pyrococcus woesei*
- MWCO: molecular weight cut-off
- SSRL: Stanford Synchrotron Radiation Lightsource
- PDB: Protein Database
- CAZy: Carbohydrate-Active Enzymes database
- Tris-HCl: tris(hydroxymethyl)aminomethane hydrochloride
- HEPES: 4-(2-hydroxyethyl)-1-piperazineethanesulfonic acid

### 1.2. Definitions

The terms "amylase" or "amylolytic enzyme" refer to an enzyme that is, among other things, capable of catalyzing the degradation of starch. α-Amylases are hydrolases that cleave the α-D-(1→4) O-glycosidic linkages in starch. Generally, α-amylases (EC 3.2.1.1; α-D-(1→4)-glucan glucanohydrolase) are defined as endo-acting enzymes cleaving α-D-(1→4) O-glycosidic linkages within the starch molecule in a random fashion yielding polysaccharides containing three or more (1-4)-α-linked D-glucose units. In contrast, the exo-acting amylolytic enzymes, such as β-amylases (EC 3.2.1.2; α-D-(1→4)-glucan maltohydrolase) and some product-specific amylases like maltogenic α-amylase (EC 3.2.1.133) cleave the polysaccharide/starch molecule from the non-reducing end of the substrate. β-amylases, α-glucosidases (EC 3.2.1.20; α-D-glucoside glucohydrolase), glucoamylase (EC 3.2.1.3; α-D-(1→4)-glucan glucohydrolase), and product-specific amylases like the maltotetraosidases (EC 3.2.1.60) and the maltohexaosidases (EC 3.2.1.98) can produce malto-oligosaccharides of a specific length or enriched syrups of specific maltooligosaccharides.

By "PcuAmyl α-amylase" is meant an enzyme having at least 80 % amino acid sequence identity to SEQ ID NO: 3 and having amylase activity (as described above). For example, a PcuAmyl α-amylase having amylase activity can have at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or even at least 99% amino acid sequence identity to SEQ ID NO: 3. In certain embodiments, the PcuAmyl α-amylase is 100% identical to SEQ ID NO: 3.

By "nucleic acid encoding a PcuAmyl α-amylase" (or variants thereof) is meant a nucleic acid (or polynucleotide) having at least 40% nucleic acid sequence identy to SEQ ID NO: 1 in which the encoded PcuAmyl α-amylase has amylase activity. For example, a nucleic acid encoding a PcuAmy1 α-amylase can have at least 45%, at least 50%, at least 60%, at least 65%, at least 70%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or even at least 99% amino acid sequence identity to SEQ ID NO: 1. In certain embodiments, the nucleic acid encoding a PcuAmyl α-amylase is 100% identical to SEQ ID NO: 1. In additional embodiments, a nucleic acid encoding a PcuAmyl α-amylase hybridizes under stringent conditions to a nucleic acid having a sequence complementary to SEQ ID NO: 1.

By "comprising" is meant including, but not limited to, whatever follows the word "comprising". Thus, use of the term "comprising" indicates that the listed element(s) is required or mandatory, but that one or more other elements are optional and may or may not be present. For example, the phrase "an enzyme comprising amino acid sequence X" means an enzyme that has amino acid sequence X but that may also include additional sequences, e.g., an amino acid or amino acid sequence that is N terminal (or C terminal) to amino acid sequence X. By "consisting of" is meant including, and limited to, whatever follows the phrase "consisting of". Thus, the phrase "consisting of" indicates that the listed element(s) is required or mandatory, and that no other element(s) may be present. For example, the phrase "an enzyme consisting of amino acid sequence X" means an enzyme that has amino acid sequence X and that does not include any additional amino acid sequences. By "consisting essentially of" is meant including any element(s) listed after the phrase, and limited to other element(s) that do not interfere with or contribute to the activity or action specified in the disclosure for the listed element(s). Thus, the phrase "consisting essentially of" indicates that the listed elements are required or mandatory, but that other elements are optional and may or may not be present depending upon whether or not they affect the activity or action of the listed elements.

"Enzyme units" herein refer to the amount of product formed per time under the specified conditions of the assay. For example, a "glucoamylase activity unit" (GAU) is defined as the amount of enzyme that produces 1 g of glucose per hour from soluble starch substrate (4% DS) at 60°C, pH 4.2. A "soluble starch unit" (SSU) is the amount of enzyme that produces 1 mg of glucose per minute from soluble starch substrate (4% DS) at pH 4.5, 50°C. DS refers to "dry solids." As another example, maltogenic amylase activity can be measured in degrees Diastatic Power (DP°) Units. This assay is based on a 30-min hydrolysis of a starch substrate at pH 4.6 and 20°C. The reducing sugar groups produced on hydrolysis are measured in a titrimetric procedure using alkaline ferricyanide. One unit of diastase activity, expressed as degrees DP (DP°), is defined as the amount of enzyme, contained in 0.1 ml of a 5% solution of the sample enzyme preparation, that will produce sufficient reducing sugars to reduce 5 mL of Fehling's solution when the sample is incubated with 100 mL of the substrate for 1 hour at 20°C.

In the case of the present α-amylases, "activity" refers to α-amylase activity, which can be measured as described herein.

The term "starch" refers to any material comprised of the complex polysaccharide carbohydrates of plants, comprised of amylose and amylopectin with the formula (C₆H₁₀O₅)ₓ, wherein X can be any number. The term includes plant-based materials such as grains, cereal, grasses, tubers and roots, and more specifically materials obtained from wheat, barley, corn, rye, rice, sorghum, brans, cassava, millet, milo, potato, sweet potato, and tapioca. The term "starch" includes granular starch. The term "granular starch" refers to raw, *i.e.,* uncooked starch, *e.g.,* starch that has not been subject to gelatinization.

The terms "wild-type" with respect to a polypeptide refers to a naturally-occurring polypeptide that does not include a man-made substitution, insertion, or deletion at one or more amino acid positions. Similarly, the term "wild-type" with respect to a polynucleotide refers to a naturally-occurring polynucleotide that does not include a man-made nucleoside change. However, note that a polynucleotide encoding a wild-type polypeptide is not limited to a naturally-occurring polynucleotide, and encompasses any polynucleotide encoding the wild-type polypeptide.

The term "parental" or "reference" with respect to a polypeptide or polynucleotide sequence means a polypeptide or polynucleotide sequence that serves as the template sequence used for generating altered (or variant) forms of the polypeptide or polynucleotide.

Reference to the wild-type polypeptide is understood to include the mature form of the polypeptide. A "mature" polypeptide (or variant thereof) is one in which a signal sequence is absent, for example, cleaved from an immature form of the polypeptide during or following expression of the polypeptide.

The term "variant" with respect to a polypeptide refers to a polypeptide that differs from a specified parental or reference polypeptide (*e.g.,* a wild-type polypeptide) in that it includes one or more naturally-occurring or man-made changes including but not limited to substitutions, insertions, or deletions (*e.g.,* truncation) of one or more amino acids. Similarly, the term "variant," with respect to a polynucleotide, refers to a polynucleotide that differs in nucleotide sequence from a specified parental or reference polynucleotide. The identity of the parental or reference polypeptide or polynucleotide will be apparent from context. A variant can include one or more specific substitutions, insertions, and/or deletions as well as having a % sequence identity to the parental sequence.

By "alteration" with respect to a polypeptide or polynucleotide sequence is meant that the polypeptide or polynucleotide is different with respect to a parental or reference sequence. An alteration may be a variation in the amino acid or polynucleotide sequence (as described above) or it may be a modification that does not impact the sequence, *e.g.,* a chemical or enzymatic modification of the polypeptide or polynucleotide.

The term "recombinant," when used in reference to a subject cell, nucleic acid, protein or vector, indicates that the subject has been modified from its native state. Thus, for example, recombinant cells express genes that are not found within the native (non-recombinant) form of the cell, or express native genes at different levels or under different conditions than found in nature. Recombinant nucleic acids differ from a native sequence by one or more nucleotides and/or are operably linked to heterologous sequences, *e.g.,* a heterologous promoter in an expression vector. Recombinant proteins may differ from a native sequence by one or more amino acids and/or are fused with heterologous sequences. A vector comprising a nucleic acid encoding an amylase is a recombinant vector.

The term "isolated" refers to a compound, polypeptide, cell, nucleic acid, amino acid, or other specified material or component that is present in a form that is not found in nature. For example, an isolated component is one that has been removed from or is present at a substantially different relative concentration with respect to at least one other material or component with which it is naturally associated as found in nature. Thus, an isolated polypeptide (or enzyme) includes, but is not limited to, a culture supernatant (or broth) containing the polypeptide that is obtained from host cells that express and secrete the polypeptide.

The term "enriched" refers to a compound, polypeptide, cell, nucleic acid, amino acid, or other specified material or component that is present in a composition at a relative or absolute concentration that is higher than a starting composition.

The term "purified" refers to a compound, polypeptide, cell, nucleic acid, amino acid, or other specified material or component that is present in a relatively pure state as compared to a starting composition. Purity in some instances may be defined in percentages, where in certain embodiments a component in a composition may be at least 10% pure (*e.g.,* on the basis of weight, on a molecular basis, etc.), at least 20% pure, at least 30% pure, at least 40% pure at least 50% pure, at least 60% pure, at least 70% pure, at least 80% pure, at least 90% pure, at least 95% pure, at least 98% pure, or even at least 99% pure.

The terms "thermostable" and "thermostability," with reference to an enzyme, refer to the ability of the enzyme to retain activity after exposure to an elevated temperature. The thermostability of an enzyme, such as an amylase enzyme, is measured by its half-life (t_{1/2}) given in minutes, hours, or days, during which half the enzyme activity is lost under defined conditions. The half-life may be calculated by measuring residual α-amylase activity following exposure to (*i.e.,* challenge by) an elevated temperature.

A "pH range," with reference to an enzyme, refers to the range of pH values under which the enzyme exhibits catalytic activity.

The terms "pH stable" and "pH stability," with reference to an enzyme, relate to the ability of the enzyme to retain activity over a wide range of pH values for a predetermined period of time (*e.g.,* 15 min., 30 min., 1 hour).

The term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e.,* N→C).

The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

By "homologue" shall mean an entity having a specified degree of identity with the subject amino acid sequences and the subject nucleotide sequences. A homologous sequence is taken to include an amino acid sequence that is at least 75%, 80%, 85%, 86%, 87%, 88%, 89%, 90%, 91%, 92%, 93%, 94%, 95%, 96%, 97%, 98% or even 99% identical to the subject sequence, using conventional sequence alignment tools (*e.g.,* Clustal, BLAST, and the like). Typically, homologues will include the same active site residues as the subject amino acid sequence, unless otherwise specified.

The term "plurality" refers to two or more.

"Hybridization" refers to the process by which one strand of nucleic acid forms a duplex with, *i.e.,* base pairs with, a complementary strand, as occurs during blot hybridization techniques and PCR techniques. A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while intermediate or low stringency hybridization can be used to identify or detect polynucleotide sequence homologs.

Moderate and high stringency hybridization conditions are well known in the art. Stringent hybridization conditions are exemplified by hybridization under the following conditions: 65°C and 0.1X SSC (where 1X SSC = 0.15 M NaCl, 0.015 M Na₃ citrate, pH 7.0). Hybridized, duplex nucleic acids are characterized by a melting temperature (Tₘ), where one-half of the hybridized nucleic acids are unpaired with the complementary strand. Mismatched nucleotides within the duplex lower the Tₘ. Very stringent hybridization conditions involve 68°C and 0.1X SSC. A nucleic acid encoding a variant α-amylase may have a Tₘ reduced by 1°C - 3°C or more compared to a duplex formed between the nucleotide of SEQ ID NO: 1 and its identical complement.

Another example of high stringency conditions includes hybridization at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. An example of moderate stringent conditions include an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. Those of skill in the art know how to adjust the temperature, ionic strength, etc. as necessary to accommodate factors such as probe length and the like.

A "synthetic" molecule is produced by *in vitro* chemical or enzymatic synthesis rather than by an organism.

The terms "transformed," "stably transformed," and "transgenic," used with reference to a cell means that the cell contains a non-native (*e.g.,* heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest (*e.g.,* an amylase) has been introduced. Exemplary host strains are microorganism cells (*e.g.,* bacteria, filamentous fungi, and yeast) capable of expressing the polypeptide of interest and/or fermenting saccharides. The term "host cell" includes protoplasts created from cells.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

The term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (*e.g.,* hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequences.

A "signal sequence" is a sequence of amino acids attached to the N-terminal portion of a protein which facilitates the secretion of the protein outside the cell. The mature form of an extracellular protein lacks the signal sequence, which is cleaved off during the secretion process.

"Biologically active" refers to a sequence having a specified biological activity, such as enzymatic activity.

The term "specific activity" refers to the quantity of substrate that can be converted to product by a given amount of enzyme or enzyme preparation, typically in a given amount of time under specified conditions. Specific activity is generally expressed as units (U)/mg of enzyme.

As used herein, "water hardness" is a measure of the minerals (*e.g.,* calcium and magnesium) present in water.

A "swatch" is a piece of material such as a fabric that has a stain applied thereto. The material can be, for example, fabrics made of cotton, polyester or mixtures of natural and synthetic fibers. The swatch can further be paper, such as filter paper or nitrocellulose, or a piece of a hard material such as ceramic, metal, or glass. For amylases, the stain is starch based, but can include blood, milk, ink, grass, tea, wine, spinach, gravy, chocolate, egg, cheese, clay, pigment, oil, or mixtures of these compounds.

A "smaller swatch" is a section of the swatch that has been cut with a single hole punch device, or has been cut with a custom manufactured 96-hole punch device, where the pattern of the multi-hole punch is matched to standard 96-well microtiter plates, or the section has been otherwise removed from the swatch. The swatch can be of textile, paper, metal, or other suitable material. The smaller swatch can have the stain affixed either before or after it is placed into the well of a 24-, 48- or 96-well microtiter plate. The smaller swatch can also be made by applying a stain to a small piece of material. For example, the smaller swatch can be a stained piece of fabric 5/8" or 0.25" in diameter. The custom manufactured punch is designed in such a manner that it delivers 96 swatches simultaneously to all wells of a 96-well plate. The device allows delivery of more than one swatch per well by simply loading the same 96-well plate multiple times. Multi-hole punch devices can be conceived of to deliver simultaneously swatches to any format plate, including but not limited to 24-well, 48-well, and 96-well plates. In another conceivable method, the soiled test platform can be a bead made of metal, plastic, glass, ceramic, or another suitable material that is coated with the soil substrate. The one or more coated beads are then placed into wells of 96-, 48-, or 24-well plates or larger formats, containing suitable buffer and enzyme.

"A cultured cell material comprising an amylase" or similar language, refers to a cell lysate or supernatant (including media) that includes an amylase as a component. The cell material may be from a heterologous host that is grown in culture for the purpose of producing the amylase.

"Percent sequence identity," "percent amino acid sequence identity," "percent gene sequence identity," and/or "percent nucleic acid/polynucleotide sequence identity," with respect to two amino acid, polynucleotide and/or gene sequences (as appropriate), refer to the percentage of residues that are identical in the two sequences when the sequences are optimally aligned. Thus, "80% amino acid sequence identity" means that 80% of the amino acids in two optimally aligned polypeptide sequences are identical.

The phrase "substantially identical" in the context of comparing a nucleic acid or a polypeptide sequence to a reference sequence refers to a polynucleotide or polypeptide that has at least 80% sequence identity to the reference sequence, including at least 85%, at least 90%, at least 95%, at least 97% , at least 98%, and at least 99% sequence identity, using a sequence alignment program or algorithm (*e.g.,* BLAST, ALIGN, CLUSTAL) under standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g.,* within a range of medium to high stringency).

Percent Sequence identity can be determined by known methods of sequence alignment. A commonly used alignment method is BLAST, although there are other methods that also find use in aligning sequences. An example of a useful algorithm for aligning two or more sequences is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (Feng and Doolittle, J Mol Evol, 35:351-360, 1987). The method is similar to that described by Higgins and Sharp (Higgins and Sharp, CABIOS 5:151-153, 1989). Useful PILEUP parameters including a default gap weight of 3.00, a default gap length weight of 0.10, and weighted end gaps.

Another example of a useful algorithm is the BLAST algorithm, described by Altschul *et al.,* (Altschul et al., J Mol Biol, 215:403-410, 1990; and Karlin et al., Proc Natl Acad Sci USA, 90:5873-5787, 1993). A particularly useful BLAST program is the WU-BLAST-2 program (*See,* Altschul et al., Meth Enzymol, 266:460-480, 1996). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

To illustrate, the percent (%) nucleic acid sequence identity would be defined as the percentage of nucleotide residues in a candidate nucleic acid sequence that are identical to the nucleotide residues of the starting nucleic acid sequence (*i.e.,* the sequence of interest). When using the BLASTN module of WU-BLAST-2 it is set to the default parameters, with overlap span and overlap fraction set to 1 and 0.125, respectively.

The NCBI BLAST2 algorithm finds the most relevant sequences in terms of biological similarity but is not recommended for query sequences of less than 20 nucleotides, for example. Example default BLAST parameters for a nucleotide sequence are:
- Word size: 11
- E-value cutoff : 10
- Scoring Matrix : NUC.3.1 (match = 1, mismatch = -3)
- Gap Opening : 5
- Gap Extension : 2
and the following parameters for protein searches:
- Word size : 3
- E-value cutoff : 10
- Scoring Matrix : BLOSUM62
- Gap Opening : 11
- Gap extension : 1

The CLUSTAL W algorithm is another example of a sequence alignment algorithm. *See* Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

Deletions are counted as non-identical residues, compared to a reference sequence. Deletions occurring at either termini are included. For example, a variant with five amino acid deletions of the C-terminus of the mature PcuAmyl polypeptide of SEQ ID NO:3 would have a percent sequence identity of 99% (475 / 480 identical residues × 100, rounded to the nearest whole number) relative to the mature polypeptide. Such a variant would be encompassed by a variant having "at least 99% sequence identity" to a mature amylase polypeptide. This can be described as percent identity over the alignment length where the query sequence is 480 amino acids in length and the mature polypeptide (subject) is 475 amino acids in length.

"Fused" polypeptide sequences are connected, *i.e.,* operably linked, via a peptide bond between two subject polypeptide sequences.

The term "filamentous fungi" refers to all filamentous forms of the subdivision Eumycotina, particularly Pezizomycotina species.

The term "degree of polymerization" (DP) refers to the number (n) of anhydro-glucopyranose units in a given saccharide. Examples of DP1 are the monosaccharides glucose and fructose. Examples of DP2 are the disaccharides maltose and sucrose. The term "DE," or "dextrose equivalent," is defined as the percentage of reducing sugar, *i.e.,* D-glucose, as a fraction of total carbohydrate in a syrup.

The term "dry solids content" (ds) refers to the total solids of a slurry in a dry weight percent basis. The term "slurry" refers to an aqueous mixture containing insoluble solids.

The phrase "simultaneous saccharification and fermentation (SSF)" refers to a process in the production of biochemicals in which a microbial organism, such as an ethanologenic microorganism, and at least one enzyme, such as an amylase, are present during the same process step. SSF includes the contemporaneous hydrolysis of starch substrates (granular, liquefied, or solubilized) to saccharides, including glucose, and the fermentation of the saccharides into alcohol or other biochemical or biomaterial in the same reactor vessel.

An "ethanologenic microorganism" refers to a microorganism with the ability to convert a sugar or oligosaccharide to ethanol.

The term "fermented beverage" refers to any beverage produced by a method comprising a fermentation process, such as a microbial fermentation, *e.g.,* a bacterial and/or fungal fermentation. "Beer" is an example of such a fermented beverage, and the term "beer" is meant to comprise any fermented wort produced by fermentation/brewing of a starch-containing plant material. Often, beer is produced exclusively from malt or adjunct, or any combination of malt and adjunct. Examples of beers include: full malted beer, beer brewed under the "Reinheitsgebot," ale, India pale ale, lager, pilsner, bitter, Happoshu (second beer), third beer, dry beer, near beer, light beer, low alcohol beer, low calorie beer, porter, bock, dopplebock, stout, porter, malt liquor, non-alcoholic beer, non-alcoholic malt liquor and the like, but also alternative cereal and malt beverages such as fruit flavored malt beverages, *e.g.,* citrus flavored, such as lemon-, orange-, lime-, or berry-flavored malt beverages, liquor flavored malt beverages, *e.g.,* vodka-, rum-, or tequila-flavored malt liquor, or coffee flavored malt beverages, such as caffeine-flavored malt liquor, and the like.

The term "malt" refers to any malted cereal grain, such as malted barley or wheat.

The term "maltogenic amylase" refers to enzymes that are capable of producing significant amounts of maltose from starch or hydrolyzed starch.

The term "adjunct" refers to any starch and/or sugar containing plant material that is not malt, such as barley or wheat malt. Examples of adjuncts include common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, cassava and syrups, such as corn syrup, sugar cane syrup, inverted sugar syrup, barley and/or wheat syrups, and the like.

The term "mash" refers to an aqueous slurry of any starch and/or sugar containing plant material, such as grist, *e.g.,* comprising crushed barley malt, crushed barley, and/or other adjunct or a combination thereof, mixed with water later to be separated into wort and spent grains.

The term "wort" refers to the unfermented liquor run-off following extracting the grist during mashing.

"Iodine-positive starch" or "IPS" refers to (1) amylose that is not hydrolyzed after liquefaction and saccharification, or (2) a retrograded starch polymer. When saccharified starch or saccharide liquor is tested with iodine, the high DPn amylose or the retrograded starch polymer binds iodine and produces a characteristic blue color. The saccharide liquor is thus termed "iodine-positive saccharide," "blue saccharide," or "blue sac."

The terms "retrograded starch" or "starch retrogradation" refer to changes that occur spontaneously in a starch paste or gel on ageing.

The term "about" refers to ± 5% to the referenced value.

"Combinatorial variants" are variants comprising two or more substitutions, deletions, and/or insertions, *e.g.,* 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, etc., or more.

As used herein, "combinable mutations" are mutations at any amino acid position that can be used to make combinatorial variants. Combinable mutations improve at least one desired property of the molecule (in this case, an α-amylase), while not significantly decreasing either expression, activity, or stability. Combinable mutations can be grouped as follows:
**Group A:** A mutation that produced a variant wherein the minimum performance indices (PI) relative to a defined parental protein for: (i) protein expression or activity in Ceralpha assay (at pH 5.6 and 7), and (ii) detergent stability or thermostability at pH 5.0 or pH 5.7 was greater than or equal to 0.9, and in addition had a PI for any of the other tests that was greater than or equal to 1.0.
**Group B:** A mutation that produced a variant wherein the minimum performance indices (PI) relative to a defined parental protein for: (i) protein expression or activity in Ceralpha assay (at pH 5.6 and 7), and (ii) detergent stability or thermostability at pH 5.0 or pH 5.7 was greater than or equal to 0.8, and in addition have a PI for any of the other tests that was greater than or equal to 1.2.
**Group C:** A mutation that produced a variant wherein the minimum performance indices (PI) relative to a defined parental protein for: (i) protein expression or activity in Ceralpha assay (at pH 5.6 and 7), and (ii) detergent stability or thermostability at pH 5.0 or pH 5.7 was greater than or equal to 0.5, and in addition have a PI for any of the other tests that was greater than or equal to 1.5.

The properties of combinable mutations are summarized in the following Table.

**Table 1. Performance properties for each group of combinable mutations**

| Performance Index (PI) | | | |
|---|---|---|---|
| Group | Protein expression or Ceralpha assays | Detergent stability or thermostability | Minimum PI in one or more tests including: cleaning or starch hydrolysis assays |
| A | ≥ 0.9 | ≥ 0.9 | X ≥ 1.0 |
| B | ≥ 0.8 | ≥ 0.8 | X ≥ 1.2 |
| C | ≥ 0.5 | ≥ 0.5 | X ≥ 1.5 |

Examples of combinable mutations are at "productive positions," as described, below. In the case of the present α-amylases, "activity" refers to α-amylase activity, which can be measured as described, herein.

As used herein, "productive positions" are amino acid positions that are tolerant to substitution with different amino acid residues, wherein the resulting variants meet a set of performance criteria for combinability, as set forth above. Productive positions can be assigned a Productivity Score as follows:

Positions where less than 15% of the substitutions at a given position fall within groups A, B, or C are given a Productivity Score of "1". Positions where less than 45%, but greater than, or equal to 15% of the substitutions at a given position fall within groups A, B, or C are given a Productivity Score of "2". Positions where less than 75%, but greater than, or equal to 30% of the substitutions at a given position fall within groups A, B, or C are given a Productivity Score of "3". Positions where 50% or more of the substitutions at a given position fall within groups A, B, or C are given a Productivity Score of "4".

Productive positions are combinable mutations.

As used herein, "suitability score" refers to the ability of one or more combinable mutations to be used to make combinatorial variants, based on the performance criteria for combinability, (*i.e.,* A, B, and C, as set forth, above) in which each of the mutations fall. A higher suitability score indicates a mutation or mutations that are more suitable for use in making combinatorial variants.

Suitability scores are described in Table 2.

**Table 2. Definitions of suitability scores**

| **Substitutions Occur in Group(s)** | **Suitability Score** |
|---|---|
| A, B and C | +++++ |
| A and B | ++++ |
| A or (B and C) | +++ |
| B | ++ |
| C | + |

### 2. α-Amylase / α-Amylase variants

The present compositions and method relate to PcuAmyl α-amylase and variants, thereof. PcuAmyl is a predicted amylase from *Paenibacillus curdlanolyticus* YK9, for which the nucleotide sequence was determined by whole genome shotgun sequencing, performed by the United States Department of Energy Joint Genome Institute (Accession No. AEDD01000000). The present variant α-amylase enzymes were discovered using a combination of experimental approaches, including the use of site evaluation libraries (SELs) and structure-based analysis.

### 2.1 α-Amylase variants of PcuAmy1 α-amylase

In one aspect, methods of producing α-amylase enzymes are provided. The variant α-amylases have one or more mutations, as set forth herein, with respect to a parental α-amylase having 80% or greater amino acid sequence identity to the mature form of PcuAmyl α-amylase.

In some embodiments, the parental α-amylase is PcuAmyl α-amylase having the amino acid sequence of SEQ ID NO: 3 (residues Arg-177 and Gly-178 are underlined):

In some embodiments, the parental α-amylase is a variant PcuAmyl amylase (PcuAmyl-vl) having deletions of residues Arg-177 and Gly-178 and the amino acid sequence of SEQ ID NO: 4:

In addition to the mutation at position T333, the variant α-amylase may one or more further combinable mutations identified by making a site evaluation library (SEL) based on PcuAmyl-vl (SEQ ID NO: 4) and testing the resulting variants for various performance criteria, such as detergent stability, thermostability, cleaning performance, amylase activity, starch liquifaction, starch saccharification, and expression levels, the detailed procedures for which are described in the Examples or otherwise known. Each variant was assayed for the different enzymatic and biochemical properties, and characterized by a performance index (PI) value, which compared the relative performance of the variant to PcuAmy1-v1 amylase for each performance criteria. A PI that is greater than 1 (*i.e.,* PI>1) indicated improved performance by a variant as compared to PcuAmyl-vl, while a PI of 1 (*i.e.,* PI=1) indicated a variant that performed the same as the PcuAmyl-vl, and a PI that is less than 1 (*i.e.,* PI<1) indicated a variant that performed worse than the PcuAmyl-vl. PI values were then used to identify combinable mutations and productive positions.

Combinable mutations are mutations at any amino acid position that improve at least one desired property of the molecule, while not significantly decreasing expression, activity, or stability. Combinable mutations are assigned to one of three Groups (*i.e.,* A, B, or C), as set forth, herein. Combinable mutations are at productive positions. Productive positions are amino acid positions that are tolerant to substitution with different amino acid residues, wherein the resulting variants meet a set of performance criteria for combinability, as set forth herein.

Combinable mutations and productive positions are not to be confused with previously-identified, single-site mutations, some of which have subsequently been found by trial and error to work in combination with other mutations. Previously-identified, single-site mutations are invariably "winners" with respect to improving any one performance or stability feature. While this makes them attractive mutations to include in varient amylases, these "winners" tend to adversly affect other performance or stability features of the variants, which often requires making additional mutations to correct the defects.

In contrast, combinable mutations may be only incrementally beneficial in improving any one performance or stability feature of a variant amylase. However, they are carefully selected to be minimally detrimental to other desired performance or stability features, making them well suited for use in combination with other combinable mutations to contruct variant amylases having desired improved enzymatic and biochemical properties without being crippled in others, resulting in robust variants having a good balance of performance, stability, and expression potential.

Further based on measured enzymatic and biochemical properties of the variant amylases, the suitability scores of the different mutations for making combinatorial variants were determined. The suitabilty score refers to the ability of one or more combinable mutations to be used to make combinatorial variants, based on the performance criteria for combinability (*i.e.,* A, B, and C, as set forth, above), in which each of the mutations fall.

The suitability scores of individual substitutions in PcuAmyl-vl are shown in Table 3. The position numbering is based on the amino acid sequence of the mature PcuAmyl polypeptide (SEQ ID NO: 3). Wild-type residues at the indicated positions are given a suitability score of +++. Substitutions more likely to be combinable with other mutations are given a suitability score of ++++, or even +++++.

| **Table 3: Suitability scores of individual substitutions in PcuAmy1-v1** | | | | | |
|---|---|---|---|---|---|
| POS | VARIANTS SUITABILITY SCORE | | | | |
| | (+) | (++) | (+++) WT AA 1^{ST}* | (++++) | (+++++) |
| 1 | | W | G | DFHINSV | EKMR |
| 2 | HNV | M | DY | E | A |
| 3 | CR | S | N | | |
| 4 | | | GMY | DFINQSW | EHLPV |
| 5 | P | IS | T | | |
| 6 | | | ICF | | |
| 9 | DNQ | V | YM | FHTW | AGS |
| 10 | | LV | FN | M | |
| 14 | HQ | | LS | ACDEMN | RT |
| 15 | | | PDELMNRSVW | AH | |
| 16 | | AQR | N | | |
| 17 | | V | DNQ | EGSY | KR |
| 19 | | | ADFHIMNTVY | K | |
| 20 | A | G | H | T | |
| 22 | | C | NS | ADEFGIKMQW | HRT |
| 23 | C | KVW | RDEIN | AM | |
| 24 | | AT | L | IMV | |
| 25 | C | | NAFQTY | E | |
| 26 | | A | NDEGHKLRV | Y | |
| 27 | C | FW | DIN | GMQSTV | EK |
| 28 | | | AGT | RS | |
| 29 | | | Q | ADFGIMNPRVWY | E |
| 30 | | | N | ADFGHLMPRSTVWY | Q |
| 31 | WY | | L | F | |
| 32 | | | KDGIMY | AS | |
| 33 | | W | N | DEHKQSTV | A |
| 34 | | | VADFGHLQRSTWY | | |
| 35 | S | | G | | |
| 36 | | | IFMV | | |
| 37 | C | V | TADEGS | K | Q |
| 38 | CMW | | AEHRST | DN | G |
| 39 | | T | V | LM | |
| 41 | G | | ILV | AS | |
| 44 | N | T | A | S | |
| 45 | | N | YFH | | |
| 47 | | S | G | A | |
| 48 | | DEIV | G | HT | K |
| 49 | LY | V | SEM | DGNT | |
| 50 | | HMV | SDENQ | GK | |
| 51 | | S | AEQV | GKLRW | |
| 53 | | T | VEN | | |
| 57 | C | L | VS | I | |
| 60 | HN | | TAE | GV | |
| 63 | | | LI | | |
| 65 | A | | EN | | |
| 67 | K | | NADGMQSVWY | EL | |
| 68 | GT | | QAS | | |
| 69 | EGSW | | K | H | R |
| 70 | W | Y | GDEFHK | | |
| 71 | LMR | D | TAS | | |
| 72 | | R | VTY | I | |
| 73 | W | | RHM | GPS | AE |
| 74 | I | V | T | | |
| 76 | H | | Y | W | |
| 78 | C | L | TDFHIQSV | AGKMNR | E |
| 79 | | MR | KAS | | |
| 80 | | | SCW | ADEFKMPRTV | G |
| 81 | ACDFGHMRTY | V | EQ | NS | |
| 82 | | I | L | V | |
| 83 | | HT | IDQS | AGMVWY | E |
| 84 | | AIKM | SEF | GPVWY | |
| 85 | L | | ACGMV | | |
| 86 | R | | VKST | AEILMQ | |
| 87 | | | NADFGLQRVY | ST | |
| 88 | | ST | NAM | CEIRVY | |
| 89 | | | LI | V | |
| 90 | | | HKQR | | |
| 91 | | CH | AEFLMSTVWY | IK | |
| 92 | S | | KADIMNQRTY | EG | |
| 93 | | | GT | ADEQRS | |
| 94 | | | IMV | | |
| 95 | | Q | ADGLMNT | FSWY | |
| 96 | | | VLT | AI | |
| 97 | N | M | Y | AFHILV | |
| 98 | E | | GA | | |
| 100 | | | VAIS | | |
| 101 | | | VEI | | |
| 102 | | | LAST | V | Q |
| 105 | AGST | M | RKL | | |
| 107 | IKMP | | NEW | AS | G |
| 110 | | CV | AKW | DEGHLNQRSTY | |
| 111 | C | L | TIK | ADFGMNSV | E |
| 112 | F | A | E | DMN | |
| 113 | | | LCIVY | DEFGNPQS | AW |
| 114 | | I | V | | |
| 115 | | FIMTW | DEHKR | AGNS | |
| 116 | | CG | AS | | |
| 117 | | | VAST | KR | |
| 120 | AR | S | D | N | |
| 121 | | AEQR | P | FST | |
| 122 | | | NHVW | ADEFGQRS | |
| 125 | | R | ND | CWY | |
| 126 | | CW | V | AELNQRST | |
| 127 | | R | EH | AFNPQSTV | K |
| 128 | | ACFSW | TM | R | IV |
| 129 | C | EQRV | TK | AG | |
| 130 | P | | SW | ADFGHIKLRTV | NQ |
| 131 | C | | T | DFGHIMNQRVW | AEPY |
| 132 | DGV | | Y | | EHQST |
| 133 | H | | QVY | DGIMNPRS | FW |
| 134 | | | I | V | |
| 135 | | | Q | ADEGHILMNTWY | RV |
| 136 | | I | AGT | | |
| 137 | | | W | Y | |
| 138 | | | TS | | |
| 139 | | | QADGKV | FLMNRWY | H |
| 140 | | I | Y | F | H |
| 141 | | AFRY | DCGIKQSV | EMW | |
| 142 | | | FY | | |
| 143 | | | PDGHIV | AFNQSW | E |
| 144 | CILY | DHKMPTV | G | AES | NQ |
| 146 | | P | GDEFV | AHKNSWY | MQ |
| 147 | | | NDFGHKQST | APR | |
| 148 | | DE | TAHMQVWY | KNR | |
| 149 | EGMV | | YIL | | F |
| 150 | | | SG | | |
| 151 | | QY | SPV | AEGHKNRT | DF |
| 152 | | Y | F | H | W |
| 153 | | FMS | KHQR | | |
| 155 | | T | RACEGNV | F | KY |
| 156 | HP | | W | | |
| 157 | | MR | Y | DFGIPQSVW | |
| 162 | ADEGHLQ | | V | C | |
| 164 | ACLMNQ | I | WT | | |
| 166 | CFKRS | AEN | QDP | GH | T |
| 167 | C | | SVWY | DEI | AFGHLQRT |
| 168 | | A | RK | LST | |
| 169 | | | GEQSTW | AFHR | DK |
| 170 | | | LK | AEGHQVWY | DRS |
| 171 | | DE | N | AS | |
| 172 | V | AW | RH | N | |
| 173 | | | IL | | |
| 174 | CE | H | Y | FW | M |
| 175 | | | K | | E |
| 176 | | A | LI | GNQ | H |
| 179 | | C | D | AEFKLMNRSTVWY | H |
| 180 | | AMNQY | DR | ET | |
| 181 | | | K | Q | H |
| 182 | | | DV | N | AEFHIKMRSTWY |
| 189 | | | S | AEGN | D |
| 190 | | | E | Q | |
| 191 | CMR | | Y | | HW |
| 192 | | | GAHMSVY | Q | E |
| 200 | | | AT | E | |
| 202 | | FN | L | | |
| 203 | A | | D | | C |
| 204 | | | F | CM | |
| 205 | C | | NIS | W | ADEFGMVY |
| 206 | L | | H | DEWY | ACMQ |
| 207 | | D | PAHLNT | EGRS | |
| 208 | | | DFGNQVY | ILRS | E |
| 210 | | | VIKM | QR | E |
| 211 | | I | NAFKSWY | HLMQRTV | |
| 212 | | S | E | AT | |
| 213 | V | | TAENS | Y | |
| 214 | | Y | KDFGILS | AMNQ | E |
| 215 | | | TADEFGILMNQSY | V | |
| 217 | | | GAS | | |
| 218 | | F | KAW | | |
| 220 | | | FMV | L | |
| 221 | | | VAL | | |
| 222 | | | NDGHQ | T | |
| 223 | | | TGRSVY | E | |
| 224 | | T | V | GIS | L |
| 225 | | CFIV | NDESW | AGMPY | |
| 226 | Y | V | LF | | |
| 227 | | | D | N | |
| 229 | | | VA | LW | I |
| 231 | CQSV | A | LT | M | |
| 234 | EN | | VGLS | AIT | |
| 237 | L | | I | | V |
| 238 | F | | KL | | CQW |
| 239 | Y | | F | | |
| 241 | G | | FCDEY | HN | IKMRSV |
| 242 | AY | | MCQ | EL | |
| 243 | V | | R | ACFGHQSWY | DEL |
| 244 | ACEGHNS | | D | | |
| 246 | | IMY | VT | A | |
| 247 | | G | N | ADEFMSV | QRW |
| 248 | | H | NCGIKVW | AEFMQRS | D |
| 249 | | | V | GN | EIM |
| 250 | | | R | | ADEFGHI MNTW |
| 251 | | | SADEFGLMNQTV | KW | |
| 252 | N | | TAEFGHKMQV | L | |
| 253 | E | | TGIKLMNPRSVY | | |
| 254 | | | GAEHIKMPQVY | R | |
| 255 | | | KADGIMNRSTVWY | | |
| 256 | | LY | NDGS | AM | |
| 257 | | | LAIKW | M | |
| 258 | G | K | FMPSW | AHTVY | |
| 259 | | N | AGHST | | |
| 260 | I | L | V | | |
| 261 | | A | G | S | |
| 264 | | | WLM | F | |
| 265 | W | EK | HDIRV | | AGM |
| 266 | G | D | YK | NPRSTW | ELMQV |
| 267 | GHK | S | D | | |
| 268 | R | W | VL | DEFGIY | AKMNPQ |
| 269 | | | NI | GHKY | ADEQST |
| 270 | A | ST | K | V | |
| 271 | | I | L | M | |
| 272 | | | NDEHIKQ | V | |
| 273 | | | SDEY | | |
| 274 | | F | Y | | |
| 275 | FWY | | ILV | M | |
| 276 | | K | TIQWY | ACEGL | D |
| 278 | | I | TV | | |
| 279 | DQV | HT | N | AGS | EW |
| 280 | | | GANQRSY | DKM | E |
| 281 | | | TDHQS | EKNR | A |
| 282 | | NY | MQ | EHILS | |
| 283 | CW | G | SAERY | DHKMT | |
| 285 | MW | Y | F | | |
| 287 | | T | VAIS | | |
| 289 | A | | L | | |
| 291 | | G | FQ | DIN | KMVY |
| 292 | | E | RK | | |
| 293 | AEL | | F | | |
| 294 | D | N | Y | | |
| 295 | | WY | D | AEHKNQ | |
| 297 | | | SAG | | |
| 298 | | F | NHIT | Q | E |
| 299 | | | GAKMQS | E | |
| 300 | | FV | GHWY | AEKQS | N |
| 302 | | W | GHMY | DENRSV | AK |
| 303 | | | YF | | |
| 306 | | | RS | | |
| 307 | | | NEG | FHKMQRST | ADLVW |
| 309 | K | | L | | |
| 310 | | | NDGHMV | KR | E |
| 311 | | Y | NADEGHQR | | |
| 313 | | | LM | | |
| 314 | | | MATV | S | |
| 315 | | | SDEGHILRTVY | MN | |
| 316 | | | SGLT | ADHINQVW | EY |
| 317 | | FWY | NAK | DEIV | Q |
| 318 | W | | PDHS | | A |
| 319 | | F | MW | DIKQRSVY | AEGT |
| 320 | | SY | KAEFL | | |
| 322 | Y | | V | | I |
| 331 | | | Q | E | A |
| 333 | | FQY | TN | S | ADEG |
| 337 | | | QD | E | |
| 338 | | T | S | | |
| 339 | | A | TE | | |
| 340 | | | V | I | |
| 341 | | HMNS | Q | EK | |
| 342 | | | SHPQR | K | |
| 345 | | V | K | | |
| 347 | | | LI | AMQ | |
| 348 | | | AG | | |
| 356 | | T | EGNW | DS | |
| 357 | | | Q | E | |
| 359 | | LS | Y | AFHMRTVW | DN |
| 361 | K | Q | C | AITV | E |
| 362 | A | | VM | L | I |
| 364 | | | YF | L | |
| 367 | M | | Y | | |
| 368 | | | YEFIKLNQRSTVW | | AD |
| 369 | | | GA | | |
| 370 | | | TS | | |
| 371 | | | SAEGY | DT | |
| 373 | | | GADEHQRS | | |
| 374 | | DM | KAEG | N | |
| 376 | V | | SEGHQ | AIRT | N |
| 377 | | | SALY | DEFGHPQRTV | N |
| 378 | | | YEGNT | SV | AD |
| 379 | | | KAGS | | |
| 380 | | | PDGWY | AEHKMRST | N |
| 381 | | | IAEFLT | Q | |
| 382 | | | M | I | |
| 383 | | | D | E | |
| 384 | CF | P | KAGMT | INSY | DV |
| 385 | | M | L | | |
| 386 | | T | LFV | M | |
| 387 | | I | NAGHQSTY | L | |
| 388 | | | ALTV | | |
| 390 | | | KHLQRY | | |
| 391 | | | VADFHLN | KM | |
| 392 | | | YF | | |
| 396 | | | TIPRSVY | EFHNQ | DLM |
| 398 | | Y | R | HN | |
| 401 | | | F | M | |
| 402 | | | D | N | |
| 403 | | | HD | | |
| 404 | CLS | GV | PW | AEMNQ | |
| 405 | | | D | N | |
| 406 | T | M | ILV | | |
| 407 | | | VI | | |
| 412 | D | AGLT | E | MQ | |
| 414 | E | | D | | |
| 415 | | | ADEGMR | KQ | |
| 416 | | | AY | DEFGIKLMNPQTV | |
| 418 | | | ASWY | EGHMRTV | KLNQ |
| 419 | | V | GH | DEFKMQRSTWY | |
| 420 | A | | S | | |
| 425 | | | LM | | |
| 426 | | | I | V | |
| 427 | | | T | DN | A |
| 430 | CH | F | PM | DESVY | AGIK |
| 431 | DN | KR | GV | S | AEPQT |
| 432 | A | | G | | |
| 433 | | | SD | HQR | |
| 435 | | D | WAFKSY | EHNQR | LM |
| 440 | | | TILMNSV | ADEH | |
| 441 | | R | SDEGHQ | | |
| 442 | Q | R | K | | HN |
| 443 | | K | A | | |
| 444 | D | HK | GN | | |
| 446 | | D | V | AEHILRST | MN |
| 448 | | | THS | R | |
| 450 | MRY | | K | HQ | |
| 451 | | L | T | | |
| 452 | AM | R | GH | Q | N |
| 453 | | | NH | | |
| 454 | C | EK | RQW | AFNSVY | L |
| 455 | | D | SEPQT | AHR | |
| 456 | | | GEN | D | |
| 457 | | | TEGNY | KSV | |
| 459 | | V | T | KQR | |
| 461 | S | E | DQ | G | |
| 462 | C | | AV | GPT | DEKMR |
| 463 | | | NAGHRSTY | EQ | |
| 465 | | | WY | | |
| 467 | RV | | NEHKLMQ | | |
| 469 | | | WADEGHNPSVY | K | |
| 471 | | A | NEGR | DKQ | |
| 472 | F | | G | DHK | AENT |
| 473 | V | | GFY | R | ADEKQS |
| 474 | | G | S | | |
| 475 | | T | V | | |
| 477 | | | VL | | |
| 480 | MV | IW | KP | EGSY | |

| | | | | | |
|---|---|---|---|---|---|
| * The wild-type amino acid residue is listed first | | | | | |

While evaluating mutations based on suitabilility score represents one refined aspect of the present compositions and methods, the identification of productive positions, which are tolerant to substitution with different amino acid residues, represents a number of significant embodiments.

Each productive position identified in the following lists with specified criteria, and each substitution identified in parenthesis following the numerical position identifier in each of these lists, represents a mutation, identified by experimental data, that either directly contributes to the performance of an amylase variant, or is determined to be combinable with other mutations to produce a amylase variant with improved performance.

The productive positions in PcuAmyl-vl that fall within the previously described Productivity Scores of "1," "2," "3," and "4," and the substitutions within those positions that are combinable, are listed below. The position numbering is based on the mature PcuAmyl polypeptide (SEQ ID NO: 3):
LIST A:
1 (G,D,E,F,H,I,K,M,N,R,S,V,W); 2 (D,A,E,H,M,N,V,Y); 3 (N,C,R,S); 4 (G,D,E,F,H,I,L,M,N,P,Q,S,V,W,Y); 5 (T,I,P,S); 6 (I,C,F); 9 (Y,A,D,F,G,H,M,N,Q,S,T,V,W); 10 (F,L,M,N,V); 14 (L,A,C,D,E,H,M,N,Q,R,S,T); 15 (P,A,D,E,H,L,M,N,R,S,V,W); 16 (N,A,Q,R); 17 (D,E,G,K,N,Q,R,S,V,Y); 19 (A,D,F,H,I,K,M,N,T,V,Y); 20 (H,A,G,T); 22 (N,A,C,D,E,F,G,H,I,K,M,Q,R,S,T,W); 23 (R,A,C,D,E,I,K,M,N,V,W); 24 (L,A,I,M,T,V); 25 (N,A,C,E,F,Q,T,Y); 26 (N,A,D,E,G,H,K,L,R,V,Y); 27 (D,C,E,F,G,I,K,M,N,Q,S,T,V,W); 28 (A,G,R,S,T); 29 (Q,A,D,E,F,G,I,M,N,P,R,V,W,Y); 30 (N,A,D,F,G,H,L,M,P,Q,R,S,T,V,W,Y); 31 (L,F,W,Y); 32 (K,A,D,G,I,M,S,Y); 33 (N,A,D,E,H,K,Q,S,T,V,W); 34 (V,A,D,F,G,H,L,Q,R,S,T,W,Y); 35 (G,S); 36 (I,F,M,V); 37 (T,A,C,D,E,G,K,Q,S,V); 38 (A,C,D,E,G,H,M,N,R,S,T,W); 39 (V,L,M,T); 41 (I,A,G,L,S,V); 44 (A,N,S,T); 45 (Y,F,H,N); 47 (G,A,S); 48 (G,D,E,H,I,K,T,V); 49 (S,D,E,G,L,M,N,T,V,Y); 50 (S,D,E,G,H,K,M,N,Q,V); 51 (A,E,G,K,L,Q,R,S,V,W); 53 (V,E,N,T); 57 (V,C,I,L,S); 60 (T,A,E,G,H,N,V); 63 (L,I); 65 (E,A,N); 67 (N,A,D,E,G,K,L,M,Q,S,V,W,Y); 68 (Q,A,G,S,T); 69 (K,E,G,H,R,S,W); 70 (G,D,E,F,H,K,W,Y); 71 (T,A,D,L,M,R,S); 72 (V,I,R,T,Y); 73 (R,A,E,G,H,M,P,S,W); 74 (T,I,V); 76 (Y,H,W); 78 (T,A,C,D,E,F,G,H,I,K,L,M,N,Q,R,S,V); 79 (K,A,M,R,S); 80 (S,A,C,D,E,F,G,K,M,P,R,T,V,W); 81 (E,A,C,D,F,G,H,M,N,Q,R,S,T,V,Y); 82 (L,I,V); 83 (I,A,D,E,G,H,M,Q,S,T,V,W,Y); 84 (S,A,E,F,G,I,K,M,P,V,W,Y); 85 (A,C,G,L,M,V); 86 (V,A,E,I,K,L,M,Q,R,S,T); 87 (N,A,D,F,G,L,Q,R,S,T,V,Y); 88 (N,A,C,E,I,M,R,S,T,V,Y); 89 (L,I,V); 90 (H,K,Q,R); 91 (A,C,E,F,H,I,K,L,M,S,T,V,W,Y); 92 (K,A,D,E,G,I,M,N,Q,R,S,T,Y); 93 (G,A,D,E,Q,R,S,T); 94 (I,M,V); 95 (A,D,F,G,L,M,N,Q,S,T,W,Y); 96 (V,A,I,L,T); 97 (Y,A,F,H,I,L,M,N,V); 98 (G,A,E); 100 (V,A,I,S); 101 (V,E,I); 102 (L,A,Q,S,T,V); 105 (R,A,G,K,L,M,S,T); 107 (N,A,E,G,I,K,M,P,S,W); 110 (A,C,D,E,G,H,K,L,N,Q,R,S,T,V,W,Y); 111 (T,A,C,D,E,F,G,I,K,L,M,N,S,V); 112 (E,A,D,F,M,N); 113 (L,A,C,D,E,F,G,I,N,P,Q,S,V,W,Y); 114 (V,I); 115 (D,A,E,F,G,H,I,K,M,N,R,S,T,W); 116 (A,C,G,S); 117 (V,A,K,R,S,T); 120 (D,A,N,R,S); 121 (P,A,E,F,Q,R,S,T); 122 (N,A,D,E,F,G,H,Q,R,S,V,W); 125 (N,C,D,R,W,Y); 126 (V,A,C,E,L,N,Q,R,S,T,W); 127 (E,A,F,H,K,N,P,Q,R,S,T,V); 128 (T,A,C,F,I,M,R,S,V,W); 129 (T,A,C,E,G,K,Q,R,V); 130 (S,A,D,F,G,H,I,K,L,N,P,Q,R,T,V,W); 131 (T,A,C,D,E,F,G,H,I,M,N,P,Q,R,V,W,Y); 132 (Y,D,E,G,H,Q,S,T,V); 133 (Q,D,F,G,H,I,M,N,P,R,S,V,W,Y); 134 (I,V); 135 (Q,A,D,E,G,H,I,L,M,N,R,T,V,W,Y); 136 (A,G,I,T); 137 (W,Y); 138 (T,S); 139 (Q,A,D,F,G,H,K,L,M,N,R,V,W,Y); 140 (Y,F,H,I); 141 (D,A,C,E,F,G,I,K,M,Q,R,S,V,W,Y); 142 (F,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 147 (N,A,D,F,G,H,K,P,Q,R,S,T); 148 (T,A,D,E,H,K,M,N,Q,R,V,W,Y); 149 (Y,E,F,G,I,L,M,V); 150 (S,G); 151 (S,A,D,E,F,G,H,K,N,P,Q,R,T,V,Y); 152 (F,H,W,Y); 153 (K,F,H,M,Q,R,S); 155 (R,A,C,E,F,G,K,N,T,V,Y); 156 (W,H,P); 157 (Y,D,F,G,I,M,P,Q,R,S,V,W); 162 (V,A,C,D,E,G,H,L,Q); 164 (W,A,C,I,L,M,N,Q,T); 166 (Q,A,C,D,E,F,G,H,K,N,P,R,S,T); 167 (S,A,C,D,E,F,G,H,I,L,Q,R,T,V,W,Y); 168 (R,A,K,L,S,T); 169 (G,A,D,E,F,H,K,Q,R,S,T,W); 170 (L,A,D,E,G,H,K,Q,R,S,V,W,Y); 171 (N,A,D,E,S); 172 (R,A,H,N,V,W); 173 (I,L); 174 (Y,C,E,F,H,M,W); 175 (K,E); 176 (L,A,G,H,I,N,Q); 179 (D,A,C,E,F,H,K,L,M,N,R,S,T,V,W,Y); 180 (D,A,E,M,N,Q,R,T,Y); 181 (K,H,Q); 182 (D,A,E,F,H,I,K,M,N,R,S,T,V,W,Y); 189 (S,A,D,E,G,N); 190 (E,Q); 191 (Y,C,H,M,R,W); 192 (G,A,E,H,M,Q,S,V,Y); 200 (A,E,T); 202 (L,F,N); 203 (D,A,C); 204 (F,C,M); 205 (N,A,C,D,E,F,G,I,M,S,V,W,Y); 206 (H,A,C,D,E,L,M,Q,W,Y); 207 (P,A,D,E,G,H,L,N,R,S,T); 208 (D,E,F,G,I,L,N,Q,R,S,V,Y); 210 (V,E,I,K,M,Q,R); 211 (N,A,F,H,I,K,L,M,Q,R,S,T,V,W,Y); 212 (E,A,S,T); 213 (T,A,E,N,S,V,Y); 214 (K,A,D,E,F,G,I,L,M,N,Q,S,Y); 215 (T,A,D,E,F,G,I,L,M,N,Q,S,V,Y); 217 (G,A,S); 218 (K,A,F,W); 220 (F,L,M,V); 221 (V,A,L); 222 (N,D,G,H,Q,T); 223 (T,E,G,R,S,V,Y); 224 (V,G,I,L,S,T); 225 (N,A,C,D,E,F,G,I,M,P,S,V,W,Y); 226 (L,F,V,Y); 227 (D,N); 229 (V,A,I,L,W); 231 (L,A,C,M,Q,S,T,V); 234 (V,A,E,G,I,L,N,S,T); 237 (I,L,V); 238 (K,C,F,L,Q,W); 239 (F,Y); 241 (F,C,D,E,G,H,I,K,M,N,R,S,V,Y); 242 (M,A,C,E,L,Q,Y); 243 (R,A,C,D,E,F,G,H,L,Q,S,V,W,Y); 244 (D,A,C,E,G,H,N,S); 246 (V,A,I,M,T,Y); 247 (N,A,D,E,F,G,M,Q,R,S,V,W); 248 (N,A,C,D,E,F,G,H,I,K,M,Q,R,S,V,W); 249 (V,E,G,I,M,N); 250 (R,A,D,E,F,G,H,I,M,N,T,W); 251 (S,A,D,E,F,G,K,L,M,N,Q,T,V,W); 252 (T,A,E,F,G,H,K,L,M,N,Q,V); 253 (T,E,G,I,K,L,M,N,P,R,S,V,Y); 254 (G,A,E,H,I,K,M,P,Q,R,V,Y); 255 (K,A,D,G,I,M,N,R,S,T,V,W,Y); 256 (N,A,D,G,L,M,S,Y); 257 (L,A,I,K,M,W); 258 (F,A,G,H,K,M,P,S,T,V,W,Y); 259 (A,G,H,N,S,T); 260 (V,I,L); 261 (G,A,S); 264 (W,F,L,M); 265 (H,A,D,E,G,I,K,M,R,V,W); 266 (Y,D,E,G,K,L,M,N,P,Q,R,S,T,V,W); 267 (D,G,H,K,S); 268 (V,A,D,E,F,G,I,K,L,M,N,P,Q,R,W,Y); 269 (N,A,D,E,G,H,I,K,Q,S,T,Y); 270 (K,A,S,T,V); 271 (L,I,M); 272 (N,D,E,H,I,K,Q,V); 273 (S,D,E,Y); 274 (Y,F); 275 (I,F,L,M,V,W,Y); 276 (T,A,C,D,E,G,I,K,L,Q,W,Y); 278 (T,I,V); 279 (N,A,D,E,G,H,Q,S,T,V,W); 280 (G,A,D,E,K,M,N,Q,R,S,Y); 281 (T,A,D,E,H,K,N,Q,R,S); 282 (M,E,H,I,L,N,Q,S,Y); 283 (S,A,C,D,E,G,H,K,M,R,T,W,Y); 285 (F,M,W,Y); 287 (V,A,I,S,T); 289 (L,A); 291 (F,D,G,I,K,M,N,Q,V,Y); 292 (R,E,K); 293 (F,A,E,L); 294 (Y,D,N); 295 (D,A,E,H,K,N,Q,W,Y); 297 (S,A,G); 298 (N,E,F,H,I,Q,T); 299 (G,A,E,K,M,Q,S); 300 (G,A,E,F,H,K,N,Q,S,V,W,Y); 302 (G,A,D,E,H,K,M,N,R,S,V,W,Y); 303 (Y,F); 306 (R,S); 307 (N,A,D,E,F,G,H,K,L,M,Q,R,S,T,V,W); 309 (L,K); 310 (N,D,E,G,H,K,M,R,V); 311 (N,A,D,E,G,H,Q,R,Y); 313 (L,M); 314 (M,A,S,T,V); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 318 (P,A,D,H,S,W); 319 (M,A,D,E,F,G,I,K,Q,R,S,T,V,W,Y); 320 (K,A,E,F,L,S,Y); 322 (V,I,Y); 331 (Q,A,E); 333 (T,A,D,E,F,G,N,Q,S,Y); 337 (Q,D,E); 338 (S,T); 339 (T,A,E); 340 (V,I); 341 (Q,E,H,K,M,N,S); 342 (S,H,K,P,Q,R); 345 (K,V); 347 (L,A,I,M,Q); 348 (A,G); 356 (E,D,G,N,S,T,W); 357 (Q,E); 359 (Y,A,D,F,H,L,M,N,R,S,T,V,W); 361 (C,A,E,I,K,Q,T,V); 362 (V,A,I,L,M); 364 (Y,F,L); 367 (Y,M); 368 (Y,A,D,E,F,I,K,L,N,Q,R,S,T,V,W); 369 (G,A); 370 (T,S); 371 (S,A,D,E,G,T,Y); 373 (G,A,D,E,H,Q,R,S); 374 (K,A,D,E,G,M,N); 376 (S,A,E,G,H,I,N,Q,R,T,V); 377 (S,A,D,E,F,G,H,L,N,P,Q,R,T,V,Y); 378 (Y,A,D,E,G,N,S,T,V); 379 (K,A,G,S); 380 (P,A,D,E,G,H,K,M,N,R,S,T,W,Y); 381 (I,A,E,F,L,Q,T); 382 (M,I); 383 (D,E); 384 (K,A,C,D,F,G,I,M,N,P,S,T,V,Y); 385 (L,M); 386 (L,F,M,T,V); 387 (N,A,G,H,I,L,Q,S,T,Y); 388 (A,L,T,V); 390 (K,H,L,Q,R,Y); 391 (V,A,D,F,H,K,L,M,N); 392 (Y,F); 396 (T,D,E,F,H,I,L,M,N,P,Q,R,S,V,Y); 398 (R,H,N,Y); 401 (F,M); 402 (D,N); 403 (H,D); 404 (P,A,C,E,G,L,M,N,Q,S,V,W); 405 (D,N); 406 (I,L,M,T,V); 407 (V,I); 412 (E,A,D,G,L,M,Q,T); 414 (D,E); 415 (A,D,E,G,K,M,Q,R); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 420 (S,A); 425 (L,M); 426 (I,V); 427 (T,A,D,N); 430 (P,A,C,D,E,F,G,H,I,K,M,S,V,Y); 431 (G,A,D,E,K,N,P,Q,R,S,T,V); 432 (G,A); 433 (S,D,H,Q,R); 435 (W,A,D,E,F,H,K,L,M,N,Q,R,S,Y); 440 (T,A,D,E,H,I,L,M,N,S,V); 441 (S,D,E,G,H,Q,R); 442 (K,H,N,Q,R); 443 (A,K); 444 (G,D,H,K,N); 446 (V,A,D,E,H,I,L,M,N,R,S,T); 448 (T,H,R,S); 450 (K,H,M,Q,R,Y); 451 (T,L); 452 (G,A,H,M,N,Q,R); 453 (N,H); 454 (R,A,C,E,F,K,L,N,Q,S,V,W,Y); 455 (S,A,D,E,H,P,Q,R,T); 456 (G,D,E,N); 457 (T,E,G,K,N,S,V,Y); 459 (T,K,Q,R,V); 461 (D,E,G,Q,S); 462 (A,C,D,E,G,K,M,P,R,T,V); 463 (N,A,E,G,H,Q,R,S,T,Y); 465 (W,Y); 467 (N,E,H,K,L,M,Q,R,V); 469 (W,A,D,E,G,H,K,N,P,S,V,Y); 471 (N,A,D,E,G,K,Q,R); 472 (G,A,D,E,F,H,K,N,T); 473 (G,A,D,E,F,K,Q,R,S,V,Y); 474 (S,G); 475 (V,T); 477 (V,L); and 480 (K,E,G,I,M,P,S,V,W,Y).

The productive positions in PcuAmyl-vl that fall within the previously described Productivity Scores of "2," "3," and "4," and the substitutions within those positions that are combinable, are listed below. The position numbering is based on the mature PcuAmyl polypeptide (SEQ ID NO: 3):
LIST B:
1 (G,D,E,F,H,I,K,M,N,R,S,V,W); 2 (D,A,E,H,M,N,V,Y); 3 (N,C,R,S); 4 (G,D,E,F,H,I,L,M,N,P,Q,S,V,W,Y); 5 (T,I,P,S); 6 (I,C,F); 9 (Y,A,D,F,G,H,M,N,Q,S,T,V,W); 10 (F,L,M,N,V); 14 (L,A,C,D,E,H,M,N,Q,R,S,T); 15 (P,A,D,E,H,L,M,N,R,S,V,W); 16 (N,A,Q,R); 17 (D,E,G,K,N,Q,R,S,V,Y); 19 (A,D,F,H,I,K,M,N,T,V,Y); 20 (H,A,G,T\); 22 (N,A,C,D,E,F,G,H,I,K,M,Q,R,S,T,W); 23 (R,A,C,D,E,I,K,M,N,V,W); 24 (L,A,I,M,T,V); 25 (N,A,C,E,F,Q,T,Y); 26 (N,A,D,E,G,H,K,L,R,V,Y); 27 (D,C,E,F,G,I,K,M,N,Q,S,T,V,W); 28 (A,G,R,S,T); 29 (Q,A,D,E,F,G,I,M,N,P,R,V,W,Y); 30 (N,A,D,F,G,H,L,M,P,Q,R,S,T,V,W,Y); 31 (L,F,W,Y); 32 (K,A,D,G,I,M,S,Y); 33 (N,A,D,E,H,K,Q,S,T,V,W); 34 (V,A,D,F,G,H,L,Q,R,S,T,W,Y); 36 (I,F,M,V); 37 (T,A,C,D,E,G,K,Q,S,V); 38 (A,C,D,E,G,H,M,N,R,S,T,W); 39 (V,L,M,T); 41 (I,A,G,L,S,V); 44 (A,N,S,T\); 45 (Y,F,H,N); 48 (G,D,E,H,I,K,T,V); 49 (S,D,E,G,L,M,N,T,V,Y); 50 (S,D,E,G,H,K,M,N,Q,V); 51 (A,E,G,K,L,Q,R,S,V,W); 53 (V,E,N,T); 57 (V,C,I,L,S); 60 (T,A,E,G,H,N,V); 67 (N,A,D,E,G,K,L,M,Q,S,V,W,Y); 68 (Q,A,G,S,T); 69 (K,E,G,H,R,S,W); 70 (G,D,E,F,H,K,W,Y); 71 (T,A,D,L,M,R,S); 72 (V,I,R,T,Y); 73 (R,A,E,G,H,M,P,S,W); 78 (T,A,C,D,E,F,G,H,I,K,L,M,N,Q,R,S,V); 79 (K,A,M,R,S); 80 (S,A,C,D,E,F,G,K,M,P,R,T,V,W); 81 (E,A,C,D,F,G,H,M,N,Q,R,S,T,V,Y); 83 (I,A,D,E,G,H,M,Q,S,T,V,W,Y); 84 (S,A,E,F,G,I,K,M,P,V,W,Y); 85 (A,C,G,L,M,V); 86 (V,A,E,I,K,L,M,Q,R,S,T); 87 (N,A,D,F,G,L,Q,R,S,T,V,Y); 88 (N,A,C,E,I,M,R,S,T,V,Y); 89 (L,I,V); 90 (H,K,Q,R); 91 (A,C,E,F,H,I,K,L,M,S,T,V,W,Y); 92 (K,A,D,E,G,I,M,N,Q,R,S,T,Y); 93 (G,A,D,E,Q,R,S,T); 95 (A,D,F,G,L,M,N,Q,S,T,W,Y); 96 (V,A,I,L,T); 97 (Y,A,F,H,I,L,M,N,V); 100 (V,A,I,S); 102 (L,A,Q,S,T,V); 105 (R,A,G,K,L,M,S,T); 107 (N,A,E,G,I,K,M,P,S,W); 110 (A,C,D,E,G,H,K,L,N,Q,R,S,T,V,W,Y); 111 (T,A,C,D,E,F,G,I,K,L,M,N,S,V); 112 (E,A,D,F,M,N); 113 (L,A,C,D,E,F,G,I,N,P,Q,S,V,W,Y); 115 (D,A,E,F,G,H,I,K,M,N,R,S,T,W); 116 (A,C,G,S); 117 (V,A,K,R,S,T); 120 (D,A,N,R,S); 121 (P,A,E,F,Q,R,S,T); 122 (N,A,D,E,F,G,H,Q,R,S,V,W); 125 (N,C,D,R,W,Y); 126 (V,A,C,E,L,N,Q,R,S,T,W); 127 (E,A,F,H,K,N,P,Q,R,S,T,V); 128 (T,A,C,F,I,M,R,S,V,W); 129 (T,A,C,E,G,K,Q,R,V); 130 (S,A,D,F,G,H,I,K,L,N,P,Q,R,T,V,W); 131 (T,A,C,D,E,F,G,H,I,M,N,P,Q,R,V,W,Y); 132 (Y,D,E,G,H,Q,S,T,V); 133 (Q,D,F,G,H,I,M,N,P,R,S,V,W,Y); 135 (Q,A,D,E,G,H,I,L,M,N,R,T,V,W,Y); 136 (A,G,I,T); 139 (Q,A,D,F,G,H,K,L,M,N,R,V,W,Y); 140 (Y,F,H,I); 141 (D,A,C,E,F,G,I,K,M,Q,R,S,V,W,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 147 (N,A,D,F,G,H,K,P,Q,R,S,T); 148 (T,A,D,E,H,K,M,N,Q,R,V,W,Y); 149 (Y,E,F,G,I,L,M,V); 151 (S,A,D,E,F,G,H,K,N,P,Q,R,T,V,Y); 152 (F,H,W,Y); 153 (K,F,H,M,Q,R,S); 155 (R,A,C,E,F,G,K,N,T,V,Y); 157 (Y,D,F,G,I,M,P,Q,R,S,V,W); 162 (V,A,C,D,E,G,H,L,Q); 164 (W,A,C,I,L,M,N,Q,T); 166 (Q,A,C,D,E,F,G,H,K,N,P,R,S,T); 167 (S,A,C,D,E,F,G,H,I,L,Q,R,T,V,W,Y); 168 (R,A,K,L,S,T); 169 (G,A,D,E,F,H,K,Q,R,S,T,W); 170 (L,A,D,E,G,H,K,Q,R,S,V,W,Y); 171 (N,A,D,E,S); 172 (R,A,H,N,V,W); 174 (Y,C,E,F,H,M,W); 176 (L,A,G,H,I,N,Q); 179 (D,A,C,E,F,H,K,L,M,N,R,S,T,V,W,Y); 180 (D,A,E,M,N,Q,R,T,Y); 182 (D,A,E,F,H,I,K,M,N,R,S,T,V,W,Y); 189 (S,A,D,E,G,N); 191 (Y,C,H,M,R,W); 192 (G,A,E,H,M,Q,S,V,Y); 204 (F,C,M); 205 (N,A,C,D,E,F,G,I,M,S,V,W,Y); 206 (H,A,C,D,E,L,M,Q,W,Y); 207 (P,A,D,E,G,H,L,N,R,S,T); 208 (D,E,F,G,I,L,N,Q,R,S,V,Y); 210 (V,E,I,K,M,Q,R); 211 (N,A,F,H,I,K,L,M,Q,R,S,T,V,W,Y); 212 (E,A,S,T); 213 (T,A,E,N,S,V,Y); 214 (K,A,D,E,F,G,I,L,M,N,Q,S,Y); 215 (T,A,D,E,F,G,I,L,M,N,Q,S,V,Y); 218 (K,A,F,W); 220 (F,L,M,V); 222 (N,D,G,H,Q,T); 223 (T,E,G,R,S,V,Y); 224 (V,G,I,L,S,T); 225 (N,A,C,D,E,F,G,I,M,P,S,V,W,Y); 226 (L,F,V,Y); 229 (V,A,I,L,W); 231 (L,A,C,M,Q,S,T,V); 234 (V,A,E,G,I,L,N,S,T); 238 (K,C,F,L,Q,W); 241 (F,C,D,E,G,H,I,K,M,N,R,S,V,Y); 242 (M,A,C,E,L,Q,Y); 243 (R,A,C,D,E,F,G,H,L,Q,S,V,W,Y); 244 (D,A,C,E,G,H,N,S); 246 (V,A,I,M,T,Y); 247 (N,A,D,E,F,G,M,Q,R,S,V,W); 248 (N,A,C,D,E,F,G,H,I,K,M,Q,R,S,V,W); 249 (V,E,G,I,M,N); 250 (R,A,D,E,F,G,H,I,M,N,T,W); 251 (S,A,D,E,F,G,K,L,M,N,Q,T,V,W); 252 (T,A,E,F,G,H,K,L,M,N,Q,V); 253 (T,E,G,I,K,L,M,N,P,R,S,V,Y); 254 (G,A,E,H,I,K,M,P,Q,R,V,Y); 255 (K,A,D,G,I,M,N,R,S,T,V,W,Y); 256 (N,A,D,G,L,M,S,Y); 257 (L,A,I,K,M,W); 258 (F,A,G,H,K,M,P,S,T,V,W,Y); 259 (A,G,H,N,S,T); 264 (W,F,L,M); 265 (H,A,D,E,G,I,K,M,R,V,W); 266 (Y,D,E,G,K,L,M,N,P,Q,R,S,T,V,W); 267 (D,G,H,K,S); 268 (V,A,D,E,F,G,I,K,L,M,N,P,Q,R,W,Y); 269 (N,A,D,E,G,H,I,K,Q,S,T,Y); 270 (K,A,S,T,V); 272 (N,D,E,H,I,K,Q,V); 273 (S,D,E,Y); 275 (I,F,L,M,V,W,Y); 276 (T,A,C,D,E,G,I,K,L,Q,W,Y); 278 (T,I,V); 279 (N,A,D,E,G,H,Q,S,T,V,W); 280 (G,A,D,E,K,M,N,Q,R,S,Y); 281 (T,A,D,E,H,K,N,Q,R,S); 282 (M,E,H,I,L,N,Q,S,Y); 283 (S,A,C,D,E,G,H,K,M,R,T,W,Y); 285 (F,M,W,Y); 287 (V,A,I,S,T); 291 (F,D,G,I,K,M,N,Q,V,Y); 292 (R,E,K); 293 (F,A,E,L); 295 (D,A,E,H,K,N,Q,W,Y); 298 (N,E,F,H,I,Q,T); 299 (G,A,E,K,M,Q,S); 300 (G,A,E,F,H,K,N,Q,S,V,W,Y); 302 (G,A,D,E,H,K,M,N,R,S,V,W,Y); 307 (N,A,D,E,F,G,H,K,L,M,Q,R,S,T,V,W); 310 (N,D,E,G,H,K,M,R,V); 311 (N,A,D,E,G,H,Q,R,Y); 314 (M,A,S,T,V); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 318 (P,A,D,H,S,W); 319 (M,A,D,E,F,G,I,K,Q,R,S,T,V,W,Y); 320 (K,A,E,F,L,S,Y); 333 (T,A,D,E,F,G,N,Q,S,Y); 341 (Q,E,H,K,M,N,S); 342 (S,H,K,P,Q,R); 347 (L,A,I,M,Q); 356 (E,D,G,N,S,T,W); 359 (Y,A,D,F,H,L,M,N,R,S,T,V,W); 361 (C,A,E,I,K,Q,T,V); 362 (V,A,I,L,M); 368 (Y,A,D,E,F,I,K,L,N,Q,R,S,T,V,W); 371 (S,A,D,E,G,T,Y); 373 (G,A,D,E,H,Q,R,S); 374 (K,A,D,E,G,M,N); 376 (S,A,E,G,H,I,N,Q,R,T,V); 377 (S,A,D,E,F,G,H,L,N,P,Q,R,T,V,Y); 378 (Y,A,D,E,G,N,S,T,V); 379 (K,A,G,S); 380 (P,A,D,E,G,H,K,M,N,R,S,T,W,Y); 381 (I,A,E,F,L,Q,T); 384 (K,A,C,D,F,G,I,M,N,P,S,T,V,Y); 386 (L,F,M,T,V); 387 (N,A,G,H,I,L,Q,S,T,Y); 388 (A,L,T,V); 390 (K,H,L,Q,R,Y); 391 (V,A,D,F,H,K,L,M,N); 396 (T,D,E,F,H,I,L,M,N,P,Q,R,S,V,Y); 398 (R,H,N,Y); 404 (P,A,C,E,G,L,M,N,Q,S,V,W); 406 (I,L,M,T,V); 412 (E,A,D,G,L,M,Q,T); 415 (A,D,E,G,K,M,Q,R); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 427 (T,A,D,N); 430 (P,A,C,D,E,F,G,H,I,K,M,S,V,Y); 431 (G,A,D,E,K,N,P,Q,R,S,T,V); 433 (S,D,H,Q,R); 435 (W,A,D,E,F,H,K,L,M,N,Q,R,S,Y); 440 (T,A,D,E,H,I,L,M,N,S,V); 441 (S,D,E,G,H,Q,R); 442 (K,H,N,Q,R); 444 (G,D,H,K,N); 446 (V,A,D,E,H,I,L,M,N,R,S,T); 448 (T,H,R,S); 450 (K,H,M,Q,R,Y); 452 (G,A,H,M,N,Q,R); 454 (R,A,C,E,F,K,L,N,Q,S,V,W,Y); 455 (S,A,D,E,H,P,Q,R,T); 456 (G,D,E,N); 457 (T,E,G,K,N,S,V,Y); 459 (T,K,Q,R,V); 461 (D,E,G,Q,S); 462 (A,C,D,E,G,K,M,P,R,T,V); 463 (N,A,E,G,H,Q,R,S,T,Y); 467 (N,E,H,K,L,M,Q,R,V); 469 (W,A,D,E,G,H,K,N,P,S,V,Y); 471 (N,A,D,E,G,K,Q,R); 472 (G,A,D,E,F,H,K,N,T); 473 (G,A,D,E,F,K,Q,R,S,V,Y); and 480 (K,E,G,I,M,P,S,V,W,Y).

The productive positions in PcuAmyl-vl that fall within the previously described Productivity Scores of "3" and "4," and the substitutions within those positions that are combinable, are listed below. The position numbering is based on the mature PcuAmyl polypeptide (SEQ ID NO: 3):
LIST C:
1 (G,D,E,F,H,I,K,M,N,R,S,V,W); 2 (D,A,E,H,M,N,V,Y); 4 (G,D,E,F,H,I,L,M,N,P,Q,S,V,W,Y); 9 (Y,A,D,F,G,H,M,N,Q,S,T,V,W); 14 (L,A,C,D,E,H,M,N,Q,R,S,T); 15 (P,A,D,E,H,L,M,N,R,S,V,W); 17 (D,E,G,K,N,Q,R,S,V,Y); 19 (A,D,F,H,I,K,M,N,T,V,Y); 22 (N,A,C,D,E,F,G,H,I,K,M,Q,R,S,T,W); 23 (R,A,C,D,E,I,K,M,N,V,W); 25 (N,A,C,E,F,Q,T,Y); 26 (N,A,D,E,G,H,K,L,R,V,Y); 27 (D,C,E,F,G,I,K,M,N,Q,S,T,V,W); 29 (Q,A,D,E,F,G,I,M,N,P,R,V,W,Y); 30 (N,A,D,F,G,H,L,M,P,Q,R,S,T,V,W,Y); 32 (K,A,D,G,I,M,S,Y); 33 (N,A,D,E,H,K,Q,S,T,V,W); 34 (V,A,D,F,G,H,L,Q,R,S,T,W,Y); 37 (T,A,C,D,E,G,K,Q,S,V); 38 (A,C,D,E,G,H,M,N,R,S,T,W); 49 (S,D,E,G,L,M,N,T,V,Y); 50 (S,D,E,G,H,K,M,N,Q,V); 51 (A,E,G,K,L,Q,R,S,V,W); 60 (T,A,E,G,H,N,V); 67 (N,A,D,E,G,K,L,M,Q,S,V,W,Y); 70 (G,D,E,F,H,K,W,Y); 73 (R,A,E,G,H,M,P,S,W); 78 (T,A,C,D,E,F,G,H,I,K,L,M,N,Q,R,S,V); 80 (S,A,C,D,E,F,G,K,M,P,R,T,V,W); 81 (E,A,C,D,F,G,H,M,N,Q,R,S,T,V,Y); 83 (I,A,D,E,G,H,M,Q,S,T,V,W,Y); 84 (S,A,E,F,G,I,K,M,P,V,W,Y); 86 (V,A,E,I,K,L,M,Q,R,S,T); 87 (N,A,D,F,G,L,Q,R,S,T,V,Y); 88 (N,A,C,E,I,M,R,S,T,V,Y); 91 (A,C,E,F,H,I,K,L,M,S,T,V,W,Y); 92 (K,A,D,E,G,I,M,N,Q,R,S,T,Y); 95 (A,D,F,G,L,M,N,Q,S,T,W,Y); 97 (Y,A,F,H,I,L,M,N,V); 105 (R,A,G,K,L,M,S,T); 107 (N,A,E,G,I,K,M,P,S,W); 110 (A,C,D,E,G,H,K,L,N,Q,R,S,T,V,W,Y); 111 (T,A,C,D,E,F,G,I,K,L,M,N,S,V); 113 (L,A,C,D,E,F,G,I,N,P,Q,S,V,W,Y); 115 (D,A,E,F,G,H,I,K,M,N,R,S,T,W); 121 (P,A,E,F,Q,R,S,T); 122 (N,A,D,E,F,G,H,Q,R,S,V,W); 126 (V,A,C,E,L,N,Q,R,S,T,W); 127 (E,A,F,H,K,N,P,Q,R,S,T,V); 128 (T,A,C,F,I,M,R,S,V,W); 129 (T,A,C,E,G,K,Q,R,V); 130 (S,A,D,F,G,H,I,K,L,N,P,Q,R,T,V,W); 131 (T,A,C,D,E,F,G,H,I,M,N,P,Q,R,V,W,Y); 132 (Y,D,E,G,H,Q,S,T,V); 133 (Q,D,F,G,H,I,M,N,P,R,S,V,W,Y); 135 (Q,A,D,E,G,H,I,L,M,N,R,T,V,W,Y); 139 (Q,A,D,F,G,H,K,L,M,N,R,V,W,Y); 141 (D,A,C,E,F,G,I,K,M,Q,R,S,V,W,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 147 (N,A,D,F,G,H,K,P,Q,R,S,T); 148 (T,A,D,E,H,K,M,N,Q,R,V,W,Y); 149 (Y,E,F,G,I,L,M,V); 151 (S,A,D,E,F,G,H,K,N,P,Q,R,T,V,Y); 155 (R,A,C,E,F,G,K,N,T,V,Y); 157 (Y,D,F,G,I,M,P,Q,R,S,V,W); 162 (V,A,C,D,E,G,H,L,Q); 164 (W,A,C,I,L,M,N,Q,T); 166 (Q,A,C,D,E,F,G,H,K,N,P,R,S,T); 167 (S,A,C,D,E,F,G,H,I,L,Q,R,T,V,W,Y); 169 (G,A,D,E,F,H,K,Q,R,S,T,W); 170 (L,A,D,E,G,H,K,Q,R,S,V,W,Y); 174 (Y,C,E,F,H,M,W); 179 (D,A,C,E,F,H,K,L,M,N,R,S,T,V,W,Y); 180 (D,A,E,M,N,Q,R,T,Y); 182 (D,A,E,F,H,I,K,M,N,R,S,T,V,W,Y); 189 (S,A,D,E,G,N); 192 (G,A,E,H,M,Q,S,V,Y); 205 (N,A,C,D,E,F,G,I,M,S,V,W,Y); 206 (H,A,C,D,E,L,M,Q,W,Y); 207 (P,A,D,E,G,H,L,N,R,S,T); 208 (D,E,F,G,I,L,N,Q,R,S,V,Y); 211 (N,A,F,H,I,K,L,M,Q,R,S,T,V,W,Y); 214 (K,A,D,E,F,G,I,L,M,N,Q,S,Y); 215 (T,A,D,E,F,G,I,L,M,N,Q,S,V,Y); 225 (N,A,C,D,E,F,G,I,M,P,S,V,W,Y); 234 (V,A,E,G,I,L,N,S,T); 241 (F,C,D,E,G,H,I,K,M,N,R,S,V,Y); 242 (M,A,C,E,L,Q,Y); 243 (R,A,C,D,E,F,G,H,L,Q,S,V,W,Y); 247 (N,A,D,E,F,G,M,Q,R,S,V,W); 248 (N,A,C,D,E,F,G,H,I,K,M,Q,R,S,V,W); 250 (R,A,D,E,F,G,H,I,M,N,T,W); 251 (S,A,D,E,F,G,K,L,M,N,Q,T,V,W); 252 (T,A,E,F,G,H,K,L,M,N,Q,V); 253 (T,E,G,I,K,L,M,N,P,R,S,V,Y); 254 (G,A,E,H,I,K,M,P,Q,R,V,Y); 255 (K,A,D,G,I,M,N,R,S,T,V,W,Y); 256 (N,A,D,G,L,M,S,Y); 258 (F,A,G,H,K,M,P,S,T,V,W,Y); 265 (H,A,D,E,G,I,K,M,R,V,W); 266 (Y,D,E,G,K,L,M,N,P,Q,R,S,T,V,W); 268 (V,A,D,E,F,G,I,K,L,M,N,P,Q,R,W,Y); 269 (N,A,D,E,G,H,I,K,Q,S,T,Y); 272 (N,D,E,H,I,K,Q,V); 276 (T,A,C,D,E,G,I,K,L,Q,W,Y); 279 (N,A,D,E,G,H,Q,S,T,V,W); 280 (G,A,D,E,K,M,N,Q,R,S,Y); 281 (T,A,D,E,H,K,N,Q,R,S); 282 (M,E,H,I,L,N,Q,S,Y); 283 (S,A,C,D,E,G,H,K,M,R,T,W,Y); 291 (F,D,G,I,K,M,N,Q,V,Y); 295 (D,A,E,H,K,N,Q,W,Y); 300 (G,A,E,F,H,K,N,Q,S,V,W,Y); 302 (G,A,D,E,H,K,M,N,R,S,V,W,Y); 307 (N,A,D,E,F,G,H,K,L,M,Q,R,S,T,V,W); 310 (N,D,E,G,H,K,M,R,V); 311 (N,A,D,E,G,H,Q,R,Y); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 319 (M,A,D,E,F,G,I,K,Q,R,S,T,V,W,Y); 333 (T,A,D,E,F,G,N,Q,S,Y); 359 (Y,A,D,F,H,L,M,N,R,S,T,V,W); 361 (C,A,E,I,K,Q,T,V); 368 (Y,A,D,E,F,I,K,L,N,Q,R,S,T,V,W); 371 (S,A,D,E,G,T,Y); 374 (K,A,D,E,G,M,N); 376 (S,A,E,G,H,I,N,Q,R,T,V); 377 (S,A,D,E,F,G,H,L,N,P,Q,R,T,V,Y); 378 (Y,A,D,E,G,N,S,T,V); 380 (P,A,D,E,G,H,K,M,N,R,S,T,W,Y); 381 (I,A,E,F,L,Q,T); 384 (K,A,C,D,F,G,I,M,N,P,S,T,V,Y); 387 (N,A,G,H,I,L,Q,S,T,Y); 391 (V,A,D,F,H,K,L,M,N); 396 (T,D,E,F,H,I,L,M,N,P,Q,R,S,V,Y); 404 (P,A,C,E,G,L,M,N,Q,S,V,W); 412 (E,A,D,G,L,M,Q,T); 415 (A,D,E,G,K,M,Q,R); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 430 (P,A,C,D,E,F,G,H,I,K,M,S,V,Y); 431 (G,A,D,E,K,N,P,Q,R,S,T,V); 435 (W,A,D,E,F,H,K,L,M,N,Q,R,S,Y); 440 (T,A,D,E,H,I,L,M,N,S,V); 446 (V,A,D,E,H,I,L,M,N,R,S,T); 454 (R,A,C,E,F,K,L,N,Q,S,V,W,Y); 455 (S,A,D,E,H,P,Q,R,T); 462 (A,C,D,E,G,K,M,P,R,T,V); 463 (N,A,E,G,H,Q,R,S,T,Y); 467 (N,E,H,K,L,M,Q,R,V); 469 (W,A,D,E,G,H,K,N,P,S,V,Y); 471 (N,A,D,E,G,K,Q,R); 472 (G,A,D,E,F,H,K,N,T); 473 (G,A,D,E,F,K,Q,R,S,V,Y); and 480 (K,E,G,I,M,P,S,V,W,Y).

The productive positions in PcuAmyl-vl that fall within the previously described Productivity Scores of "4," and the substitutions within those positions that are combinable, are listed below. The position numbering is based on the mature PcuAmyl polypeptide (SEQ ID NO: 3):
LIST D:
1 (G,D,E,F,H,I,K,M,N,R,S,V,W); 4 (G,D,E,F,H,I,L,M,N,P,Q,S,V,W,Y); 9 (Y,A,D,F,G,H,M,N,Q,S,T,V,W); 22 (N,A,C,D,E,F,G,H,I,K,M,Q,R,S,T,W); 27 (D,C,E,F,G,I,K,M,N,Q,S,T,V,W); 29 (Q,A,D,E,F,G,I,M,N,P,R,V,W,Y); 30 (N,A,D,F,G,H,L,M,P,Q,R,S,T,V,W,Y); 34 (V,A,D,F,G,H,L,Q,R,S,T,W,Y); 67 (N,A,D,E,G,K,L,M,Q,S,V,W,Y); 78 (T,A,C,D,E,F,G,H,I,K,L,M,N,Q,R,S,V); 80 (S,A,C,D,E,F,G,K,M,P,R,T,V,W); 81 (E,A,C,D,F,G,H,M,N,Q,R,S,T,V,Y); 83 (I,A,D,E,G,H,M,Q,S,T,V,W,Y); 84 (S,A,E,F,G,I,K,M,P,V,W,Y); 91 (A,C,E,F,H,I,K,L,M,S,T,V,W,Y); 110 (A,C,D,E,G,H,K,L,N,Q,R,S,T,V,W,Y); 111 (T,A,C,D,E,F,G,I,K,L,M,N,S,V); 113 (L,A,C,D,E,F,G,I,N,P,Q,S,V,W,Y); 115 (D,A,E,F,G,H,I,K,M,N,R,S,T,W); 122 (N,A,D,E,F,G,H,Q,R,S,V,W); 126 (V,A,C,E,L,N,Q,R,S,T,W); 130 (S,A,D,F,G,H,I,K,L,N,P,Q,R,T,V,W); 131 (T,A,C,D,E,F,G,H,I,M,N,P,Q,R,V,W,Y); 133 (Q,D,F,G,H,I,M,N,P,R,S,V,W,Y); 135 (Q,A,D,E,G,H,I,L,M,N,R,T,V,W,Y); 139 (Q,A,D,F,G,H,K,L,M,N,R,V,W,Y); 141 (D,A,C,E,F,G,I,K,M,Q,R,S,V,W,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 148 (T,A,D,E,H,K,M,N,Q,R,V,W,Y); 151 (S,A,D,E,F,G,H,K,N,P,Q,R,T,V,Y); 157 (Y,D,F,G,I,M,P,Q,R,S,V,W); 166 (Q,A,C,D,E,F,G,H,K,N,P,R,S,T); 167 (S,A,C,D,E,F,G,H,I,L,Q,R,T,V,W,Y); 170 (L,A,D,E,G,H,K,Q,R,S,V,W,Y); 179 (D,A,C,E,F,H,K,L,M,N,R,S,T,V,W,Y); 182 (D,A,E,F,H,I,K,M,N,R,S,T,V,W,Y); 205 (N,A,C,D,E,F,G,I,M,S,V,W,Y); 208 (D,E,F,G,I,L,N,Q,R,S,V,Y); 211 (N,A,F,H,I,K,L,M,Q,R,S,T,V,W,Y); 214 (K,A,D,E,F,G,I,L,M,N,Q,S,Y); 215 (T,A,D,E,F,G,I,L,M,N,Q,S,V,Y); 225 (N,A,C,D,E,F,G,I,M,P,S,V,W,Y); 241 (F,C,D,E,G,H,I,K,M,N,R,S,V,Y); 243 (R,A,C,D,E,F,G,H,L,Q,S,V,W,Y); 247 (N,A,D,E,F,G,M,Q,R,S,V,W); 248 (N,A,C,D,E,F,G,H,I,K,M,Q,R,S,V,W); 250 (R,A,D,E,F,G,H,I,M,N,T,W); 253 (T,E,G,I,K,L,M,N,P,R,S,V,Y); 255 (K,A,D,G,I,M,N,R,S,T,V,W,Y); 265 (H,A,D,E,G,I,K,M,R,V,W); 266 (Y,D,E,G,K,L,M,N,P,Q,R,S,T,V,W); 268 (V,A,D,E,F,G,I,K,L,M,N,P,Q,R,W,Y); 269 (N,A,D,E,G,H,I,K,Q,S,T,Y); 276 (T,A,C,D,E,G,I,K,L,Q,W,Y); 283 (S,A,C,D,E,G,H,K,M,R,T,W,Y); 302 (G,A,D,E,H,K,M,N,R,S,V,W,Y); 307 (N,A,D,E,F,G,H,K,L,M,Q,R,S,T,V,W); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 319 (M,A,D,E,F,G,I,K,Q,R,S,T,V,W,Y); 359 (Y,A,D,F,H,L,M,N,R,S,T,V,W); 368 (Y,A,D,E,F,I,K,L,N,Q,R,S,T,V,W); 377 (S,A,D,E,F,G,H,L,N,P,Q,R,T,V,Y); 380 (P,A,D,E,G,H,K,M,N,R,S,T,W,Y); 384 (K,A,C,D,F,G,I,M,N,P,S,T,V,Y); 396 (T,D,E,F,H,I,L,M,N,P,Q,R,S,V,Y); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 430 (P,A,C,D,E,F,G,H,I,K,M,S,V,Y); 435 (W,A,D,E,F,H,K,L,M,N,Q,R,S,Y); and 454 (R,A,C,E,F,K,L,N,Q,S,V,W,Y).

In some embodiments, the combinable positions and substitutions demonstrate favorable surface modifications, particularly charge surface modifications, as described in detail, herein.

Although the foregoing mutations were identified using PcuAmyl-vl (SEQ ID NO: 4), they are expected to produce similar effects in PcuAmyl (SEQ ID NO: 3).

In addition to the mutation at position T333, the present α-amylase variants may have at least one further combinable mutation at a productive position corresponding to the combinable mutations at productive positions described, above, in Lists A, B, C, and/or D, and/or a combinable mutation as described in Table 3 or 4 (which use SEQ ID NO: 3 for numbering). In some embodiments, the suitability score of the at least one further mutation is +++, ++++, or +++++. In some embodiments, the suitability score of the at least one further mutation is ++++, or +++++. In some embodiments, the suitability score of the at least one further mutation is +++++. In some embodiments, the variants have a plurality (*e.g.,* 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more) combinable mutations.

### 2.2 Additional mutations

In some embodiments, in addition to one or more of the mutations described above (*e.g.,* in Section 2.1), the present amylases further include one or more mutations that provide a further performance or stability benefit. Exemplary performance benfits include but are not limited to increased hydrolysis of a starch substrate, increased grain, cereal or other starch substrate liquifaction and/or saccharification performance, increased cleaning performance, increased thermal stability, increased storage stability, increased solubility, an altered pH profile, decreased calcium dependence, increased specific activity, modified substrate specificity, modified substrate binding, modified pH-dependent activity, modified pH-dependent stability, increased oxidative stability, and increased expression. In some cases, the performance benefit is realized at a relatively low temperature. In some cases, the performance benefit is realized at relatively high temperature.

In some embodiments, the present PcuAmyl α-amylase variants additionally have at least one mutation in the calcium binding loop based on the work of Suzuki et al. (1989) J. Biol. Chem. 264:18933-938. Exemplary mutations include a deletion or substitution at one or more residues corresponding to Arg-177, Gly-178, Asp-179, or Gly-180 in SEQ ID NO: 3. In particular embodiments, the mutation corresponds to the deletion of Arg-177 and Gly-178 or Asp-179 and Gly-180 (using SEQ ID NO: 3 numbering). Homologous residues in other amylases can be determined by structural alignment, or by primary structure alignment.

In some embodiments, the present α-amylase variants additionally have at least one mutation known to produce a performance, stability, or solubility benefit in other microbial α-amylases, including but not limited to those having a similar fold and/or having 60% or greater amino acid sequence identity to PcuAmyl (SEQ ID NO: 3) or any of the well-known *Bacillus* amylases (*e.g.,* from *B. lichenifomis, B. stearothermophilus, and B. amyloliquifaciens*)*,* Carbohydrate-Active Enzymes database (CAZy) Family 13 amylases, or any amylase that has heretofore been referred to by the descriptive term, "Termamyl-like." Amino acid sequence identity can be determined using Clustal W with default parameters.

Furthermore, the present amylases may include any number of conservative or non-natural or chemically modified amino acid substitutions. The reader will appreciate that some of the above mentioned conservative mutations can be produced by genetic manpulation, while others are produced by introducing synthetic amino acids into a polypeptide by genetic or other means.

The present amylase may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective amylase polypeptides. The present amylase polypeptides may also be truncated to remove the N or C-termini, so long as the resulting polypeptides retain amylase activity.

The present amylase may be a "chimeric" or "hybrid" polypeptide, in that it includes at least a portion of a first amylase polypeptide, and at least a portion of a second amylase polypeptide (such chimeric amylases have recently been "rediscovered" as domain-swap amylases). The present amylases may further include heterologous signal sequence, an epitope to allow tracking or purification, or the like. Exemplary heterologous signal sequences are from *B. licheniformis* amylase (LAT), *B. subtilis* (AmyE or AprE), and *Streptomyces* CelA.

### 2.3. Nucleic acids encoding variant α-amylase polypeptides

Nucleic acids encoding PcuAmyl α-amylase, or a variant may find use in the methods of the invention.

Nucleic acids may encode a "full-length" ("fl" or "FL") amylase, which includes a signal sequence, only the mature form of an amylase, which lacks the signal sequence, or a truncated form of an amylase, which lacks the N or C-terminus of the mature form.

A nucleic acid that encodes an α-amylase can be operably linked to various promoters and regulators in a vector suitable for expressing the α-amylase in host cells. Exemplary promoters are from *B. licheniformis* amylase (LAT), *B. subtilis* (AmyE or AprE), and *Streptomyces* CelA. Such a nucleic acid can also be linked to other coding sequences, *e.g.,* to encode a chimeric polypeptide.

### 3. Production of Amylases and Variants Thereof

The present amylases and variants thereof (referred to below as "amylases") can be produced in host cells, for example, by secretion or intracellular expression. A cultured cell material (e.g., a whole-cell broth) comprising a amylase can be obtained following secretion of the amylase into the cell medium. Optionally, the amylase can be isolated from the host cells, or even isolated from the cell broth, depending on the desired purity of the final amylase. A gene encoding an amylase can be cloned and expressed according to methods well known in the art. Suitable host cells include bacterial, fungal (including yeast and filamentous fungi), and plant cells (including algae). Particularly useful host cells include *Aspergillus niger, Aspergillus oryzae* or *Trichoderma reesei.* Other host cells include bacterial cells, *e.g., Bacillus subtilis* or *B. licheniformis,* as well as *Streptomyces.*

The host cell further may express a nucleic acid encoding a homologous or heterologous glucoamylase, *i.e.,* a glucoamylase that is not the same species as the host cell, or one or more other enzymes. The glucoamylase may be a variant glucoamylase, such as one of the glucoamylase variants disclosed in U.S. Patent No. 8,058,033 (Danisco US Inc.), for example. Additionally, the host may express one or more accessory enzymes, proteins, peptides. These may benefit liquefaction, saccharification, fermentation, SSF, etc., processes. Furthermore, the host cell may produce biochemicals in addition to enzymes used to digest the various feedstock(s). Such host cells may be useful for fermentation or simultaneous saccharification and fermentation processes to reduce or eliminate the need to add enzymes.

### 3.1. Vectors

A DNA construct comprising a nucleic acid encoding an amylase or variant thereof as described herein can be constructed such that the amylase (or variant) is expressed in a host cell. One representative nucleic acid that encodes an amylase is SEQ ID NO: 1. Because of the well-known degeneracy in the genetic code, variant polynucleotides that encode an identical amino acid sequence can be designed and made with routine skill. It is also well-known in the art to optimize codon use for a particular host cell. Nucleic acids encoding variant amylases can be incorporated into a vector. Vectors can be transferred to a host cell using well-known transformation techniques, such as those disclosed below.

The vector may be any vector that can be transformed into and replicated within a host cell. For example, a vector comprising a nucleic acid encoding a variant amylase can be transformed and replicated in a bacterial host cell as a means of propagating and amplifying the vector. The vector also may be transformed into an expression host, so that the encoding nucleic acids can be expressed as a functional amylase. Host cells that serve as expression hosts can include filamentous fungi, for example. The Fungal Genetics Stock Center (FGSC) Catalogue of Strains lists suitable vectors for expression in fungal host cells. *See* FGSC, Catalogue of Strains, University of Missouri, *at* www.fgsc.net (last modified January 17, 2007). A representative vector is pJG153, a promoterless Cre expression vector that can be replicated in a bacterial host. *See* Harrison et al. (June 2011) Applied Environ. Microbiol. 77: 3916-22. pJG153can be modified with routine skill to comprise and express a nucleic acid encoding an amylase variant.

A nucleic acid encoding a variant amylase can be operably linked to a suitable promoter, which allows transcription in the host cell. The promoter may be any DNA sequence that shows transcriptional activity in the host cell of choice and may be derived from genes encoding proteins either homologous or heterologous to the host cell. Exemplary promoters for directing the transcription of the DNA sequence encoding a variant amylase, especially in a bacterial host, are the promoter of the lac operon of *E. coli,* the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus niger* neutral α-amylase, A. *niger* acid stable α-amylase, A. *niger* glucoamylase, *Rhizomucor miehei* lipase, A. *oryzae* alkaline protease, A. *oryzae* triose phosphate isomerase, or A. *nidulans* acetamidase. When a gene encoding an amylase is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. *cbh1* is an endogenous, inducible promoter from *T. reesei. See* Liu et al. (2008) "Improved heterologous gene expression in Trichoderma reesei by cellobiohydrolase I gene (cbhl) promoter optimization," Acta Biochim. Biophys. Sin (Shanghai) 40(2): 158-65.

The coding sequence can be operably linked to a signal sequence. The DNA encoding the signal sequence may be the DNA sequence naturally associated with the amylase gene to be expressed or from a different Genus or species. A signal sequence and a promoter sequence comprising a DNA construct or vector can be introduced into a fungal host cell and can be derived from the same source. For example, the signal sequence is the *cbh1* signal sequence that is operably linked to a *cbh1* promoter.

An expression vector may also comprise a suitable transcription terminator and, in eukaryotes, polyadenylation sequences operably linked to the DNA sequence encoding a variant amylase. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUB110, pE194, pAMBl, and pIJ702.

The vector may also comprise a selectable marker, *e.g.,* a gene the product of which complements a defect in the isolated host cell, such as the *dal* genes from *B. subtilis* or *B. licheniformis,* or a gene that confers antibiotic resistance such as, *e.g.,* ampicillin, kanamycin, chloramphenicol or tetracycline resistance. Furthermore, the vector may comprise *Aspergillus* selection markers such as *amdS, argB, niaD* and *xxsC*, a marker giving rise to hygromycin resistance, or the selection may be accomplished by co-transformation, such as known in the art. *See e.g.,* International PCT Application WO 91/17243.

Intracellular expression may be advantageous in some respects, *e.g*., when using certain bacteria or fungi as host cells to produce large amounts of amylase for subsequent enrichment or purification. Extracellular secretion of amylase into the culture medium can also be used to make a cultured cell material comprising the isolated amylase.

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the amylase to a host cell organelle such as a peroxisome, or to a particular host cell compartment. Such a targeting sequence includes but is not limited to the sequence, SKL. For expression under the direction of control sequences, the nucleic acid sequence of the amylase is operably linked to the control sequences in proper manner with respect to expression.

The procedures used to ligate the DNA construct encoding an amylase, the promoter, terminator and other elements, respectively, and to insert them into suitable vectors containing the information necessary for replication, are well known to persons skilled in the art (*see, e.g.,* Sambrook et al., MOLECULAR CLONING: A LABORATORY MANUAL, 2nd ed., Cold Spring Harbor, 1989; 3rd ed., 2001; and 4th ed., 2012.

### 3.2. Transformation and Culture of Host Cells

An isolated cell, either comprising a DNA construct or an expression vector, is advantageously used as a host cell in the recombinant production of an amylase. The cell may be transformed with the DNA construct encoding the enzyme, conveniently by integrating the DNA construct (in one or more copies) in the host chromosome. This integration is generally considered to be an advantage, as the DNA sequence is more likely to be stably maintained in the cell. Integration of the DNA constructs into the host chromosome may be performed according to conventional methods, *e.g*., by homologous or heterologous recombination. Alternatively, the cell may be transformed with an expression vector as described above in connection with the different types of host cells.

The present invention also relates to a recombinant host cell comprising the described nucleic acid. The recombinant host cell can be advantageously used in the recombinant production of the disclosed polypeptides. The host cell can have a vector comprising the polynucleotide sequence which is introduced into the host cell. The induced vector can be maintained in the host cell as a chromosomal integrant or as a self-replicating extra-chromosomal vector.

The host cell can be any convenient cell, including a fungal cell (*e.g.,* a filamentous fungal cell), a bacterial cell, a yeast cell, a plant cell, a seaweed or an algal cell, etc.

Examples of suitable bacterial host cells (or organisms) include Gram positive bacterial species such as *Bacillaceae* including *Bacillus subtilis, Bacillus licheniformis, Bacillus lentus, Bacillus brevis, Geobacillus* (formerly *Bacillus*) *stearothermophilus, Bacillus alkalophilus, Bacillus amyloliquefaciens, Bacillus coagulans, Bacillus lautus, Bacillus megaterium,* and *Bacillus thuringiensis*; *Streptomyces* species such as *Streptomyces murinus;* lactic acid bacterial species including *Lactococcus* sp. such as *Lactococcus lactis*; *Lactobacillus* sp. including *Lactobacillus reuteri*; *Leuconostoc* sp.; *Pediococcus* sp.; and *Streptococcus* sp. Alternatively, strains of a Gram negative bacterial species belonging to *Enterobacteriaceae* including *E. coli, Pseudomonas sp.* or to *Pseudomonadaceae* can be selected as the host organism.

The host cell can be a eukaryote, such as a mammalian, insect, plant, or fungal cell. In certain embodiments, the host cell is a fungal cell. The phyla *Ascomycota, Basidiomycota*, *Chytridiomycota*, *Zygomycota* and *Oomycota* and all mitosporic fungi are included herein when referring to "fungi". In another embodiment, the fungal host cell is a yeast cell. As used herein examples of "yeast" include ascosporogenous yeast (*Endomycetales*), basidiosporogenous yeast, and yeast belonging to the Fungi Imperfecti (*Blastomycetes*)*.* (See "Biology and Activities of Yeast", Skinner, F. A., Passmore, S. M., and Davenport, R. R., eds, Soc. App. Bacteriol. Symposium Series No. 9, 1980A; and Ainsworth and Bisby's Dictionary of The Fungi, 8th edition, 1995, CAB International, University Press, Cambridge, UK for a description of yeast and fungi, respectively).

A suitable yeast host cell can be selected from the biotechnologically relevant yeasts species such as but not limited to yeast species such as *Pichia* sp., *Hansenula* sp., *Candida sp.,* or *Kluyveromyces sp., Yarrowinia sp., Schizosaccharomyces* species or a species of *Saccharomyces,* including *Saccharomyces cerevisiae* or a species belonging to *Schizosaccharomyces* such as, for example, *S. pombe* species. In certaion embodiments, the yeast host cell is a *Saccharomyces carlsbergensis, Saccharomyces cerevisiae, Saccharomyces diastaticus, Saccharomyces douglasii*, *Saccharomyces kluyveri, Saccharomyces norbensis, Saccharomyces oviformis, Kluyveromyces lactis,* or a *Yarrowia lipolytica* cell. A strain of the methylotrophic yeast species, *Pichia pastoris*, can also be used as the host organism.

In certain embodiments, the host cell is a filamentous fungal host cell is, *e.g.,* a cell of a species of, but not limited to, *Acremonium, Aspergillus, Fusarium, Humicola, Mucor, Myceliophthora, Neurospora, Penicillium, Thielavia, Tolypocladium,* or *Trichoderma.* As such, suitable host organisms among filamentous fungi include species of *Aspergillus, e.g., Aspergillus niger, Aspergillus oryzae, Aspergillus tubigensis, Aspergillus awamori,* or *Aspergillus nidulans.* Alternatively, strains of a *Fusarium* species, *e.g., Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell, or of a *Rhizomucor* species such as *Rhizomucor miehei* can be used as the host organism. Other suitable strains include *Thermomyces* and *Mucor* species. The filamentous fungal host cell can be a *Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* or *Trichoderma viride* cell.

A suitable procedure for transformation of *Aspergillus* host cells includes, for example, that described in EP 238023. An amylase expressed by a fungal host cell can be glycosylated, *i.e.,* will comprise a glycosyl moiety. The glycosylation pattern can be the same or different as present in the wild-type amylase. The type and/or degree of glycosylation may impart changes in enzymatic and/or biochemical properties.

It can be advantageous to delete genes from expression hosts, where the gene deficiency can be cured by the transformed expression vector. Known methods can be used to obtain a fungal host cell having one or more inactivated genes. Gene inactivation can be accomplished by complete or partial deletion, by insertional inactivation or by any other means that renders a gene nonfunctional for its intended purpose, such that the gene is prevented from expression of a functional protein. For example, genes from a *Trichoderma* sp. or other filamentous fungal host that have been cloned can be deleted, for example, *cbh1, cbh2, egl1,* and *egl2* genes. Gene deletion can be accomplished by inserting a form of the desired gene to be inactivated into a plasmid by methods known in the art.

Introduction of a DNA construct or vector into a host cell includes techniques such as transformation; electroporation; nuclear microinjection; transduction; transfection, *e.g*., lipofection mediated and DEAE-Dextrin mediated transfection; incubation with calcium phosphate DNA precipitate; high velocity bombardment with DNA-coated microprojectiles; and protoplast fusion. General transformation techniques are known in the art. *See, e.g.,* Sambrook *et al.* (2001), *supra.* The expression of heterologous protein in *Trichoderma* is described, for example, in U.S. Patent No. 6,022,725. Reference is also made to Cao et al. (2000) Science 9:991-1001 for transformation of *Aspergillus* strains. Genetically stable transformants can be constructed with vector systems whereby the nucleic acid encoding an amylase is stably integrated into a host cell chromosome. Transformants are then selected and purified by known techniques.

The preparation of *Trichoderma* sp. for transformation, for example, may involve the preparation of protoplasts from fungal mycelia. *See* Campbell et al. (1989) Curr. Genet. 16: 53-56. The mycelia can be obtained from germinated vegetative spores. The mycelia are treated with an enzyme that digests the cell wall, resulting in protoplasts. The protoplasts are protected by the presence of an osmotic stabilizer in the suspending medium. These stabilizers include sorbitol, mannitol, potassium chloride, magnesium sulfate, and the like. Usually the concentration of these stabilizers varies between 0.8 M and 1.2 M, *e.g.,* a 1.2 M solution of sorbitol can be used in the suspension medium.

Uptake of DNA into a host *Trichoderma* sp. strain depends upon the calcium ion concentration. Generally, between about 10-50 mM CaCl₂ is used in an uptake solution. Additional suitable compounds include a buffering system, such as TE buffer (10 mM Tris, pH 7.4; 1 mM EDTA) or 10 mM MOPS, pH 6.0 and polyethylene glycol. The polyethylene glycol is believed to fuse the cell membranes, thus permitting the contents of the medium to be delivered into the cytoplasm of the *Trichoderma* sp. strain. This fusion frequently leaves multiple copies of the plasmid DNA integrated into the host chromosome.

Transformation of *Trichoderma* sp. can use protoplasts or cells that have been subjected to a permeability treatment, typically at a density of 10⁵ to 10⁷/mL, particularly 2x10⁶/mL. A volume of 100 µL of these protoplasts or cells in an appropriate solution (e.g., 1.2 M sorbitol and 50 mM CaCl₂) may be mixed with the desired DNA. Generally, a high concentration of PEG is added to the uptake solution. From 0.1 to 1 volume of 25% PEG 4000 can be added to the protoplast suspension; however, it is useful to add about 0.25 volumes to the protoplast suspension. Additives, such as dimethyl sulfoxide, heparin, spermidine, potassium chloride and the like, may also be added to the uptake solution to facilitate transformation. Similar procedures are available for other fungal host cells. *See, e.g.,* U.S. Patent No. 6,022,725.

In another embodiment, the fungal host cell is a filamentous fungal cell. "Filamentous fungi" include all filamentous forms of the subdivision Eumycota and Oomycota (as defined by Hawksworth *et al.,* 1995, supra). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as Saccharomyces cerevisiae is by budding of a unicellular thallus and carbon catabolism may be fermentative.

### 3.3. Expression

A method of producing an amylase may comprise cultivating a host cell as described above under conditions conducive to the production of the enzyme and recovering the enzyme from the cells and/or culture medium.

The medium used to cultivate the cells may be any conventional medium suitable for growing the host cell in question and obtaining expression of an amylase. Suitable media and media components are available from commercial suppliers or may be prepared according to published recipes (*e.g.,* as described in catalogues of the American Type Culture Collection).

An enzyme secreted from the host cells can be used in a whole broth preparation. In the present methods, the preparation of a spent whole fermentation broth of a recombinant microorganism can be achieved using any cultivation method known in the art resulting in the expression of an α-amylase. Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the amylase to be expressed or isolated. The term "spent whole fermentation broth" is defined herein as unfractionated contents of fermentation material that includes culture medium, extracellular proteins (*e.g.,* enzymes), and cellular biomass. It is understood that the term "spent whole fermentation broth" also encompasses cellular biomass that has been lysed or permeabilized using methods well known in the art.

An enzyme secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

The polynucleotide encoding an amylase in a vector can be operably linked to a control sequence that is capable of providing for the expression of the coding sequence by the host cell, *i.e.* the vector is an expression vector. The control sequences may be modified, for example by the addition of further transcriptional regulatory elements to make the level of transcription directed by the control sequences more responsive to transcriptional modulators. The control sequences may in particular comprise promoters.

Host cells may be cultured under suitable conditions that allow expression of an amylase. Expression of the enzymes may be constitutive such that they are continually produced, or inducible, requiring a stimulus to initiate expression. In the case of inducible expression, enzyme production can be initiated when required by, for example, addition of an inducer substance to the culture medium, for example dexamethasone or IPTG or Sophorose. Polypeptides can also be produced recombinantly in an *in vitro* cell-free system, such as the TNT™ (Promega) rabbit reticulocyte system.

An expression host also can be cultured in the appropriate medium for the host, under aerobic conditions. Shaking or a combination of agitation and aeration can be provided, with production occurring at the appropriate temperature for that host, *e.g.,* from about 25°C to about 75°C (*e.g.,* 30°C to 45°C), depending on the needs of the host and production of the desired variant amylase. Culturing can occur from about 12 to about 100 hours or greater (and any hour value there between, *e.g*., from 24 to 72 hours). Typically, the culture broth is at a pH of about 2.0 to about 8.0, again depending on the culture conditions needed for the host relative to production of an amylase.

### 3.4. Identification of Amylase Activity

To evaluate the expression of an amylase in a host cell, assays can measure the expressed enzyme, corresponding mRNA, or α-amylase activity. For example, suitable assays include Northern blotting, reverse transcriptase polymerase chain reaction, and *in situ* hybridization, using an appropriately labeled hybridizing probe. Suitable assays also include measuring amylase activity in a sample, for example, by assays directly measuring reducing sugars such as glucose in the culture media. For example, glucose concentration may be determined using glucose reagent kit No. 15-UV (Sigma Chemical Co.) or an instrument, such as Technicon Autoanalyzer. α-Amylase activity also may be measured by any known method, such as the PAHBAH or ABTS assays, described below. Amylase assays are described in detail in the Examples, particularly Example 1.

### 3.5. Methods for Enriching or Purifying Variants Amylases

Fermentation, separation, and concentration techniques are well known in the art and can be used to prepare variant α-amylase polypeptide-containing solutions of desired purity and concentration.

After fermentation, a fermentation broth is obtained. The microbial cells and various suspended solids, including residual raw fermentation materials, can be removed by conventional separation techniques in order to obtain an amylase solution. Filtration, centrifugation, microfiltration, rotary vacuum drum filtration, ultrafiltration, centrifugation followed by ultra-filtration, extraction, or chromatography, or the like, can be used.

It is often desirable to concentrate a variant α-amylase polypeptide-containing solution in order to optimize recovery. Use of unconcentrated solutions can result in increased incubation time in order to collect the enriched or purified enzyme precipitate.

The enzyme containing solution can be concentrated using conventional concentration techniques until the desired enzyme level is obtained. Concentration of the enzyme containing solution may be achieved by any of the techniques discussed herein. Exemplary methods of enrichment and purification include but are not limited to rotary vacuum filtration and/or ultrafiltration.

The enzyme solution can be concentrated into a concentrated enzyme solution until the enzyme activity of the concentrated variant α-amylase polypeptide-containing solution is at a desired level.

Concentration may be performed using, *e.g.,* a precipitation agent, such as a metal halide precipitation agent. Metal halide precipitation agents include but are not limited to alkali metal chlorides, alkali metal bromides and blends of two or more of these metal halides. Exemplary metal halides include sodium chloride, potassium chloride, sodium bromide, potassium bromide and blends of two or more of these metal halides. The metal halide precipitation agent, sodium chloride, can also be used as a preservative.

The metal halide precipitation agent is used in an amount effective to precipitate an amylase. The selection of at least an effective amount and an optimum amount of metal halide effective to cause precipitation of the enzyme, as well as the conditions of the precipitation for maximum recovery including incubation time, pH, temperature and concentration of enzyme, will be readily apparent to one of ordinary skill in the art, after routine testing.

Generally, at least about 5% w/v (weight/volume) to about 25% w/v of metal halide is added to the concentrated enzyme solution, and usually at least 8% w/v. Generally, no more than about 25% w/v of metal halide is added to the concentrated enzyme solution and usually no more than about 20% w/v. The optimal concentration of the metal halide precipitation agent will depend, among others, on the nature of the specific variant α-amylase polypeptide and on its concentration in the concentrated enzyme solution.

Another alternative way to precipitate the enzyme is to use organic compounds. Exemplary organic compound precipitating agents include: 4-hydroxybenzoic acid, alkali metal salts of 4-hydroxybenzoic acid, alkyl esters of 4-hydroxybenzoic acid, and blends of two or more of these organic compounds. The addition of the organic compound precipitation agents can take place prior to, simultaneously with or subsequent to the addition of the metal halide precipitation agent, and the addition of both precipitation agents, organic compound and metal halide, may be carried out sequentially or simultaneously.

Generally, the organic precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitation agents can be, for example, linear or branched alkyl esters of 4-hydroxybenzoic acid, wherein the alkyl group contains from 1 to 10 carbon atoms, and blends of two or more of these organic compounds. Exemplary organic compounds are linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl esters of 4-hydroxybenzoic acid, butyl ester of 4-hydroxybenzoic acid, ethyl ester of 4-hydroxybenzoic acid and blends of two or more of these organic compounds can also be used. Additional organic compounds also include but are not limited to 4-hydroxybenzoic acid methyl ester (named methyl PARABEN), 4-hydroxybenzoic acid propyl ester (named propyl PARABEN), which also are both amylase preservative agents. For further descriptions, *see, e.g.,* U.S. Patent No. 5,281,526.

Addition of the organic compound precipitation agent provides the advantage of high flexibility of the precipitation conditions with respect to pH, temperature, variant amylase concentration, precipitation agent concentration, and time of incubation.

The organic compound precipitation agent is used in an amount effective to improve precipitation of the enzyme by means of the metal halide precipitation agent. The selection of at least an effective amount and an optimum amount of organic compound precipitation agent, as well as the conditions of the precipitation for maximum recovery including incubation time, pH, temperature and concentration of enzyme, will be readily apparent to one of ordinary skill in the art, in light of the present disclosure, after routine testing.

Generally, at least about 0.01% w/v of organic compound precipitation agent is added to the concentrated enzyme solution and usually at least about 0.02% w/v. Generally, no more than about 0.3% w/v of organic compound precipitation agent is added to the concentrated enzyme solution and usually no more than about 0.2% w/v.

The concentrated polypeptide solution, containing the metal halide precipitation agent, and the organic compound precipitation agent, can be adjusted to a pH, which will, of necessity, depend on the enzyme to be enriched or purified. Generally, the pH is adjusted at a level near the isoelectric point of the amylase. The pH can be adjusted at a pH in a range from about 2.5 pH units below the isoelectric point (pI) up to about 2.5 pH units above the isoelectric point.

The incubation time necessary to obtain an enriched or purified enzyme precipitate depends on the nature of the specific enzyme, the concentration of enzyme, and the specific precipitation agent(s) and its (their) concentration. Generally, the time effective to precipitate the enzyme is between about 1 to about 30 hours; usually it does not exceed about 25 hours. In the presence of the organic compound precipitation agent, the time of incubation can still be reduced to less about 10 hours and in most cases even about 6 hours.

Generally, the temperature during incubation is between about 4°C and about 50°C. Usually, the method is carried out at a temperature between about 10°C and about 45°C (*e.g.,* between about 20°C and about 40°C). The optimal temperature for inducing precipitation varies according to the solution conditions and the enzyme or precipitation agent(s) used.

The overall recovery of enriched or purified enzyme precipitate, and the efficiency with which the process is conducted, is improved by agitating the solution comprising the enzyme, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the enriched or purified enzyme is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration, or the like. Further enrichment or purification of the enzyme precipitate can be obtained by washing the precipitate with water. For example, the enriched or purified enzyme precipitate is washed with water containing the metal halide precipitation agent, or with water containing the metal halide and the organic compound precipitation agents.

During fermentation, a variant α-amylase polypeptide accumulates in the culture broth. For the isolation, enrichment, or purification of the desired variant α-amylase, the culture broth is centrifuged or filtered to eliminate cells, and the resulting cell-free liquid is used for enzyme enrichment or purification. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme-active fraction. For further enrichment or purification, a conventional procedure such as ion exchange chromatography may be used.

Enriched or purified enzymes are useful for laundry and cleaning applications. For example, they can be used in laundry detergents and spot removers. They can be made into a final product that is either liquid (solution, slurry) or solid (granular, powder).

A more specific example of enrichment or purification, is described in Sumitani et al. (2000) "New type of starch-binding domain: the direct repeat motif in the C-terminal region of Bacillus sp. 195 α-amylase contributes to starch binding and raw starch degrading," Biochem. J. 350: 477-484, and is briefly summarized here: The enzyme obtained from 4 liters of a *Streptomyces lividans* TK24 culture supernatant was treated with (NH₄)₂SO₄ at 80% saturation. The precipitate was recovered by centrifugation at 10,000 × g (20 min. and 4°C) and re-dissolved in 20 mM Tris/HCl buffer (pH 7.0) containing 5 mM CaCl₂. The solubilized precipitate was then dialyzed against the same buffer. The dialyzed sample was then applied to a Sephacryl S-200 column, which had previously been equilibrated with 20 mM Tris/HCl buffer, (pH 7.0), 5 mM CaCl₂, and eluted at a linear flow rate of 7 mL/hr with the same buffer. Fractions from the column were collected and assessed for activity as judged by enzyme assay and SDS-PAGE. The protein was further purified as follows. A Toyopearl HW55 column (Tosoh Bioscience, Montgomeryville, PA; Cat. No. 19812) was equilibrated with 20 mM Tris/HCl buffer (pH 7.0) containing 5 mM CaCl₂ and 1.5 M (NH₄)₂SO₄. The enzyme was eluted with a linear gradient of 1.5 to 0 M (NH₄)₂SO₄ in 20 mM Tris/HCL buffer, pH 7.0 containing 5 mM CaCl₂. The active fractions were collected, and the enzyme precipitated with (NH₄)₂SO₄ at 80% saturation. The precipitate was recovered, re-dissolved, and dialyzed as described above. The dialyzed sample was then applied to a Mono Q HR5/5 column (Amersham Pharmacia; Cat. No. 17-5167-01) previously equilibrated with 20 mM Tris/HCl buffer (pH 7.0) containing 5 mM CaCl₂, at a flow rate of 60 mL/hour. The active fractions are collected and added to a 1.5 M (NH₄)₂SO₄ solution. The active enzyme fractions were re-chromatographed on a Toyopearl HW55 column, as before, to yield a homogeneous enzyme as determined by SDS-PAGE.

For production scale recovery, variant α-amylase polypeptides can be enriched or partially purified as generally described above by removing cells via flocculation with polymers. Alternatively, the enzyme can be enriched or purified by microfiltration followed by concentration by ultrafiltration using conventional membranes and equipment. However, for some applications, the enzyme does not need to be enriched or purified, and whole broth culture can be lysed and used without further treatment. The enzyme can then be processed, for example, into granules.

### 4. Compositions and Uses of Variant Amylases

Variants amylases are useful for a variety of industrial applications. For example, variant amylases are useful in a starch conversion process, particularly in a saccharification process of a starch that has undergone liquefaction. The desired end-product may be any product that may be produced by the enzymatic conversion of the starch substrate. For example, the desired product may be a syrup rich in glucose and maltose, which can be used in other processes, such as the preparation of HFCS, or which can be converted into a number of other useful products, such as ascorbic acid intermediates (*e.g.,* gluconate; 2-keto-L-gulonic acid; 5-keto-gluconate; and 2,5-diketogluconate); 1,3-propanediol; aromatic amino acids (*e.g.,* tyrosine, phenylalanine and tryptophan); organic acids (*e.g.,* lactate, pyruvate, succinate, isocitrate, and oxaloacetate); amino acids (*e.g.,* serine and glycine); antibiotics; antimicrobials; enzymes; vitamins; and hormones.

The starch conversion process may be a precursor to, or simultaneous with, a fermentation process designed to produce alcohol for fuel or drinking (*i.e.,* potable alcohol). One skilled in the art is aware of various fermentation conditions that may be used in the production of these end-products. Variant amylases are also useful in compositions and methods of food preparation. These various uses of variant amylases are described in more detail below.

### 4.1. Preparation of Starch Substrates

Those of general skill in the art are well aware of available methods that may be used to prepare starch substrates for use in the processes disclosed herein. For example, a useful starch substrate may be obtained from tubers, roots, stems, legumes, cereals or whole grain. More specifically, the granular starch may be obtained from corn, cobs, wheat, barley, rye, triticale, milo, sago, millet, cassava, tapioca, sorghum, rice, peas, bean, banana, or potatoes. Corn contains about 60-68% starch; barley contains about 55-65% starch; millet contains about 75-80% starch; wheat contains about 60-65% starch; and polished rice contains 70-72% starch. Specifically contemplated starch substrates are corn starch and wheat starch. The starch from a grain may be ground or whole and includes corn solids, such as kernels, bran and/or cobs. The starch may also be highly refined raw starch or feedstock from starch refinery processes. Various starches also are commercially available. For example, corn starch is available from Cerestar, Sigma, and Katayama Chemical Industry Co. (Japan); wheat starch is available from Sigma; sweet potato starch is available from Wako Pure Chemical Industry Co. (Japan); and potato starch is available from Nakaari Chemical Pharmaceutical Co. (Japan).

The starch substrate can be a crude starch from milled whole grain, which contains non-starch fractions, *e.g*., germ residues and fibers. Milling may comprise either wet milling or dry milling or grinding. In wet milling, whole grain is soaked in water or dilute acid to separate the grain into its component parts, *e.g*., starch, protein, germ, oil, kernel fibers. Wet milling efficiently separates the germ and meal (*i.e.,* starch granules and protein) and is especially suitable for production of syrups. In dry milling or grinding, whole kernels are ground into a fine powder and often processed without fractionating the grain into its component parts. In some cases, oils from the kernels are recovered. Dry ground grain thus can comprise significant amounts of non-starch carbohydrate compounds, in addition to starch.

### 4.2. Gelatinization and Liquefaction of Starch

As used herein, the term "liquefaction" or "liquefy" means a process by which starch is converted to less viscous and shorter chain dextrins. Generally, this process involves gelatinization of starch simultaneously with or followed by the addition of an α-amylase, although additional liquefaction-inducing enzymes optionally may be added. In some embodiments, the starch substrate prepared as described above is slurried with water. The starch slurry may contain starch as a weight percent of dry solids of about 10-55%, about 20-45%, about 30-45%, about 30-40%, or about 30-35%. α-Amylase (EC 3.2.1.1) may be added to the slurry, with a metering pump, for example. The α-amylase typically used for this application is a thermally stable, bacterial α-amylase, such as a *Geobacillus stearothermophilus* α-amylase. The α-amylase is usually supplied, for example, at about 1500 units per kg dry matter of starch. To optimize α-amylase stability and activity, the pH of the slurry typically is adjusted to about pH 5.5-6.5 and about 1 mM of calcium (about 40 ppm free calcium ions) can also be added. *Geobacillus stearothermophilus* variants or other α-amylases may require different conditions. Bacterial α-amylase remaining in the slurry following liquefaction may be deactivated via a number of methods, including lowering the pH in a subsequent reaction step or by removing calcium from the slurry in cases where the enzyme is dependent upon calcium.

The slurry of starch plus the α-amylase may be pumped continuously through a jet cooker, which is steam heated to 105°C. Gelatinization occurs rapidly under these conditions, and the enzymatic activity, combined with the significant shear forces, begins the hydrolysis of the starch substrate. The residence time in the jet cooker is brief. The partly gelatinized starch may be passed into a series of holding tubes maintained at 105-110°C and held for 5-8 min. to complete the gelatinization process ("primary liquefaction"). Hydrolysis to the required DE is completed in holding tanks at 85-95°C or higher temperatures for about 1 to 2 hours ("secondary liquefaction"). These tanks may contain baffles to discourage back mixing. As used herein, the term "minutes of secondary liquefaction" refers to the time that has elapsed from the start of secondary liquefaction to the time that the Dextrose Equivalent (DE) is measured. The slurry is then allowed to cool to room temperature. This cooling step can be 30 minutes to 180 minutes, *e.g.* 90 minutes to 120 minutes. The liquefied starch typically is in the form of a slurry having a dry solids content (w/w) of about 10-50%; about 10-45%; about 15-40%; about 20-40%; about 25-40%; or about 25-35%.

Liquefaction with variant amylases advantageously can be conducted at low pH, eliminating the requirement to adjust the pH to about pH 5.5-6.5. Variants amylases can be used for liquefaction at a pH range of 2 to 7, *e.g.,* pH 3.0 - 7.5, pH 4.0 - 6.0, or pH 4.5 - 5.8. Variant amylases can maintain liquefying activity at a temperature range of about 85°C - 95°C, *e.g.,* 85°C, 90°C, or 95°C. For example, liquefaction can be conducted with 800 µg an amylase in a solution of 25% DS corn starch for 10 min at pH 5.8 and 85°C, or pH 4.5 and 95°C, for example. Liquefying activity can be assayed using any of a number of known viscosity assays in the art.

### 4.3. Saccharification

The liquefied starch can be saccharified into a syrup rich in lower DP (*e.g.,* DP1 + DP2) saccharides, using variant amylases, optionally in the presence of another enzyme(s). The exact composition of the products of saccharification depends on the combination of enzymes used, as well as the type of granular starch processed. Advantageously, the syrup obtainable using the provided variant amylases may contain a weight percent of DP2 of the total oligosaccharides in the saccharified starch exceeding 30%, *e.g.,* 45% - 65% or 55% - 65%. The weight percent of (DPI + DP2) in the saccharified starch may exceed about 70%, *e.g.,* 75% - 85% or 80% - 85%. The present amylases also produce a relatively high yield of glucose, *e.g.,* DP1 > 20%, in the syrup product.

Whereas liquefaction is generally run as a continuous process, saccharification is often conducted as a batch process. Saccharification typically is most effective at temperatures of about 60-65°C and a pH of about 4.0-4.5, *e.g.,* pH 4.3, necessitating cooling and adjusting the pH of the liquefied starch. Saccharification may be performed, for example, at a temperature between about 40°C, about 50°C, or about 55°C to about 60°C or about 65°C. Saccharification is normally conducted in stirred tanks, which may take several hours to fill or empty. Enzymes typically are added either at a fixed ratio to dried solids as the tanks are filled or added as a single dose at the commencement of the filling stage. A saccharification reaction to make a syrup typically is run over about 24-72 hours, for example, 24-48 hours. When a maximum or desired DE has been attained, the reaction is stopped by heating to 85°C for 5 min., for example. Further incubation will result in a lower DE, eventually to about 90 DE, as accumulated glucose re-polymerizes to isomaltose and/or other reversion products via an enzymatic reversion reaction and/or with the approach of thermodynamic equilibrium. When using an amylase, saccharification optimally is conducted at a temperature range of about 30°C to about 75°C, *e.g.,* 45°C - 75°C or 47°C - 74°C. The saccharification may be conducted over a pH range of about pH 3 to about pH 7, *e.g.,* pH 3.0 - pH 7.5, pH 3.5 - pH 5.5, pH 3.5, pH 3.8, or pH 4.5.

An amylase may be added to the slurry in the form of a composition. Amylase can be added to a slurry of a granular starch substrate in an amount of about 0.6 - 10 ppm ds, *e.g.,* 2 ppm ds. An amylase can be added as a whole broth, clarified, enriched, partially purified, or purified enzyme. The specific activity of the amylase may be about 300 U/mg of enzyme, for example, measured with the PAHBAH assay. The amylase also can be added as a whole broth product.

An amylase may be added to the slurry as an isolated enzyme solution. For example, an amylase can be added in the form of a cultured cell material produced by host cells expressing an amylase. An amylase may also be secreted by a host cell into the reaction medium during the fermentation or SSF process, such that the enzyme is provided continuously into the reaction. The host cell producing and secreting amylase may also express an additional enzyme, such as a glucoamylase. For example, U.S. Patent No. 5,422,267 discloses the use of a glucoamylase in yeast for production of alcoholic beverages. For example, a host cell, *e.g., Trichoderma reesei* or *Aspergillus niger,* may be engineered to co-express PcuAmyl, or a variant thereof, and a glucoamylase, *e.g.,* a variant GA, *Aspergillus niger* GA, *Aspergillus niger* GA variant, HgGA, HgGA variant, TrGA, or a TrGA variant, during saccharification. The host cell can be genetically modified so as not to express its endogenous glucoamylase and/or other enzymes, proteins or other materials. The host cell can be engineered to express a broad spectrum of various saccharolytic enzymes. For example, the recombinant yeast host cell can comprise nucleic acids encoding a glucoamylase, an alpha-glucosidase, an enzyme that utilizes pentose sugar, an α-amylase, a pullulanase, an isoamylase, and/or an isopullulanase. *See*, *e.g.,* WO 2011/153516 A2.

### 4.4. Isomerization

The soluble starch hydrolysate produced by treatment with amylase can be converted into high fructose starch-based syrup (HFSS), such as high fructose corn syrup (HFCS). This conversion can be achieved using a glucose isomerase, particularly a glucose isomerase immobilized on a solid support. The pH is increased to about 6.0 to about 8.0, *e.g.,* pH 7.5 (depending on the isomerase), and Ca²⁺ is removed by ion exchange. Suitable isomerases include Sweetzyme®, IT (Novozymes A/S); G-zyme® IMGI, and G-zyme® G993, Ketomax®, G-zyme® G993, G-zyme® G993 liquid, and GenSweet® IGI. Following isomerization, the mixture typically contains about 40-45% fructose, *e.g.,* 42% fructose.

### 4.5. Fermentation

The soluble starch hydrolysate, particularly a glucose rich syrup, can be fermented by contacting the starch hydrolysate with a fermenting organism typically at a temperature around 32°C, such as from 30°C to 35°C. Fermentation products include metabolites, including but not limited to organic acids, amino acids, biofuels, and other biochemical, including, but not limited to, ethanol, citric acid, succinic acid, monosodium glutamate, gluconic acid, sodium gluconate, calcium gluconate, potassium gluconate, itaconic acid and other carboxylic acids, glucono delta-lactone, sodium erythorbate, lysine, omega 3 fatty acid, butanol, isoprene, 1,3-propanediol, and biodiesel.

Ethanologenic microorganisms include yeast, such as *Saccharomyces cerevisiae* and bacteria, *e.g., Zymomonas moblis,* expressing alcohol dehydrogenase and pyruvate decarboxylase. The ethanologenic microorganism can express xylose reductase and xylitol dehydrogenase, which convert xylose to xylulose. Improved strains of ethanologenic microorganisms, which can withstand higher temperatures, for example, are known in the art and can be used. *See* Liu et al. (2011) Sheng Wu Gong Cheng Xue Bao 27(7): 1049-56. Commercial sources of yeast include ETHANOL RED® (LeSaffre); Thermosacc® (Lallemand); RED STAR® (Red Star); FERMIOL® (DSM Specialties); and SUPERSTART® (Alltech). Butanologenic microorganisms may include, for example, butanologenic *Clostridia,* such as *Clostridium acetobutylicum, Clostridium beijerinckii, Clostridium saccharobutylicum,* and *Clostridium saccharobutylacetonicum. See, e.g.,* Ezeji et al. (2007) "Bioproduction of butanol from biomass: from genes to bioreactors," Curr. Opin. Biotechnol. 18(3): 220-27. Microorganisms that produce other metabolites, such as citric acid and lactic acid, by fermentation are also known in the art. *See, e.g.,* Papagianni (2007) "Advances in citric acid fermentation by Aspergillus niger: biochemical aspects, membrane transport and modeling," Biotechnol. Adv. 25(3): 244-63; John et al. (2009) "Direct lactic acid fermentation: focus on simultaneous saccharification and lactic acid production," Biotechnol. Adv. 27(2): 145-52.

The saccharification and fermentation processes may be carried out as an SSF process. Fermentation may comprise subsequent enrichment, purification, and recovery of ethanol, for example. During the fermentation, the ethanol content of the broth or "beer" may reach about 8-18% v/v, *e.g.,* 14-15% v/v. The broth may be distilled to produce enriched, *e.g.,* 96% pure, solutions of ethanol. Further, CO₂ generated by fermentation may be collected with a CO₂ scrubber, compressed, and marketed for other uses, *e.g*., carbonating beverage or dry ice production. Solid waste from the fermentation process may be used as protein-rich products, *e.g*., livestock feed.

As mentioned above, an SSF process can be conducted with fungal cells that express and secrete amylase continuously throughout SSF. The fungal cells expressing amylase also can be the fermenting microorganism, *e.g*., an ethanologenic microorganism. Ethanol production thus can be carried out using a fungal cell that expresses sufficient amylase so that less or no enzyme has to be added exogenously. The fungal host cell can be from an appropriately engineered fungal strain. Fungal host cells that express and secrete other enzymes, in addition to PcuAmyl, or a variant, thereof, can also be used. Such cells may express glucoamylase and/or a trehalase, isoamylase, hexokinase, xylanase, glucose isomerase, xylose isomerase, phosphatase, pullulanase, phytase, *beta*-amylase, *alpha*-amylase that is not PcuAmyl, or a variant, thereof, *alpha*-glucosidase, isoamylase, beta-amylase cellulase, xylanase, other hemicellulases, protease, cellulase, lipase, cutinase, *beta*-glucosidase, pectinase, esterase, redox enzymes, transferase, branching enzyme, lyase or other hydrolases, or other enzyme.

A variation on this process is a "fed-batch fermentation" system, where the substrate is added in increments as the fermentation progresses. Fed-batch systems are useful when catabolite repression may inhibit the metabolism of the cells and where it is desirable to have limited amounts of substrate in the medium. The actual substrate concentration in fed-batch systems is estimated by the changes of measurable factors such as pH, dissolved oxygen and the partial pressure of waste gases, such as CO₂. Batch and fed-batch fermentations are common and well known in the art.

Continuous fermentation is an open system where a defined fermentation medium is added continuously to a bioreactor, and an equal amount of conditioned medium is removed simultaneously for processing. Continuous fermentation generally maintains the cultures at a constant high density where cells are primarily in log phase growth. Continuous fermentation permits modulation of cell growth and/or product concentration. For example, a limiting nutrient such as the carbon source or nitrogen source is maintained at a fixed rate and all other parameters are allowed to moderate. Because growth is maintained at a steady state, cell loss due to medium being drawn off should be balanced against the cell growth rate in the fermentation. Methods of optimizing continuous fermentation processes and maximizing the rate of product formation are well known in the art of industrial microbiology.

### 4.6. Compositions Comprising Amylases or Variant Amylases

Amylases or variant amylases as described herein may be combined with a glucoamylase (EC 3.2.1.3), *e.g.,* a *Trichoderma* glucoamylase or variant thereof. An exemplary glucoamylase is *Trichoderma reesei* glucoamylase (TrGA) and variants thereof that possess superior specific activity and thermal stability. *See* U.S. Published Applications Nos. 2006/0094080, 2007/0004018, and 2007/0015266 (Danisco US Inc.). Suitable variants of TrGA include those with glucoamylase activity and at least 80%, at least 90%, or at least 95% sequence identity to wild-type TrGA. Variant amylases advantageously increase the yield of glucose produced in a saccharification process catalyzed by TrGA.

Alternatively, the glucoamylase may be another glucoamylase derived from plants (including algae), fungi, or bacteria. For example, the glucoamylases may be *Aspergillus niger* G1 or G2 glucoamylase or its variants (*e.g.,* Boel et al. (1984) EMBO J. 3: 1097-1102; WO 92/00381; WO 00/04136 (Novo Nordisk A/S)); and A. *awamori* glucoamylase *(e.g.,* WO 84/02921 (Cetus Corp.)). Other contemplated *Aspergillus* glucoamylase include variants with enhanced thermal stability, *e.g.,* G137A and G139A (Chen et al. (1996) Prot. Eng. 9: 499-505); D257E and D293E/Q (Chen et al. (1995) Prot. Eng. 8: 575-582); N182 (Chen et al. (1994) Biochem. J. 301: 275-281); A246C (Fierobe et al. (1996) Biochemistry, 35: 8698-8704); and variants with Pro residues in positions A435 and S436 (Li et al. (1997) Protein Eng. 10: 1199-1204). Other contemplated glucoamylases include *Talaromyces* glucoamylases, in particular derived from *T. emersonii* (*e.g.,* WO 99/28448 (Novo Nordisk A/S), *T. leycettanus* (*e.g.,* U.S. Patent No. RE 32,153 (CPC International, Inc.)), *T. duponti,* or *T. thermophilus* (*e.g.,* U.S. Patent No. 4,587,215). Contemplated bacterial glucoamylases include glucoamylases from the genus *Clostridium,* in particular *C*. *thermoamylolyticum* (*e.g.,* EP 135,138 (CPC International, Inc.) and *C*. *thermohydrosulfuricum* (*e.g.,* WO 86/01831 (Michigan Biotechnology Institute)). Suitable glucoamylases include the glucoamylases derived from *Aspergillus oryzae*, such as a glucoamylase shown in SEQ ID NO:2 in WO 00/04136 (Novo Nordisk A/S). Also suitable are commercial glucoamylases, such as AMG 200L; AMG 300 L; SAN™ SUPER and AMG™ E (Novozymes); OPTIDEX® 300 and OPTIDEX L-400 (Danisco US Inc.); AMIGASE™ and AMIGASE™ PLUS (DSM); G-ZYME® G900 (Enzyme Bio-Systems); and G-ZYME® G990 ZR (*A*. *niger* glucoamylase with a low protease content). Still other suitable glucoamylases include *Aspergillus fumigatus* glucoamylase, *Talaromyces* glucoamylase, *Thielavia* glucoamylase, *Trametes* glucoamylase, *Thermomyces* glucoamylase, *Athelia* glucoamylase, or *Humicola* glucoamylase (*e.g.,* HgGA). Glucoamylases typically are added in an amount of about 0.1- 2 glucoamylase units (GAU)/g ds, *e.g.,* about 0.16 GAU/g ds, 0.23 GAU/g ds, or 0.33 GAU/g ds.

Other suitable enzymes that can be used in combinations with amylase include phytase, protease, pullulanase, β-amylase, isoamylase, a different α-amylase, alpha-glucosidase, cellulase, xylanase, other hemicellulases, beta-glucosidase, transferase, pectinase, lipase, cutinase, esterase, redox enzymes, or a combination thereof. For example, a debranching enzyme, such as an isoamylase (EC 3.2.1.68), may be added in effective amounts well known to the person skilled in the art. A pullulanase (EC 3.2.1.41), *e.g*., PROMOZYME®, is also suitable. Pullulanase typically is added at 100 U/kg ds. Further suitable enzymes include proteases, such as fungal and bacterial proteases. Fungal proteases include those obtained from *Aspergillus,* such as *A*. *niger, A. awamori, A. oryzae; Mucor* (*e.g., M. miehei*); *Rhizopus*; and *Trichoderma.*

β-Amylases (EC 3.2.1.2) are exo-acting maltogenic amylases, which catalyze the hydrolysis of 1,4-α-glucosidic linkages into amylopectin and related glucose polymers, thereby releasing maltose. β-Amylases have been isolated from various plants and microorganisms. *See* Fogarty et al. (1979) in PROGRESS IN INDUSTRIAL MICROBIOLOGY, Vol. 15, pp. 112-115. These β-Amylases have optimum temperatures in the range from 40°C to 65°C and optimum pH in the range from about 4.5 to about 7.0. Contemplated β-amylases include, but are not limited to, β-amylases from barley SPEZYME® BBA 1500, SPEZYME® DBA, OPTIMALT™ ME, OPTIMALT™ BBA (Danisco US Inc.); and NOVOZYM™ WBA (Novozymes A/S) .

Compositions comprising the present amylases may be aqueous or non-aqueous formulations, granules, powders, gels, slurries, pastes, etc., which may further comprise any one or more of the additional enzymes listed, herein, along with buffers, salts, preservatives, water, co-solvents, surfactants, and the like. Such compositions may work in combination with endogenous enzymes or other ingredients already present in a slurry, water bath, washing machine, food or drink product, etc, for example, endogenous plant (including algal) enzymes, residual enzymes from a prior processing step, and the like.

### 4.7. Compositions and Methods for Baking and Food Preparation

The present invention also relates to a "food composition," including but not limited to a food product, animal feed and/or food/feed additives, comprising an amylase, and methods for preparing such a food composition comprising mixing variant amylase with one or more food ingredients, or uses thereof.

Furthermore, the present invention relates to the use of an amylase in the preparation of a food composition, wherein the food composition is baked subsequent to the addition of the polypeptide of the invention. As used herein the term "baking composition" means any composition and/or additive prepared in the process of providing a baked food product, including but not limited to bakers flour, a dough, a baking additive and/or a baked product. The food composition or additive may be liquid or solid.

As used herein, the term "flour" means milled or ground cereal grain. The term "flour" also may mean Sago or tuber products that have been ground or mashed. In some embodiments, flour may also contain components in addition to the milled or mashed cereal or plant matter. An example of an additional component, although not intended to be limiting, is a leavening agent. Cereal grains include wheat, oat, rye, and barley. Tuber products include tapioca flour, cassava flour, and custard powder. The term "flour" also includes ground corn flour, maize-meal, rice flour, whole-meal flour, self-rising flour, tapioca flour, cassava flour, ground rice, enriched flower, and custard powder.

For the commercial and home use of flour for baking and food production, it is important to maintain an appropriate level of α-amylase activity in the flour. A level of activity that is too high may result in a product that is sticky and/or doughy and therefore unmarketable. Flour with insufficient α-amylase activity may not contain enough sugar for proper yeast function, resulting in dry, crumbly bread, or baked products. Accordingly, an amylase, by itself or in combination with another α-amylase(s), may be added to the flour to augment the level of endogenous α-amylase activity in flour.

An amylase can further be added alone or in a combination with other amylases to prevent or retard staling, *i.e.,* crumb firming of baked products. The amount of anti-staling amylase will typically be in the range of 0.01-10 mg of enzyme protein per kg of flour, *e.g.,* 0.5 mg/kg ds. Additional anti-staling amylases that can be used in combination with an amylase include an endo-amylase, *e.g.,* a bacterial endo-amylase from *Bacillus.* The additional amylase can be another maltogenic α-amylase (EC 3.2.1.133), *e.g.,* from *Bacillus.* NOVAMYL® is an exemplary maltogenic α-amylase from *B. stearothermophilus* strain NCIB 11837 and is described in Christophersen et al. (1997) Starch 50: 39-45. Other examples of anti-staling endo-amylases include bacterial α-amylases derived from *Bacillus,* such as *B. licheniformis* or *B. amyloliquefaciens.* The anti-staling amylase may be an exo-amylase, such as β-amylase, *e.g.,* from plant sources, such as soy bean, or from microbial sources, such as *Bacillus.*

The baking composition comprising an amylase further can comprise a phospholipase or enzyme with phospholipase activity. An enzyme with phospholipase activity has an activity that can be measured in Lipase Units (LU). The phospholipase may have A₁ or A₂ activity to remove fatty acid from the phospholipids, forming a lysophospholipid. It may or may not have lipase activity, *i.e.,* activity on triglyceride substrates. The phospholipase typically has a temperature optimum in the range of 30-90°C., *e.g.,* 30-70°C. The added phospholipases can be of animal origin, for example, from pancreas, *e.g*., bovine or porcine pancreas, snake venom or bee venom. Alternatively, the phospholipase may be of microbial origin, *e.g.,* from filamentous fungi, yeast or bacteria, for example.

The phospholipase is added in an amount that improves the softness of the bread during the initial period after baking, particularly the first 24 hours. The amount of phospholipase will typically be in the range of 0.01-10 mg of enzyme protein per kg of flour, *e.g*., 0.1-5 mg/kg. That is, phospholipase activity generally will be in the range of 20-1000 LU/kg of flour, where a Lipase Unit is defined as the amount of enzyme required to release 1 µmol butyric acid per minute at 30°C, pH 7.0, with gum arabic as emulsifier and tributyrin as substrate.

Compositions of dough generally comprise wheat meal or wheat flour and/or other types of meal, flour or starch such as corn flour, cornstarch, rye meal, rye flour, oat flour, oatmeal, soy flour, sorghum meal, sorghum flour, potato meal, potato flour or potato starch. The dough may be fresh, frozen or par-baked. The dough can be a leavened dough or a dough to be subjected to leavening. The dough may be leavened in various ways, such as by adding chemical leavening agents, *e.g.,* sodium bicarbonate or by adding a leaven, *i.e.,* fermenting dough. Dough also may be leavened by adding a suitable yeast culture, such as a culture of *Saccharomyces cerevisiae* (baker's yeast), *e.g.,* a commercially available strain of *S*. *cerevisiae.*

The dough may also comprise other conventional dough ingredients, *e.g*., proteins, such as milk powder, gluten, and soy; eggs (*e.g.,* whole eggs, egg yolks or egg whites); an oxidant, such as ascorbic acid, potassium bromate, potassium iodate, azodicarbonamide (ADA) or ammonium persulfate; an amino acid such as L-cysteine; a sugar; or a salt, such as sodium chloride, calcium acetate, sodium sulfate or calcium sulfate. The dough further may comprise fat, *e.g.,* triglyceride, such as granulated fat or shortening. The dough further may comprise an emulsifier such as mono- or diglycerides, diacetyl tartaric acid esters of mono- or diglycerides, sugar esters of fatty acids, polyglycerol esters of fatty acids, lactic acid esters of monoglycerides, acetic acid esters of monoglycerides, polyoxyethylene stearates, or lysolecithin. In particular, the dough can be made without addition of emulsifiers.

The dough product may be any processed dough product, including fried, deep fried, roasted, baked, steamed and boiled doughs, such as steamed bread and rice cakes. In one embodiment, the food product is a bakery product. Typical bakery (baked) products include bread - such as loaves, rolls, buns, bagels, pizza bases etc. pastry, pretzels, tortillas, cakes, cookies, biscuits, crackers etc.

Optionally, an additional enzyme may be used together with the anti-staling amylase and the phospholipase. The additional enzyme may be a second amylase, such as an amyloglucosidase, a β-amylase, a cyclodextrin glucanotransferase, or the additional enzyme may be a peptidase, in particular an exopeptidase, a transglutaminase, a lipase, a cellulase, a xylanase, a protease, a protein disulfide isomerase, *e.g*., a protein disulfide isomerase as disclosed in WO 95/00636, for example, a glycosyltransferase, a branching enzyme (1,4-α-glucan branching enzyme), a 4-α-glucanotransferase (dextrin glycosyltransferase) or an oxidoreductase, *e.g*., a peroxidase, a laccase, a glucose oxidase, a pyranose oxidase, a lipooxygenase, an L-amino acid oxidase or a carbohydrate oxidase. The additional enzyme(s) may be of any origin, including mammalian and plant, and particularly of microbial (bacterial, yeast or fungal) origin and may be obtained by techniques conventionally used in the art.

The xylanase is typically of microbial origin, *e.g*., derived from a bacterium or fungus, such as a strain of *Aspergillus.* Xylanases include PENTOPAN® and NOVOZYM 384®, for example, which are commercially available xylanase preparations produced from *Trichoderma reesei.* The amyloglucosidase may be an A. *niger* amyloglucosidase (such as AMG®). Other useful amylase products include GRINDAMYL® A 1000 or A 5000 (Grindsted Products, Denmark) and AMYLASE® H or AMYLASE® P (DSM). The glucose oxidase may be a fungal glucose oxidase, in particular an *Aspergillus niger* glucose oxidase (such as GLUZYME®). An exemplary protease is NEUTRASE®.

The process may be used for any kind of baked product prepared from dough, either of a soft or a crisp character, either of a white, light or dark type. Examples are bread, particularly white, whole-meal or rye bread, typically in the form of loaves or rolls, such as, but not limited to, French baguette-type bread, pita bread, tortillas, cakes, pancakes, biscuits, cookies, pie crusts, crisp bread, steamed bread, pizza and the like.

An amylase may be used in a pre-mix, comprising flour together with an anti-staling amylase, a phospholipase, and/or a phospholipid. The pre-mix may contain other dough-improving and/or bread-improving additives, *e.g*., any of the additives, including enzymes, mentioned above. An amylase can be a component of an enzyme preparation comprising an anti-staling amylase and a phospholipase, for use as a baking additive.

The enzyme preparation is optionally in the form of a granulate or agglomerated powder. The preparation can have a narrow particle size distribution with more than 95% (by weight) of the particles in the range from 25 to 500 µm. Granulates and agglomerated powders may be prepared by conventional methods, *e.g.,* by spraying an amylase onto a carrier in a fluid-bed granulator. The carrier may consist of particulate cores having a suitable particle size. The carrier may be soluble or insoluble, *e.g*., a salt (such as NaCl or sodium sulfate), a sugar (such as sucrose or lactose), a sugar alcohol (such as sorbitol), starch, rice, corn grits, or soy.

Enveloped particles, *i.e.,* α-amylase particles, can comprise an amylase. To prepare enveloped α-amylase particles, the enzyme is contacted with a food grade lipid in sufficient quantity to suspend all of the α-amylase particles. Food grade lipids, as used herein, may be any naturally organic compound that is insoluble in water but is soluble in non-polar organic solvents such as hydrocarbon or diethyl ether. Suitable food grade lipids include, but are not limited to, triglycerides either in the form of fats or oils that are either saturated or unsaturated. Examples of fatty acids and combinations thereof which make up the saturated triglycerides include, but are not limited to, butyric (derived from milk fat), palmitic (derived from animal and plant fat), and/or stearic (derived from animal and plant fat). Examples of fatty acids and combinations thereof which make up the unsaturated triglycerides include, but are not limited to, palmitoleic (derived from animal and plant fat), oleic (derived from animal and plant fat), linoleic (derived from plant oils), and/or linolenic (derived from linseed oil). Other suitable food grade lipids include, but are not limited to, monoglycerides and diglycerides derived from the triglycerides discussed above, phospholipids and glycolipids.

The food grade lipid, particularly in the liquid form, is contacted with a powdered form of the α-amylase particles in such a fashion that the lipid material covers at least a portion of the surface of at least a majority, *e.g*., 100% of the α-amylase particles. Thus, each α-amylase particle is individually enveloped in a lipid. For example, all or substantially all of the α-amylase particles are provided with a thin, continuous, enveloping film of lipid. This can be accomplished by first pouring a quantity of lipid into a container, and then slurrying the α-amylase particles so that the lipid thoroughly wets the surface of each α-amylase particle. After a short period of stirring, the enveloped α-amylase particles, carrying a substantial amount of the lipids on their surfaces, are recovered. The thickness of the coating so applied to the particles of α-amylase can be controlled by selection of the type of lipid used and by repeating the operation in order to build up a thicker film, when desired.

The storing, handling and incorporation of the loaded delivery vehicle can be accomplished by means of a packaged mix. The packaged mix can comprise the enveloped α-amylase. However, the packaged mix may further contain additional ingredients as required by the manufacturer or baker. After the enveloped α-amylase has been incorporated into the dough, the baker continues through the normal production process for that product.

The advantages of enveloping the α-amylase particles are two-fold. First, the food grade lipid protects the enzyme from thermal denaturation during the baking process for those enzymes that are heat labile. Consequently, while the α-amylase is stabilized and protected during the proving and baking stages, it is released from the protective coating in the final baked good product, where it hydrolyzes the glucosidic linkages in polyglucans. The loaded delivery vehicle also provides a sustained release of the active enzyme into the baked good. That is, following the baking process, active α-amylase is continually released from the protective coating at a rate that counteracts, and therefore reduces the rate of, staling mechanisms.

In general, the amount of lipid applied to the α-amylase particles can vary from a few percent of the total weight of the α-amylase to many times that weight, depending upon the nature of the lipid, the manner in which it is applied to the α-amylase particles, the composition of the dough mixture to be treated, and the severity of the dough-mixing operation involved.

The loaded delivery vehicle, *i.e.,* the lipid-enveloped enzyme, is added to the ingredients used to prepare a baked good in an effective amount to extend the shelf-life of the baked good. The baker computes the amount of enveloped α-amylase, prepared as discussed above, that will be required to achieve the desired anti-staling effect. The amount of the enveloped α-amylase required is calculated based on the concentration of enzyme enveloped and on the proportion of α-amylase to flour specified. A wide range of concentrations has been found to be effective, although, as has been discussed, observable improvements in anti-staling do not correspond linearly with the α-amylase concentration, but above certain minimal levels, large increases in α-amylase concentration produce little additional improvement. The α-amylase concentration actually used in a particular bakery production could be much higher than the minimum necessary to provide the baker with some insurance against inadvertent under-measurement errors by the baker. The lower limit of enzyme concentration is determined by the minimum anti-staling effect the baker wishes to achieve.

A method of preparing a baked good may comprise: a) preparing lipid-coated α-amylase particles, where substantially all of the α-amylase particles are coated; b) mixing a dough containing flour; c) adding the lipid-coated α-amylase to the dough before the mixing is complete and terminating the mixing before the lipid coating is removed from the α-amylase; d) proofing the dough; and e) baking the dough to provide the baked good, where the α-amylase is inactive during the mixing, proofing and baking stages and is active in the baked good.

The enveloped α-amylase can be added to the dough during the mix cycle, *e.g.,* near the end of the mix cycle. The enveloped α-amylase is added at a point in the mixing stage that allows sufficient distribution of the enveloped α-amylase throughout the dough; however, the mixing stage is terminated before the protective coating becomes stripped from the α-amylase particle(s). Depending on the type and volume of dough, and mixer action and speed, anywhere from one to six minutes or more might be required to mix the enveloped α-amylase into the dough, but two to four minutes is average. Thus, several variables may determine the precise procedure. First, the quantity of enveloped α-amylase should have a total volume sufficient to allow the enveloped α-amylase to be spread throughout the dough mix. If the preparation of enveloped α-amylase is highly concentrated, additional oil may need to be added to the pre-mix before the enveloped α-amylase is added to the dough. Recipes and production processes may require specific modifications; however, good results generally can be achieved when 25% of the oil specified in a bread dough formula is held out of the dough and is used as a carrier for a concentrated enveloped α-amylase when added near the end of the mix cycle. In bread or other baked goods, particularly those having a low fat content, *e.g*., French-style breads, an enveloped α-amylase mixture of approximately 1% of the dry flour weight is sufficient to admix the enveloped α-amylase properly with the dough. The range of suitable percentages is wide and depends on the formula, finished product, and production methodology requirements of the individual baker. Second, the enveloped α-amylase suspension should be added to the mix with sufficient time for complete mixture into the dough, but not for such a time that excessive mechanical action strips the protective lipid coating from the enveloped α-amylase particles.

### 4.8. Textile Desizing Compositions and Use

Variant α-amylases may be formulated into compositions for treating fabrics (*e.g.,* to desize a textile). Fabric-treating methods are well known in the art (*see, e.g.,* U.S. Patent No. 6,077,316). For example, the feel and appearance of a fabric can be improved by a method comprising contacting the fabric with an amylase in a solution. The fabric can be treated with the solution under pressure.

An amylase can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. An amylase can be applied during or after the weaving to remove these sizing starch or starch derivatives. After weaving, an amylase can be used to remove the size coating before further processing the fabric to ensure a homogeneous and wash-proof result.

An amylase can be used alone or with other desizing chemical reagents and/or desizing enzymes to desize fabrics, including cotton-containing fabrics, as detergent additives, *e.g.,* in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of amylolytic enzymes to provide softness to the fabric and make the cotton more accessible to the subsequent enzymatic finishing steps. An amylase can be used in methods of finishing denim garments (*e.g.,* a "bio-stoning process"), enzymatic desizing and providing softness to fabrics, and/or finishing process.

### 4.9. Cleaning Compositions

An amylase polypeptide can be used as a component in detergent compositions for hand washing, laundry washing, dishwashing, and other hard-surface cleaning. Such compositions include heavy duty liquid (HDL), heavy duty dry (HDD), and hand (maual) laundry detergent compositions, including unit dose format laundry detergent compositions, and automatic dishwashing (ADW) and hand (manual) dishwashing compositions, including unit dose format dishwashing compositions.

### 4.9.1. Overview

Typically an amylase is incorporated into detergents at or near a concentration conventionally used for amylase in detergents. For example, an amylase polypeptide may be added in amount corresponding to 0.00001 - 1 mg (calculated as pure enzyme protein) of amylase per liter of wash/dishwash liquor. Exemplary formulations are provided herein, as exemplified by the following:

An amylase polypeptide may be a component of a detergent composition, as the only enzyme or with other enzymes including other amylolytic enzymes. As such, it may be included in the detergent composition in the form of a non-dusting granulate, a stabilized liquid, or a protected enzyme. Non-dusting granulates may be produced, *e.g.,* as disclosed in U.S. Patent Nos. 4,106,991 and 4,661,452 and may optionally be coated by methods known in the art. Examples of waxy coating materials are poly(ethylene oxide) products (polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Other enzyme stabilizers are known in the art. Protected enzymes may be prepared according to the method disclosed in for example EP 238 216. Polyols have long been recognized as stabilizers of proteins, as well as improving protein solubility.

The detergent composition may be in any useful form, *e.g*., as powders, granules, pastes, or liquid. A liquid detergent may be aqueous, typically containing up to about 70% of water and 0% to about 30% of organic solvent. It may also be in the form of a compact gel type containing only about 30% water.

The detergent composition comprises one or more surfactants, each of which may be anionic, nonionic, cationic, or zwitterionic. The detergent will usually contain 0% to about 50% of anionic surfactant, such as linear alkylbenzenesulfonate (LAS); α-olefinsulfonate (AOS); alkyl sulfate (fatty alcohol sulfate) (AS); alcohol ethoxysulfate (AEOS or AES); secondary alkanesulfonates (SAS); α-sulfo fatty acid methyl esters; alkyl- or alkenylsuccinic acid; or soap. The composition may also contain 0% to about 40% of nonionic surfactant such as alcohol ethoxylate (AEO or AE), carboxylated alcohol ethoxylates, nonylphenol ethoxylate, alkylpolyglycoside, alkyldimethylamineoxide, ethoxylated fatty acid monoethanolamide, fatty acid monoethanolamide, or polyhydroxy alkyl fatty acid amide (as described for example in WO 92/06154).

The detergent composition may additionally comprise one or more other enzymes, such as proteases, another amylolytic enzyme, cutinase, lipase, cellulase, pectate lyase, perhydrolase, xylanase, peroxidase, and/or laccase in any combination.

The detergent may contain about 1% to about 65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (*e.g.,* SKS-6 from Hoechst). The detergent may also be unbuilt, *i.e.* essentially free of detergent builder. The enzymes can be used in any composition compatible with the stability of the enzyme. Enzymes generally can be protected against deleterious components by known forms of encapsulation, for example, by granulation or sequestration in hydro gels. Enzymes, and specifically amylases, either with or without starch binding domains, can be used in a variety of compositions including laundry and dishwashing applications, surface cleaners, as well as in compositions for ethanol production from starch or biomass.

The detergent may comprise one or more polymers. Examples include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may contain a bleaching system, which may comprise a H₂O₂ source such as perborate or percarbonate, which may be combined with a peracid-forming bleach activator such as tetraacetylethylenediamine (TAED) or nonanoyloxybenzenesulfonate (NOBS). Alternatively, the bleaching system may comprise peroxyacids (*e.g.,* the amide, imide, or sulfone type peroxyacids). The bleaching system can also be an enzymatic bleaching system, for example, perhydrolase, such as that described in International PCT Application WO 2005/056783.

The enzymes of the detergent composition may be stabilized using conventional stabilizing agents, *e.g.,* a polyol such as propylene glycol or glycerol; a sugar or sugar alcohol; lactic acid; boric acid or a boric acid derivative such as, *e.g.,* an aromatic borate ester; and the composition may be formulated as described in, *e.g.,* WO 92/19709 and WO 92/19708.

The detergent may also contain other conventional detergent ingredients such as *e.g.,* fabric conditioners including clays, foam boosters, suds suppressors, anti-corrosion agents, soil-suspending agents, anti-soil redeposition agents, dyes, bactericides, tarnish inhibiters, optical brighteners, or perfumes.

The pH (measured in aqueous solution at use concentration) is usually neutral or alkaline, *e.g.,* pH about 7.0 to about 11.0.

Particular forms of detergent compositions for inclusion of the present α-amylase are described, below. Many of these composition can be provided in unit dose format for ease of use. Unit dose formulations and packaging are described in, for example, US20090209445A1, US20100081598A1, US7001878B2, EP1504994B1, WO2001085888A2, WO2003089562A1, WO2009098659A1, WO2009098660A1, WO2009112992A1, WO2009124160A1, WO2009152031A1, WO2010059483A1, WO2010088112A1, WO2010090915A1, WO2010135238A1, WO2011094687A1, WO2011094690A1, WO2011127102A1, WO2011163428A1, WO2008000567A1, WO2006045391A1, WO2006007911A1, WO2012027404A1, EP1740690B1, WO2012059336A1, US6730646B1, WO2008087426A1, WO2010116139A1, and WO2012104613A1.

### 4.9.2. Heavy Duty Liquid (HDL) laundry detergent composition

Exemplary HDL laundry detergent compositions includes a detersive surfactant (10%-40% wt/wt), including an anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof), and optionally non-ionic surfactant (selected from a group of linear or branched or random chain, substituted or unsubstituted alkyl alkoxylated alcohol, for example a C₈-C₁₈ alkyl ethoxylated alcohol and/or C₆-C₁₂ alkyl phenol alkoxylates), wherein the weight ratio of anionic detersive surfactant (with a hydrophilic index (HIc) of from 6.0 to 9) to non-ionic detersive surfactant is greater than 1:1. Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition may optionally include, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05wt%-10wt%) and/or random graft polymers (typically comprising of hydrophilic backbone comprising monomers selected from the group consisting of: unsaturated C₁-C₆ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅ alkyl group, polypropylene, polybutylene, vinyl ester of a saturated C₁-C₆ mono-carboxylic acid, C₁-C₆ alkyl ester of acrylic or methacrylic acid, and mixtures thereof.

The composition may include additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), antiredeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition may further include saturated or unsaturated fatty acid, *e.g.,* saturated or unsaturated C₁₂-C₂₄ fatty acid (0 wt% to 10 wt%); deposition aids (examples for which include polysaccharides, *e.g*., cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition may further include dye transfer inhibiting agents, examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents, examples of which include ethylene-diamine-tetraacetic acid (EDTA), diethylene triamine penta methylene phosphonic acid (DTPMP), hydroxy-ethane diphosphonic acid (HEDP), ethylenediamine N,N'-disuccinic acid (EDDS), methyl glycine diacetic acid (MGDA), diethylene triamine penta acetic acid (DTPA), propylene diamine tetracetic acid (PDT A), 2-hydroxypyridine-N-oxide (HPNO), or methyl glycine diacetic acid (MGDA), glutamic acid N,N-diacetic acid (N,N-dicarboxymethyl glutamic acid tetrasodium salt (GLDA), nitrilotriacetic acid (NTA), 4,5-dihydroxy-m-benzenedisulfonic acid, citric acid and any salts thereof, N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), and derivatives thereof.

The composition can further include enzymes (generally about 0.01 wt% active enzyme to 0.03wt% active enzyme) selected from proteases, amylases, lipases, cellulases, choline oxidases, peroxidases/oxidases, pectate lyases, mannanases, cutinases, laccases, phospholipases, lysophospholipases, acyltransferases, perhydrolases, arylesterases, and any mixture thereof. The composition may include an enzyme stabilizer (examples of which include polyols such as propylene glycol or glycerol, sugar or sugar alcohol, lactic acid, reversible protease inhibitor, boric acid, or a boric acid derivative, *e.g*., an aromatic borate ester, or a phenyl boronic acid derivative such as 4-formylphenyl boronic acid).

The composition optionally includes silicone or fatty-acid based suds suppressors; heuing dyes, calcium and magnesium cations, visual signaling ingredients, anti-foam (0.001 wt% to about 4.0wt%), and/or structurant/thickener (0.01 wt% to 5wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

The composition can be any liquid form, for example a liquid or gel form, or any combination thereof. The composition may be in any unit dose form, for example a pouch.

### 4.9.3. Heavy Duty Dry/Solid (HDD) laundry detergent composition

Exemplary HDD laundry detergent compositions includes a detersive surfactant, including anionic detersive surfactants (*e.g.,* linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant *(e.g.,* linear or branched or random chain, substituted or unsubstituted C₈-C₁₈ alkyl ethoxylates, and/or C₆-C₁₂ alkyl phenol alkoxylates), cationic detersive surfactants (*e.g.,* alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (*e.g.,* alkanolamine sulpho-betaines), ampholytic surfactants, semi-polar non-ionic surfactants, and mixtures thereof; builders including phosphate free builders (for example zeolite builders examples which include zeolite A, zeolite X, zeolite P and zeolite MAP in the range of Owt% to less than 10wt%), phosphate builders (for example sodium tri-polyphosphate in the range of Owt% to less than 10wt%), citric acid, citrate salts and nitrilotriacetic acid, silicate salt (*e.g.,* sodium or potassium silicate or sodium meta-silicate in the range of Owt% to less than 10wt%, or layered silicate (SKS-6)); carbonate salt (*e.g.,* sodium carbonate and/or sodium bicarbonate in the range of 0 wt% to less than 80 wt%); and bleaching agents including photobleaches (*e.g.,* sulfonated zinc phthalocyanines, sulfonated aluminum phthalocyanines, xanthenes dyes, and mixtures thereof) hydrophobic or hydrophilic bleach activators (*e.g.,* dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, nonanoyloxybenzene sulfonate-NOBS, nitrile quats, and mixtures thereof), sources of hydrogen peroxide (*e.g.,* inorganic perhydrate salts examples of which include mono or tetra hydrate sodium salt of perborate, percarbonate, persulfate, perphosphate, or persilicate), preformed hydrophilic and/or hydrophobic peracids (*e.g.,* percarboxylic acids and salts, percarbonic acids and salts, perimidic acids and salts, peroxymonosulfuric acids and salts, and mixtures thereof), and/or bleach catalysts (*e.g*., imine bleach boosters (examples of which include iminium cations and polyions), iminium zwitterions, modified amines, modified amine oxides, N-sulphonyl imines, N-phosphonyl imines, N-acyl imines, thiadiazole dioxides, perfluoroimines, cyclic sugar ketones, and mixtures thereof, and metal-containing bleach catalysts (*e.g.,* copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid), and watersoluble salts thereof).

The composition can include enzymes, *e.g*., proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and any mixture thereof.

The composition may optionally include additional detergent ingredients including perfume microcapsules, starch encapsulated perfume accord, hueing agents, additional polymers, including fabric integrity and cationic polymers, dye-lock ingredients, fabric-softening agents, brighteners (for example C.I. Fluorescent brighteners), flocculating agents, chelating agents, alkoxylated polyamines, fabric deposition aids, and/or cyclodextrin.

### 4.9.4. Automatic dishwashing (ADW) detergent composition

Exemplary ADW detergent composition includes non-ionic surfactants, including ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% including phosphate builders (*e.g.,* mono-phosphates, di-phosphates, tri-polyphosphates, other oligomeric-poylphosphates, sodium tripolyphosphate-STPP) and phosphate-free builders (*e.g*., amino acid-based compounds including methylglycine-diacetic acid (MGDA) and salts and derivatives thereof, glutamic-N,N-diacetic acid (GLDA) and salts and derivatives thereof, iminodisuccinic acid (IDS) and salts and derivatives thereof, carboxy methyl inulin and salts and derivatives thereof, nitrilotriacetic acid (NTA), diethylene triamine penta acetic acid (DTPA), B-alaninediacetic acid (B-ADA) and their salts, homopolymers and copolymers of poly-carboxylic acids and their partially or completely neutralized salts, monomeric polycarboxylic acids and hydroxycarboxylic acids and their salts in the range of 0.5% to 50% by weight; sulfonated/carboxylated polymers in the range of about 0.1 % to about 50% by weight to provide dimensional stability; drying aids in the range of about 0.1 % to about 10% by weight (*e.g.,* polyesters, especially anionic polyesters, optionally together with further monomers with 3 to 6 functionalities - typically acid, alcohol or ester functionalities which are conducive to polycondensation, polycarbonate-, polyurethane- and/or polyurea-polyorganosiloxane compounds or precursor compounds, thereof, particularly of the reactive cyclic carbonate and urea type); silicates in the range from about 1 % to about 20% by weight (including sodium or potassium silicates for example sodium disilicate, sodium meta-silicate and crystalline phyllosilicates); inorganic bleach (*e.g.,* perhydrate salts such as perborate, percarbonate, perphosphate, persulfate and persilicate salts) and organic bleach (*e.g.,* organic peroxyacids, including diacyl and tetraacylperoxides, especially diperoxydodecanedioc acid, diperoxytetradecanedioc acid, and diperoxyhexadecanedioc acid); bleach activators (*i.e.,* organic peracid precursors in the range from about 0.1 % to about 10% by weight); bleach catalysts (*e.g.,* manganese triazacyclononane and related complexes, Co, Cu, Mn, and Fe bispyridylamine and related complexes, and pentamine acetate cobalt(III) and related complexes); metal care agents in the range from about 0.1% to 5% by weight (*e.g.,* benzatriazoles, metal salts and complexes, and/or silicates); enzymes in the range from about 0.01 to 5.0 mg of active enzyme per gram of automatic dishwashing detergent composition (*e.g.,* proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and mixtures thereof); and enzyme stabilizer components (*e.g.,* oligosaccharides, polysaccharides, and inorganic divalent metal salts).

### 4.9.5. Additional detergent compositions

Additional exemplary detergent formulations to which the present amylase can be added are described, below, in the numbered paragraphs.
1) A detergent composition formulated as a granulate having a bulk density of at least 600 g/L comprising linear alkylbenzenesulfonate (calculated as acid) about 7% to about 12%; alcohol ethoxysulfate (*e.g.,* C₁₂₋₁₈ alcohol, 1-2 ethylene oxide (EO)) or alkyl sulfate (*e.g.,* C₁₆₋₁₈) about 1% to about 4%; alcohol ethoxylate (*e.g*., C₁₄₋₁₅ alcohol, 7 EO) about 5% to about 9%; sodium carbonate (*e.g.,* Na₂CO₃) about 14% to about 20%; soluble silicate (*e.g.,* Na₂O, 2SiO₂) about 2 to about 6%; zeolite (*e.g*., NaA1SiO₄) about 15% to about 22%; sodium sulfate *(e.g.,* Na₂SO₄) 0% to about 6%; sodium citrate/citric acid (*e.g.,* C₆H₅Na₃O₇/C₆H₈O₇) about 0% to about 15%; sodium perborate (*e.g.,* NaBO₃H₂O) about 11% to about 18%; TAED about 2% to about 6%; carboxymethylcellulose (CMC) and 0% to about 2%; polymers (*e.g.,* maleic/acrylic acid, copolymer, PVP, PEG) 0-3%; enzymes (calculated as pure enzyme) 0.0001-0.1% protein; and minor ingredients (*e.g.,* suds suppressors, perfumes, optical brightener, photobleach) 0-5%.
2) A detergent composition formulated as a granulate having a bulk density of at least 600 g/L comprising linear alkylbenzenesulfonate (calculated as acid) about 6% to about 11%; alcohol ethoxysulfate (*e.g.,* C₁₂₋₁₈ alcohol, 1-2 EO) or alkyl sulfate (*e.g.,* C₁₆₋₁₈) about 1% to about 3%; alcohol ethoxylate (*e.g.,* C₁₄₋₁₅ alcohol, 7 EO) about 5% to about 9%; sodium carbonate (*e.g.,* Na₂CO₃) about 15% to about 21%; soluble silicate (*e.g.,* Na₂O, 2SiO₂) about 1% to about 4%; zeolite (*e.g.,* NaA1SiO₄) about 24% to about 34%; sodium sulfate (*e.g,.* Na₂SO₄) about 4% to about 10%; sodium citrate/citric acid (*e.g.,* C₆H₅Na₃O₇/ C₆H₈O₇) 0% to about 15%; carboxymethylcellulose (CMC) 0% to about 2%; polymers (*e.g.,* maleic/acrylic acid copolymer, PVP, PEG) 1-6%; enzymes (calculated as pure enzyme protein) 0.0001-0.1%; minor ingredients *(e.g.,* suds suppressors, perfume) 0-5%.
3) A detergent composition formulated as a granulate having a bulk density of at least 600 g/L comprising linear alkylbenzenesulfonate (calculated as acid) about 5% to about 9%; alcohol ethoxylate (*e.g.,* C₁₂₋₁₅ alcohol, 7 EO) about 7% to about 14%; Soap as fatty acid (*e.g.,* C₁₆₋₂₂ fatty acid) about 1 to about 3%; sodium carbonate (as Na₂CO₃) about 10% to about 17%; soluble silicate (*e.g.,* Na₂O, 2SiO₂) about 3% to about 9%; zeolite (as NaA1SiO₄) about 23% to about 33%; sodium sulfate *(e.g.,* Na₂SO₄) 0% to about 4%; sodium perborate (*e.g.,* NaBO₃H₂O) about 8% to about 16%; TAED about 2% to about 8%; phosphonate (*e.g.,* EDTMPA) 0% to about 1%; carboxymethylcellulose (CMC) 0% to about 2%; polymers (*e.g.,* maleic/acrylic acid copolymer, PVP, PEG) 0-3%; enzymes (calculated as pure enzyme protein) 0.0001-0.1%; minor ingredients (*e.g.,* suds suppressors, perfume, optical brightener) 0-5%.
4) A detergent composition formulated as a granulate having a bulk density of at least 600 g/L comprising linear alkylbenzenesulfonate (calculated as acid) about 8% to about 12%; alcohol ethoxylate (*e.g.,* C₁₂₋₁₅ alcohol, 7 EO) about 10% to about 25%; sodium carbonate (as Na₂CO₃) about 14% to about 22%; soluble silicate (*e.g.,* Na₂O, 2SiO₂) about 1% to about 5%; zeolite (*e.g.,* NaA1SiO₄) about 25% to about 35%; sodium sulfate (*e.g.,* Na₂SO₄) 0% to about 10%; carboxymethylcellulose (CMC) 0% to about 2%; polymers (*e.g.,* maleic/acrylic acid copolymer, PVP, PEG) 1-3%; enzymes (calculated as pure enzyme protein) 0.0001-0.1%; and minor ingredients (*e.g.,* suds suppressors, perfume) 0-5%.
5) An aqueous liquid detergent composition comprising linear alkylbenzenesulfonate (calculated as acid) about 15% to about 21%; alcohol ethoxylate (*e.g.,* C₁₂₋₁₅ alcohol, 7 EO or C₁₂₋₁₅ alcohol, 5 EO) about 12% to about 18%; soap as fatty acid (*e.g.,* oleic acid) about 3% to about 13%; alkenylsuccinic acid (C₁₂₋₁₄) 0% to about 13%; aminoethanol about 8% to about 18%; citric acid about 2% to about 8%; phosphonate 0% to about 3%; polymers (*e.g.,* PVP, PEG) 0% to about 3%; borate (*e.g.,* B₄O₇) 0% to about 2%; ethanol 0% to about 3%; propylene glycol about 8% to about 14%; enzymes (calculated as pure enzyme protein) 0.0001-0.1%; and minor ingredients (*e.g.,* dispersants, suds suppressors, perfume, optical brightener) 0-5%.
6) An aqueous structured liquid detergent composition comprising linear alkylbenzenesulfonate (calculated as acid) about 15% to about 21%; alcohol ethoxylate (*e.g.,* C₁₂₋₁₅ alcohol, 7 EO, or C₁₂₋₁₅ alcohol, 5 EO) 3-9%; soap as fatty acid *(e.g.,* oleic acid) about 3% to about 10%; zeolite (as NaA1SiO₄) about 14% to about 22%; potassium citrate about 9% to about 18%; borate (*e.g.,* B₄O₇) 0% to about 2%; carboxymethylcellulose (CMC) 0% to about 2%; polymers *(e.g.,* PEG, PVP) 0% to about 3%; anchoring polymers such as, *e.g.,* lauryl methacrylate/acrylic acid copolymer; molar ratio 25:1, MW 3800) 0% to about 3%;glycerol 0% to about 5%; enzymes (calculated as pure enzyme protein) 0.0001-0.1%; and minor ingredients (*e.g.,* dispersants, suds suppressors, perfume, optical brighteners) 0-5%.
7) A detergent composition formulated as a granulate having a bulk density of at least 600 g/L comprising fatty alcohol sulfate about 5% to about 10%; ethoxylated fatty acid monoethanolamide about 3% to about 9%; soap as fatty acid 0-3%; sodium carbonate (*e.g*., Na₂CO₃) about 5% to about 10%; Soluble silicate (*e.g*., Na₂O, 2SiO₂) about 1% to about 4%; zeolite (*e.g.,* NaAlSiO₄) about 20% to about 40%; Sodium sulfate (*e.g.,* Na₂SO₄) about 2% to about 8%; sodium perborate (*e.g*., NaBO₃H₂O) about 12% to about 18%; TAED about 2% to about 7%; polymers (*e.g*., maleic/acrylic acid copolymer, PEG) about 1% to about 5%; enzymes (calculated as pure enzyme protein) 0.0001-0.1%; and minor ingredients (*e.g.,* optical brightener, suds suppressors, perfume) 0-5%.
8) A detergent composition formulated as a granulate comprising linear alkylbenzenesulfonate (calculated as acid) about 8% to about 14%; ethoxylated fatty acid monoethanolamide about 5% to about 11%; soap as fatty acid 0% to about 3%; sodium carbonate (*e.g*., Na₂CO₃) about 4% to about 10%; soluble silicate (Na₂O, 2SiO₂) about 1% to about 4%; zeolite (*e.g.,* NaAlSiO₄) about 30% to about 50%; sodium sulfate (*e.g.,* Na₂SO₄) about 3% to about 11%; sodium citrate (*e.g*., C₆H₅Na₃O₇) about 5% to about 12%; polymers *(e.g.,* PVP, maleic/acrylic acid copolymer, PEG) about 1% to about 5%; enzymes (calculated as pure enzyme protein) 0.0001-0.1%; and minor ingredients (*e.g*., suds suppressors, perfume) 0-5%.
9) A detergent composition formulated as a granulate comprising linear alkylbenzenesulfonate (calculated as acid) about 6% to about 12%; nonionic surfactant about 1% to about 4%; soap as fatty acid about 2% to about 6%; sodium carbonate (*e.g*., Na₂CO₃) about 14% to about 22%; zeolite (*e.g.,* NaAlSiO₄) about 18% to about 32%; sodium sulfate (*e.g.,* Na₂SO₄) about 5% to about 20%; sodium citrate (e.g., C₆H₅Na₃O₇) about 3% to about 8%; sodium perborate (*e.g.,* NaBO₃H₂O) about 4% to about 9%; bleach activator (*e.g.,* NOBS or TAED) about 1% to about 5%; carboxymethylcellulose (CMC) 0% to about 2%; polymers (*e.g.,* polycarboxylate or PEG) about 1% to about 5%; enzymes (calculated as pure enzyme protein) 0.0001-0.1%; and minor ingredients (*e.g*., optical brightener, perfume) 0-5%.
10) An aqueous liquid detergent composition comprising linear alkylbenzenesulfonate (calculated as acid) about 15% to about 23%; alcohol ethoxysulfate (*e.g*., C₁₂₋₁₅ alcohol, 2-3 EO) about 8% to about 15%; alcohol ethoxylate (*e.g*., C₁₂₋₁₅ alcohol, 7 EO, or C₁₂₋₁₅ alcohol, 5 EO) about 3% to about 9%; soap as fatty acid (*e.g.,* lauric acid) 0% to about 3%; aminoethanol about 1% to about 5%; sodium citrate about 5% to about 10%; hydrotrope (*e.g.,* sodium toluensulfonate) about 2% to about 6%; borate (*e.g.,* B₄O₇) 0% to about 2%; carboxymethylcellulose 0% to about 1%; ethanol about 1% to about 3%; propylene glycol about 2% to about 5%; enzymes (calculated as pure enzyme protein) 0.0001-0.1%; and minor ingredients (e.g., polymers, dispersants, perfume, optical brighteners) 0-5%.
11) An aqueous liquid detergent composition comprising linear alkylbenzenesulfonate (calculated as acid) about 20% to about 32%; alcohol ethoxylate (e.g., C₁₂₋₁₅ alcohol, 7 EO, or C₁₂₋₁₅ alcohol, 5 EO) 6-12%; aminoethanol about 2% to about 6%; citric acid about 8% to about 14%; borate (*e.g.,* B₄O₇) about 1% to about 3%; polymer *(e.g.,* maleic/acrylic acid copolymer, anchoring polymer such as, *e.g.,* lauryl methacrylate/acrylic acid copolymer) 0% to about 3%; glycerol about 3% to about 8%; enzymes (calculated as pure enzyme protein) 0.0001-0.1%; and minor ingredients (*e.g*., hydrotropes, dispersants, perfume, optical brighteners) 0-5%.
12) A detergent composition formulated as a granulate having a bulk density of at least 600 g/L comprising anionic surfactant (linear alkylbenzenesulfonate, alkyl sulfate, α-olefinsulfonate, α-sulfo fatty acid methyl esters, alkanesulfonates, soap) about 25% to about 40%; nonionic surfactant (*e.g*., alcohol ethoxylate) about 1% to about 10%; sodium carbonate *(e.g.,* Na₂CO₃) about 8% to about 25%; soluble silicates (*e.g.,* Na₂O, 2SiO₂) about 5% to about 15%; sodium sulfate (*e.g*., Na₂SO₄) 0% to about 5%; zeolite (NaAlSiO₄) about 15% to about 28%; sodium perborate (*e.g*., NaBO₃·4H₂O) 0% to about 20%; bleach activator (TAED or NOBS) about 0% to about 5%; enzymes (calculated as pure enzyme protein) 0.0001-0.1%; minor ingredients (*e.g*., perfume, optical brighteners) 0-3%.
13) Detergent compositions as described in compositions 1)-12) *supra,* wherein all or part of the linear alkylbenzenesulfonate is replaced by (C₁₂-C₁₈) alkyl sulfate.
14) A detergent composition formulated as a granulate having a bulk density of at least 600 g/L comprising (C₁₂-C₁₈) alkyl sulfate about 9% to about 15%; alcohol ethoxylate about 3% to about 6%; polyhydroxy alkyl fatty acid amide about 1% to about 5%; zeolite *(e.g.,* NaAlSiO₄) about 10% to about 20%; layered disilicate (*e.g*., SK56 from Hoechst) about 10% to about 20%; sodium carbonate (*e.g.,* Na₂CO₃) about 3% to about 12%; soluble silicate *(e.g.,* Na₂O, 2SiO₂) 0% to about 6%; sodium citrate about 4% to about 8%; sodium percarbonate about 13% to about 22%; TAED about 3% to about 8%; polymers (*e.g*., polycarboxylates and PVP) 0% to about 5%; enzymes (calculated as pure enzyme protein) 0.0001-0.1%; and minor ingredients (*e.g*., optical brightener, photobleach, perfume, suds suppressors) 0-5%.
15) A detergent composition formulated as a granulate having a bulk density of at least 600 g/L comprising (C₁₂-C₁₈) alkyl sulfate about 4% to about 8%; alcohol ethoxylate about 11% to about 15%; soap about 1% to about 4%; zeolite MAP or zeolite A about 35% to about 45%; sodium carbonate (as Na₂CO₃) about 2% to about 8%; soluble silicate (*e.g*., Na₂O, 2SiO₂) 0% to about 4%; sodium percarbonate about 13% to about 22%; TAED 1-8%; carboxymethylcellulose (CMC) 0% to about 3%; polymers (*e.g*., polycarboxylates and PVP) 0% to about 3%; enzymes (calculated as pure enzyme protein) 0.0001-0.1%; and minor ingredients *(e.g.,* optical brightener, phosphonate, perfume) 0-3%.
16) Detergent formulations as described in 1)-15) supra, which contain a stabilized or encapsulated peracid, either as an additional component or as a substitute for already specified bleach systems.
17) Detergent compositions as described supra in 1), 3), 7), 9), and 12), wherein perborate is replaced by percarbonate.
18) Detergent compositions as described supra in 1), 3), 7), 9), 12), 14), and 15), which additionally contain a manganese catalyst. The manganese catalyst for example is one of the compounds described in "Efficient manganese catalysts for low-temperature bleaching," Nature 369: 637-639 (1994).
19) Detergent composition formulated as a non-aqueous detergent liquid comprising a liquid nonionic surfactant such as, *e.g.,* linear alkoxylated primary alcohol, a builder system (e.g., phosphate), an enzyme(s), and alkali. The detergent may also comprise anionic surfactant and/or a bleach system.

As above, the present amylase polypeptide may be incorporated at a concentration conventionally employed in detergents. It is at present contemplated that, in the detergent composition, the enzyme may be added in an amount corresponding to 0.00001-1.0 mg (calculated as pure enzyme protein) of amylase polypeptide per liter of wash liquor.

The detergent composition may also contain other conventional detergent ingredients, *e.g*., deflocculant material, filler material, foam depressors, anti-corrosion agents, soil-suspending agents, sequestering agents, anti-soil redeposition agents, dehydrating agents, dyes, bactericides, fluorescers, thickeners, and perfumes.

The detergent composition may be formulated as a hand (manual) or machine (automatic) laundry detergent composition, including a laundry additive composition suitable for pre-treatment of stained fabrics and a rinse added fabric softener composition, or be formulated as a detergent composition for use in general household hard surface cleaning operations, or be formulated for manual or automatic dishwashing operations.

Any of the cleaning compositions described, herein, may include any number of additional enzymes. In general the enzyme(s) should be compatible with the selected detergent, (*e.g.,* with respect to pH-optimum, compatibility with other enzymatic and non-enzymatic ingredients, and the like), and the enzyme(s) should be present in effective amounts. The following enzymes are provided as examples.

*Proteases:* Suitable proteases include those of animal, vegetable or microbial origin. Chemically modified or protein engineered mutants are included, as well as naturally processed proteins. The protease may be a serine protease or a metalloprotease, an alkaline microbial protease, a trypsin-like protease, or a chymotrypsin-like protease. Examples of alkaline proteases are subtilisins, especially those derived from *Bacillus, e.g.,* subtilisin Novo, subtilisin Carlsberg, subtilisin 309, subtilisin 147, and subtilisin 168 (*see, e.g.,* WO 89/06279). Additional examples include those mutant proteases described in U.S. Pat. Nos. RE 34,606, 5,955,340, 5,700,676, 6,312,936, and 6,482,628. Examples of trypsin-like proteases are trypsin (*e.g.,* of porcine or bovine origin), and *Fusarium* proteases (*see, e.g.,* WO 89/06270 and WO 94/25583). Examples of useful proteases also include but are not limited to the variants described in WO 92/19729, WO 98/20115, WO 98/20116, and WO 98/34946. Commercially available protease enzymes include but are not limited to: ALCALASE®, SAVINASE®, PRIMASE™, DURALASE™, ESPERASE®, BLAZE™, POLARZYME®, OVOZYME®, KANNASE®, LIQUANASE®, NEUTRASE®, RELASE®, and ESPERASE® (Novo Nordisk A/S and Novozymes A/S), MAXATASE®, MAXACAL™, MAXAPEM™, PROPERASE®, PURAFECT®, PURAFECT OXP™, PURAFECT PRIME™, FNA™, FN2™, FN3™, OPTICLEAN®, OPTIMASE®, PURAMAX™, EXCELLASE™, and PURAFAST™ (Danisco US, Inc./DuPont Industrial Biosciences, Palo Alto, California, USA), BLAP™ and BLAP™ variants (Henkel Kommanditgesellschaft auf Aktien, Duesseldorf, Germany), and KAP (B. alkalophilus subtilisin; Kao Corp., Tokyo, Japan). Another exemplary proteases NprE from *Bacillus amyloliquifaciens* and ASP from *Cellulomonas* sp. strain 69B4 (Danisco US, Inc./DuPont Industrial Biosciences, Palo Alto, California, USA). Various proteases are described in WO95/23221, WO 92/21760, WO 09/149200, WO 09/149144, WO 09/149145, WO 11/072099, WO 10/056640, WO 10/056653, WO 11/140364, WO 12/151534, U.S. Pat. Publ. No. 2008/0090747, and U.S. Pat. Nos. 5,801,039, 5,340,735, 5,500,364, 5,855,625, US RE 34,606, 5,955,340, 5,700,676, 6,312,936, and 6,482,628, and various other patents. In some further embodiments, metalloproteases find use in the present invention, including but not limited to the neutral metalloprotease described in WO 07/044993. Suitable proteases include naturally occuring proteases or engineered variants specifically selected or engineered to work at relatively low temperatures.

*Lipases:* Suitable lipases include those of bacterial or fungal origin. Chemically modified, proteolytically modified, or protein engineered mutants are included. Examples of useful lipases include but are not limited to lipases from *Humicola* (synonym *Thermomyces*), *e.g.,* from *H. lanuginosa* (*T. lanuginosus*) (*see e.g.,* EP 258068 and EP 305216), from *H. insolens* (*see e.g.,* WO 96/13580); a *Pseudomonas* lipase (*e.g.,* from *P. alcaligenes* or *P. pseudoalcaligenes*; *see, e.g.,* EP 218 272), *P. cepacia* (*see e.g.,* EP 331 376), *P. stutzeri* (*see e.g.,* GB 1,372,034), *P. fluorescens, Pseudomonas* sp. strain SD 705 (*see e.g.,* WO 95/06720 and WO 96/27002), *P. wisconsinensis* (*see e.g.,* WO 96/12012); a *Bacillus* lipase *(e.g.,* from *B. subtilis; see e.g.,* Dartois et al. Biochemica et Biophysica Acta, 1131: 253-360 (1993)), *B. stearothermophilus* (*see e.g.,* JP 64/744992), or *B. pumilus* (*see e.g.,* WO 91/16422). Additional lipase variants contemplated for use in the formulations include those described for example in: WO 92/05249, WO 94/01541, WO 95/35381, WO 96/00292, WO 95/30744, WO 94/25578, WO 95/14783, WO 95/22615, WO 97/04079, WO 97/07202, EP 407225, and EP 260105. Some commercially available lipase enzymes include LIPOLASE® and LIPOLASE ULTRA™ (Novo Nordisk A/S and Novozymes A/S).

*Polyesterases:* Suitable polyesterases can be included in the composition, such as those described in, for example, WO 01/34899, WO 01/14629, and US6933140.

Amylases: The compositions can be combined with other amylases, such as non-production enhanced amylase. These can include commercially available amylases, such as but not limited to STAINZYME®, NATALASE®, DURAMYL®, TERMAMYL®, FUNGAMYL® and BAN™ (Novo Nordisk A/S and Novozymes A/S); RAPIDASE®, POWERASE®, and PURASTAR® (from Danisco US Inc.).

*Cellulases:* Cellulases can be added to the compositions. Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed for example in U.S. Patent Nos. 4,435,307; 5,648,263; 5,691,178; 5,776,757; and WO 89/09259. Exemplary cellulases contemplated for use are those having color care benefit for the textile. Examples of such cellulases are cellulases described in for example EP 0495257, EP 0531372, WO 96/11262, WO 96/29397, and WO 98/08940. Other examples are cellulase variants, such as those described in WO 94/07998; WO 98/12307; WO 95/24471; PCT/DK98/00299; EP 531315; U.S. Patent Nos. 5,457,046; 5,686,593; and 5,763,254. Commercially available cellulases include CELLUZYME® and CAREZYME® (Novo Nordisk A/S and Novozymes A/S); CLAZINASE® and PURADAX HA® (Danisco US Inc.); and KAC-500(B)™ (Kao Corporation).

*Peroxidases*/*Oxidases*: Suitable peroxidases/oxidases contemplated for use in the compositions include those of plant, bacterial or fungal origin. Chemically modified or protein engineered mutants are included. Examples of useful peroxidases include peroxidases from *Coprinus, e.g.,* from C. *cinereus,* and variants thereof as those described in WO 93/24618, WO 95/10602, and WO 98/15257. Commercially available peroxidases include for example GUARDZYME™ (Novo Nordisk A/S and Novozymes A/S).

The detergent composition can also comprise 2,6-β-D-fructan hydrolase, which is effective for removal/cleaning of biofilm present on household and/or industrial textile/laundry.

The detergent enzyme(s) may be included in a detergent composition by adding separate additives containing one or more enzymes, or by adding a combined additive comprising all of these enzymes. A detergent additive, *i.e.* a separate additive or a combined additive, can be formulated *e.g*., as a granulate, a liquid, a slurry, and the like. Exemplary detergent additive formulations include but are not limited to granulates, in particular non-dusting granulates, liquids, in particular stabilized liquids or slurries.

Non-dusting granulates may be produced, *e.g.,* as disclosed in U.S. Patent Nos. 4,106,991 and 4,661,452 and may optionally be coated by methods known in the art. Examples of waxy coating materials are poly(ethylene oxide) products (*e.g*., polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition may be in any convenient form, *e.g.,* a bar, a tablet, a powder, a granule, a paste, or a liquid. A liquid detergent may be aqueous, typically containing up to about 70% water, and 0% to about 30% organic solvent. Compact detergent gels containing about 30% or less water are also contemplated. The detergent composition can optionally comprise one or more surfactants, which may be non-ionic, including semi-polar and/or anionic and/or cationic and/or zwitterionic. The surfactants can be present in a wide range, from about 0.1% to about 60% by weight.

When included therein the detergent will typically contain from about 1% to about 40% of an anionic surfactant, such as linear alkylbenzenesulfonate, α-olefinsulfonate, alkyl sulfate (fatty alcohol sulfate), alcohol ethoxysulfate, secondary alkanesulfonate, α-sulfo fatty acid methyl ester, alkyl- or alkenylsuccinic acid, or soap.

When included therein, the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl-N-alkyl derivatives of glucosamine ("glucamides").

The detergent may contain 0% to about 65% of a detergent builder or complexing agent such as zeolite, diphosphate, triphosphate, phosphonate, carbonate, citrate, nitrilotriacetic acid, ethylenediaminetetraacetic acid (EDTA), diethylenetriaminepentaacetic acid, alkyl- or alkenylsuccinic acid, soluble silicates or layered silicates (*e.g*.,SKS-6 from Hoechst).

The detergent may comprise one or more polymers. Exemplary polymers include carboxymethylcellulose (CMC), poly(vinylpyrrolidone) (PVP), poly(ethylene glycol) (PEG), poly(vinyl alcohol) (PVA), poly(vinylpyridine-N-oxide), poly(vinylimidazole), polycarboxylates *e.g*., polyacrylates, maleic/acrylic acid copolymers), and lauryl methacrylate/acrylic acid copolymers.

The enzyme(s) of the detergent composition may be stabilized using conventional stabilizing agents, *e.g.,* as polyol (*e.g.,* propylene glycol or glycerol), a sugar or sugar alcohol, lactic acid, boric acid, or a boric acid derivative (e.g., an aromatic borate ester), or a phenyl boronic acid derivative (*e.g*., 4-formylphenyl boronic acid). The composition may be formulated as described in WO 92/19709 and WO 92/19708.

It is contemplated that in the detergent compositions, in particular the enzyme variants, may be added in an amount corresponding to about 0.01 to about 100 mg of enzyme protein per liter of wash liquor (*e.g*., about 0.05 to about 5.0 mg of enzyme protein per liter of wash liquor or 0.1 to about 1.0 mg of enzyme protein per liter of wash liquor).

Yet additional exemplary detergent formulations to which the present amylase can be added (or is in some cases identified as a component) are listed in the following Tables:

Numerous exemplary detergent formulations to which the present amylases can be added (or is in some cases are identified as a component of) are described in WO2013063460. These include commercially available unit dose detergent formulations/packages such as PUREX® UltraPacks (Henkel), FINISH® Quantum (Reckitt Benckiser), CLOROX™ 2 Packs (Clorox), OxiClean Max Force Power Paks (Church & Dwight), TIDE® Stain Release, CASCADE® ActionPacs, and TIDE® Pods™ (Procter & Gamble), PS.

### 4.9.6. Methods of Assessing Amylase Activity in Detergent Compositions

Numerous α-amylase cleaning assays are known in the art, including swatch and micro-swatch assays. The appended Examples describe only a few such assays.

In order to further illustrate the compositions and methods, and advantages thereof, the following specific examples are given with the understanding that they are illustrative rather than limiting.

### 4.10. Brewing Compositions

A PcuAmyl variant may be a component of a brewing composition used in a process of providing a fermented beverage, such as brewing. It is believed that non-fermentable carbohydrates form the majority of the dissolved solids in the final beer. This residue remains because of the inability of malt amylases to hydrolyze the alpha-1,6-linkages of the starch. The non-fermentable carbohydrates contribute about 50 calories per 12 ounces (about 340 grams) of beer. The PcuAmy1 or variant thereof, usually in combination with a glucoamylase and optionally a pullulanase and/or isoamylase, assist in converting the starch into dextrins and fermentable sugars, lowering the residual non-fermentable carbohydrates in the final beer.

The principal raw materials used in making these beverages are water, hops and malt. In addition, but also exclusively, adjuncts such as common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, and syrups, such as corn syrup, sugar cane syrup, inverted sugar syrup, barley and/or wheat syrups, and the like may be used as a source of starch.

For a number of reasons, the malt, which is produced principally from selected varieties of barley, has an important effect on the overall character and quality of the beer. First, the malt is the primary flavoring agent in beer. Second, the malt provides the major portion of the fermentable sugar. Third, the malt provides the proteins, which will contribute to the body and foam character of the beer. Fourth, the malt provides the necessary enzymatic activity during mashing. Hops also contribute significantly to beer quality, including flavoring. In particular, hops (or hops constituents) add desirable bittering substances to the beer. In addition, the hops act as protein precipitants, establish preservative agents and aid in foam formation and stabilization.

Cereals (grains), such as barley, oats, wheat, but also corn and rice are often used for brewing, both in industry and for home brewing, but also other plant components, such as hops are often added. The components used in brewing may be unmalted or may be malted, *i.e.,* partially germinated, resulting in an increase in the levels of enzymes, including α-amylase. For successful brewing, adequate levels of α-amylase enzyme activity are necessary for formation of the appropriate levels of sugars for fermentation. PcuAmy1 amylase, or a variant thereof, by itself or in combination with another α-amylase(s), accordingly may be added to the components used for brewing.

As used herein, the term "stock" means grains and plant components that are crushed or broken. For example, barley used in beer production is a grain that has been coarsely ground or crushed to yield a consistency appropriate for producing a mash for fermentation. As used herein, the term "stock" includes any of the aforementioned types of plants and grains in crushed or coarsely ground forms. The methods described herein may be used to determine α-amylase activity levels in both flours and stock.

Processes for making beer are well known in the art. *See, e.g.,* Wolfgang Kunze (2004) "Technology Brewing and Malting," Research and Teaching Institute of Brewing, Berlin (VLB), 3rd edition. Briefly, the process involves: (a) preparing a mash, (b) filtering the mash to prepare a wort, and (c) fermenting the wort to obtain a fermented beverage, such as beer. Typically, milled or crushed malt, malt and adjunct, or adjunct is mixed with water and held for a period of time under controlled temperatures to permit the enzymes present in the malt and/or adjunct to convert the starch present in the malt into fermentable sugars. The mash is then transferred to a mash filter where the liquid is separated from the grain residue. This sweet liquid is called "wort," and the left over grain residue is called "spent grain." The mash is typically subjected to an extraction, which involves adding water to the mash in order to recover the residual soluble extract from the spent grain. The wort is then boiled vigorously to sterilize the wort and help develop the color, flavor and odor. Hops are added at some point during the boiling. The wort is cooled and transferred to a fermenter.

The wort is then contacted in a fermenter with yeast. The fermenter may be chilled to stop fermentation. The yeast, which may flocculate, is removed. Finally, the beer is cooled and stored for a period of time, during which the beer clarifies and its flavor develops, and any material that might impair the appearance, flavor and shelf life of the beer settles out. The beer usually contains from about 2% to about 10% v/v alcohol, although beer with a higher alcohol content, *e.g.,* 18% v/v, may be obtained. Prior to packaging, the beer is carbonated and, optionally, filtered and pasteurized.

The brewing composition comprising PcuAmy1, or a variant thereof, often but not necessarily in combination with one or more exogenous enzymes, such as glucoamylase(s), pullulanase(s) and/or isoamylase(s) and any combination thereof, may be added to the mash of step (a) above, such as during the preparation of the mash. Alternatively, or in addition, the brewing composition may be added to the mash of step (b) above, *i.e.,* during the filtration of the mash. Alternatively, or in addition, the brewing composition may be added to the wort of step (c) above, such as during the fermenting of the wort.

One aspect of the invention relates to the use of PcuAmy1, or variant thereof, in the production of a fermented beverage, such as a beer.

Another aspect concerns a method of providing a fermented beverage comprising the step of contacting a mash and/or a wort with PcuAmy1, or a variant thereof.

A further aspect relates to a method of providing a fermented beverage comprising the steps of: (a) preparing a mash, (b) filtering the mash to obtain a wort, and (c) fermenting the wort to obtain a fermented beverage, such as a beer, wherein PcuAmy1, or a variant, thereof is added to: (i) the mash of step (a) and/or (ii) the wort of step (b) and/or (iii) the wort of step (c).

According to yet another aspect, a fermented beverage, such as a beer, is produced or provided by a method comprising the step(s) of (1) contacting a mash and/or a wort with PcuAmy1, or a variant thereof; and/or (2) (a) preparing a mash, (b) filtering the mash to obtain a wort, and (c) fermenting the wort to obtain a fermented beverage, such as a beer, wherein PcuAmy1, or a variant thereof, is added to: (i) the mash of step (a) and/or (ii) the wort of step (b) and/or (iii) the wort of step (c).

Particular embodiments pertains to any of the above use, method or fermented beverage, wherein said fermented beverage is a beer, such as full malted beer, beer brewed under the "Reinheitsgebot," ale, IPA, lager, bitter, Happoshu (second beer), third beer, dry beer, near beer, light beer, low alcohol beer, low calorie beer, porter, bock beer, stout, malt liquor, non-alcoholic beer, non-alcoholic malt liquor and the like, but also alternative cereal and malt beverages such as fruit flavored malt beverages, *e.g*., citrus flavored, such as lemon-, orange-, lime-, or berry-flavored malt beverages, liquor flavored malt beverages, *e.g*., vodka-, rum-, or tequila-flavored malt liquor, or coffee flavored malt beverages, such as caffeine-flavored malt liquor, and the like.

### 4.11. Reduction of Iodine-Positive Starch

A PcuAmyl amylase variant may reduce the iodine-positive starch (IPS), when used in a method of liquefaction and/or saccharification. One source of IPS is from amylose that escapes hydrolysis and/or from retrograded starch polymer. Starch retrogradation occurs spontaneously in a starch paste, or gel on ageing, because of the tendency of starch molecules to bind to one another followed by an increase in crystallinity. Solutions of low concentration become increasingly cloudy due to the progressive association of starch molecules into larger articles. Spontaneous precipitation takes place and the precipitated starch appears to be reverting to its original condition of cold-water insolubility. Pastes of higher concentration on cooling set to a gel, which on ageing becomes steadily firmer due to the increasing association of the starch molecules. This arises because of the strong tendency for hydrogen bond formation between hydroxy groups on adjacent starch molecules. *See* J.A. Radley, ed., STARCH AND ITS DERIVATIVES 194-201 (Chapman and Hall, London (1968)).

The presence of IPS in saccharide liquor negatively affects final product quality and represents a major issue with downstream processing. IPS plugs or slows filtration system, and fouls the carbon columns used for purification. When IPS reaches sufficiently high levels, it may leak through the carbon columns and decrease production efficiency. Additionally, it may results in hazy final product upon storage, which is unacceptable for final product quality. The amount of IPS can be reduced by isolating the saccharification tank and blending the contents back. IPS nevertheless will accumulate in carbon columns and filter systems, among other things. The use of variant amylases is expected to improve overall process performance by reducing the amount of IPS.

### EXAMPLES

### Example 1

### Assays

Various assays used herein are set forth, below, for ease of reading. Any deviations from the protocols in later Examples are indicated in the relevant sections. In these experiments, a spectrophotometer was used to measure the absorbance of the products formed after the completion of the reactions. All assays were performed with culture supernatants treated with chelex beads.

### A. Chelex bead treatment of culture supernatants

96-well microtiter plates (MTPs) containing growing cultures were removed from incubators and Enzyscreen lids were replaced with disposable plastic sealers (Nunc Cat. No. 236366; Rochester, NY, USA). Cells were separated from culture supernatant via centrifugation (1118 RCF, 5 minutes). 150 µL supernatant was removed from each well and transferred to filter plates (Millipore Multiscreen HTS,Billerica, MA, USA) containing Chelex beads prepared as described below. Plates were shaken vigorously for 5 minutes and supernatant from 3 replicate growth plates were collected into a single deep-well microtiter plate (Axygen, PDW-11-C) using a vacuum manifold device. Plates containing supernatants were sealed and stored at 4°C.

Chelex-100 beads, 200-400 mesh (BioRad, Hercules, CA, USA) were washed twice with 2 bed-volumes of 1 M HCl followed by 5 bed-volumes of ultrapure water on a sintered glass filter apparatus. 2 bed-volumes of 1 M KOH were used to wash the beads followed by another 5 bed-volume wash with ultrapure water. Filtered beads were transferred to a beaker and suspended with enough ultrapure water to produce slurry capable of mixing. The pH of the slurry was adjusted to 8-8.5 using HCl. The liquid was removed and the beads were dried using a scintered glass filter. A slurry of beads (40% w/v) was prepared in ultra pure water and its pH was adjusted to 8.0 using KOH/HCl. A slurry having a constant consistency was maintained by vigorous mixing. A bubble paddle reservoir device (V&P Scientific, San Diego, CA, USA) was used to transfer 100 µL of slurry to all wells of filter plates. Liquid was removed using a vacuum manifold device.

### B. Protein Determination Assay

Protein determination assays were performed using chelex bead-treated culture supernatant from cultures grown in 96-well micro-titer plates (MTPs) over 3 days at 37°C with shaking at 300 rpm and 80% humidity. A fresh 96-well round-bottom MTP containing 25 µL supernatant per well was used for a High Performance Liquid Chromatography (HPLC) protein determination method. Supernatants were diluted four fold into 25 mM sodium acetate pH 5.5, and 10 µL of each diluted sample was analyzed. An Agilent 1200 (Hewlett Packard) HPLC equipped with a Poroshell 300SB-C8 (Agilent Technologies Santa Clara, CA, USA) column was used. Sample was bound to the column using 25 mM sodium acetate pH 5.5 and eluted over a gradient up to 70% acetonitrile. Absorbance was measured at 220 nm, integrated using ChemStation software (Agilent Technologies) and the protein concentration of samples was determined based on a standard curve of purified PcuAmy1-v1 protein.

### C. Ceralpha α-Amylase Activity Assay

The Ceralpha α-amylase assay was performed using the Ceralpha Kit (Megazyme, Wicklow, Ireland). The assay involves incubating culture supernatant with a substrate mixture under defined conditions, and the reaction is terminated (and color developed) by the addition of borate buffer (200 mM Boric acid/NaOH buffer, pH 10.2). The substrate is a mixture of the defined oligosaccharide "nonreducing-end blocked *p*-nitrophenyl maltoheptaoside" (BPNPG7) and excess levels of α-glucosidase (which has no action on the native substrate due to the presence of the "blocking group"). On hydrolysis of the oligosaccharide by *endo*acting α-amylase, the excess quantities of α-glucosidase present in the mixture give instantaneous and quantitative hydrolysis of the *p*-nitrophenyl maltosaccharide fragment to glucose and free *p-*nitrophenol. The absorbance at 405 nm was measured, which relates directly to the level of amylase in the sample analyzed.

The equipment used for this assay included a Biomek FX Robot (Beckman Coulter Brea, CA, USA); a SpectraMAX MTP Reader (type 340-Molecular Devices, Sunnyvale, CA, USA) and iEMS incubator/shaker (Thermo Scientific, Rockford, IL, USA). The reagent and solutions used were:
1) *p*-nitrophenyl maltoheptaoside (BPNPG7) substrate (Megazyme Ceralpha HR kit);
2) 50 mM Malate buffer, 0.005% TWEEN® 80, pH 5.6 or 50 mM MOPS, 0.005% TWEEN® 80, pH 7 (dilution buffers); and
3) 200 mM Boric acid / NaOH buffer, pH 10.2 (STOP buffer).

A vial containing 54.5 mg BPNPG7 substrate was dissolved in 10 mL of MilliQ water and then diluted into 30 mL of dilution buffer to make up 40 mL of the working substrate (1.36 mg/mL). The amylase samples (fermentation supernatant) were diluted 40X with dilution buffer. The assay was performed by adding 5 µL of diluted amylase solution into the wells of a MTP followed by the addition of 55 µL of diluted BPNPG7 working substrate solution. The solutions were mixed and the MTP was sealed with a plate seal and placed in an incubator/shaker (iEMS- Thermo Scientific) for 4 minutes at 25°C. The reaction was terminated by adding 70 µL STOP buffer and the absorbance was read at wavelength 400 nm in an MTP-Reader. A non-enzyme control was used to correct for background absorbance values.

### D. Thermostability Assay

The thermostability of PcuAmy1-v1 and variants was measured by determining the loss of amylase activity after a defined heat stress, using the Ceralpha α-amylase assay. The equipment used for this assay included a Biomek FX Robot (Beckman Coulter); a SpectraMAX MTP Reader (type 340-Molecular Devices), a Tetrad2DNA Engine PCR machine (Biorad), and iEMS incubator/shaker (Thermo Scientific). The reagent solutions used were (* not in all assays):
1) Heat stress buffers
   a) 50 mM KOAc pH 5.0 (0.25 mM CaCl₂, 10 mM NaCl)
   b) 50 mM KOAc pH 5.7 (0.125 mM CaCl₂, 2.2 mM NaCl)
2) p-nitrophenyl maltoheptaoside (BPNPG7) substrate (Megazyme Ceralpha HR kit):
3) 50 mM Malate buffer, 0.005% TWEEN® 80, pH 5.6 (dilution buffer); and
4) 200 mM Boric acid / NaOH, pH 10.2 (STOP buffer).
5) Amylase culture supernatant: 1:10 master dilution enzyme plates were diluted 1:10 in each of the four heat stress buffers in a PCR plate

5 µL of the diluted enzyme samples were added to a 96-well PCR plate containing 55 µL of diluted BPNPG7 working substrate solution and the initial amylase activity of the samples was determined using the Ceralpha α-amylase assay as described in Section C. The original diluted samples were subjected to heat stress for 3-6 minutes in a PCR thermocycler as follows: Buffers (a) 61°C and (b) 71°C, The heat stressed samples were cooled immediately to room temperature and 5 µL aliquots were assayed for amylase acitivity using the Ceralpha α-amylase assay as described in Section C. For each variant, the ratio of the initial and residual amylase activities was used to calculate the thermostability as follows: Thermostability = [t_{residual} value] / [tᵢₙᵢₜᵢₐₗ value], so the heat stability activity ratio was calculated based on enzyme activity after heat incubation divided by enzyme activity before heat incubation. For each sample (variants) the performance index (PI) is calculated. The performance index for thermostability stability is determined by comparing the thermostability of the variant enzyme with that of the similarly treated PcuAmy1-v1 enzyme (SEQ ID NO: 4).

### E. Starch Hydrolysis Assays (Corn Flour, Corn Starch, and Peak Viscosity Application assays)

Starch hydrolysis of corn flour and corn starch were used to measure specific activity of PcuAmy1-v1 and variants. Activity was measured as reducing ends generated by the enzymatic breakdown of corn flour or corn starch. The reducing ends generated during the incubation with either substrate were quantified using a PAHBAH (p-hydroxybenzoic acid hydrazide) assay. The equipment used for the assay included a Biomek FX Robot (Beckman Coulter); a SpectraMAX MTP Reader (type 340-Molecular Devices), a Tetrad2DNA Engine PCR machine (Biorad), and iEMS incubator/shaker (Thermo Scientific), and a Bubble Paddle Reservoir.

Azure Farms Organic Corn Flour (Norco, CA) was ground to a fine powder using a consumer coffee grinder and then sifted to obtain a <250 micron (µm) fraction. The sifted corn flour was washed extensively with MilliQ water by repeated suspension and centrifugation. Cargill Farms Organic Corn Starch material was also washed extensively with MilliQ water by repeated suspension and centrifugation.

Both corn flour and corn starch washed fractions were suspended in MilliQ water containing 0.005% sodium azide as 20% (w/w) stock solutions. The stock solutions were further diluted with a 20X stock buffer solution to 10.9% w/v corn flour and corn starch solutions (final buffer concentration: 55 mM KOAc, pH 5).

55 µL of the diluted corn flour and corn starch substrates were added to PCR microtiter plates along with 5 µL of 1:10 diluted enzyme samples using a bubble paddle reservoir. The plates were sealed and placed at 86°C for 5 minutes followed by a ramp down to 45°C. The starch hydrolysis reaction was terminated by addition of 70 µL 0.1 N NaOH. The plates were sealed and centrifuged for 3 minutes at 1610 RCF. The starch hydrolysis reaction products from both reactions were analyzed by the PAHBAH assay as described below.

For the Peak Viscosity application assay, 55 µL of the diluted corn flour substrate were added to PCR microtiter plates along with 5 µL of 1:10 diluted enzyme samples using a bubble paddle reservoir. The plates were sealed and placed at 59°C for 5 minutes followed by a ramp down to 45°C. The starch hydrolysis reaction was terminated by addition of 70 µL 0.1 N NaOH. The plates were sealed and centrifuged for 3 minutes at 1610 RCF. The starch hydrolysis reaction products from the reaction were analyzed by the PAHBAH assay as described below.

*PAHBAH assay:* Aliquots of 80 µL of 0.5 N NaOH were added to all wells of an empty PCR plate (a "PAHBAH reaction plate"), followed by 20 µL of PAHBAH reagent (5% w/v p-hydroxybenzoic acid hydrazide (Sigma Cat. No. H9882, St. Luis, MO, USA), dissolved in 0.5 N HCl). The solutions were mixed by pipetting up and down. 20 µL of the starch hydrolysis reaction supernatants were added to each well of the PAHBAH reaction plate. The plates were sealed and placed in a thermocycler, programmed for 2 minutes at 95°C to develop color, and then cooled to 20°C. Samples of 80 µL of the developed PAHBAH reaction mixtures were transferred to a fresh plate, and absorbance was measured at 450 nm in a spectrophotometer.

### F. CS-28 Rice Starch Microswatch Assay

The principle of this amylase assay is the liberation of an orange-dye due to the hydrolysis of rice starch incorporated in a cotton microswatch. The absorbance at 488 nm of the wash liquid is measured and this relates to the level of amylase activity in the sample analyzed at the desired conditions (pH, temperature, and buffer).

The equipment used for this assay included a Biomek FX Robot (Beckman Coulter), a SpectraMAX MTP Reader (type 340-Molecular Devices) and iEMS incubator/shaker (Thermo Scientific). The reagent and solutions used were:
1) CS-28 Microswatches (rice starch, colored);
2) 10 mM HEPES, 2 mM CaCl₂, 0.005% TWEEN 80 buffer, pH 8.0, conductivity 1mS/cm;
3) 25 mM CAPS, 2 mM CaCl₂, 0.005% TWEEN 80 buffer, pH 10.0; conductivity 5mS/cm (adjusted with 5M NaCl); and
4) 10 mM NaCl, 0.1 mM CaCl₂, 0.005% TWEEN 80.

CS-28 microswatches of 5.5 mm circular diameter were provided by the Center for Testmaterials (CFT, Vlaardingen, The Netherlands). Two microswatches were placed in each well of a 96-well Corning 9017 flat bottomed polystyrene MTP. The culture supernatants were diluted hundred fold in 10 mM NaCl, 0.1 mM CaCl₂, 0.005% TWEEN®80 solution.

The incubator/shaker was set at the desired temperature, 25°C (ambient temperature) or 50°C. 171 µL or 150 µL of either HEPES or CAPS buffer, respectively, was added to each well of microswatch containing MTP and subsequently 9 µL or 30 µL of diluted enzyme solution was added to each well resulting in a total volume of 180 µL/well. The MTP was sealed with a plate seal and placed in the iEMS incubator/shaker and incubated for 15 minutes at 1150 rpm at 25°C for cleaning at pH 8, low conductivity (1 mS/cm), or 15 minutes at 1150 rpm at 50°C for cleaning at pH 10, high conductivity (5 mS/cm). Following incubation under the appropriate conditions, 100 µL of solution from each well was transferred to a new MTP, and the absorbance at 488 nm was measured using a MTP-spectrophotometer. Controls containing two microswatches and buffer but no enzyme were included for subtraction of background cleaning performance.

Each absorbance value was corrected by subtracting the blank (obtained after incubation of microswatches in the absence of enzyme), and the resulting absorbance provided a measure of the hydrolytic activity. A performance index (PI) was calculated for each sample.

For calculation of the wash performance indices (PI), the Langmuir equation was used to fit the data based on the PcuAmy1-v1 enzyme (SEQ ID NO: 4) control. Using the protein concentration of the variants, the expected performance based on the curve-fit was calculated. The observed performance was divided by the calculated performance. This value was then divided by the performance of the PcuAmy1-v1enzyme (SEQ ID NO: 4).

### G. Detergent Stability Assay

The stability of the reference amylase (PcuAmy1-v1enzyme (SEQ ID NO: 4)) and variants thereof was determined by measuring their activity after incubation under defined conditions, in the presence of a 10% detergent mixture (commercially purchased Persil Color Gel detergent, Henkel (Düsseldorf, Germany), purchased in 2011). The detergent was heat-inactivated before use, and the initial and residual amylase activities were determined using the Ceralpha α-amylase assay as described in section C, above.

The equipment used for this assay included a Biomek FX Robot (Beckman Coulter); a SpectraMAX MTP Reader (type 340-Molecular Devices), a Tetrad2DNA Engine PCR machine (Biorad), and iEMS incubator/shaker (Thermo Scientific). The reagent solutions used were:
1) p-nitrophenyl maltoheptaoside (BPNPG7) substrate (Megazyme Ceralpha HR kit):
2) Liquid detergent (Persil color gel, enzyme inactivated by heating for 2 hrs at 80°C);
3) 50 mM MOPS, 0.1 mM CaCl₂, 0.005% TWEEN®80 buffer, pH 7 (dilution buffer);
4) 10% detergent solution diluted in dilution buffer;
5) 200 mM Boric acid / NaOH buffer, pH 10 (STOP buffer)
6) Amylase culture supernatants

97 µL of a 10% detergent solution was added to a 96-well PCR plate and mixed with 3 µL of the culture supernatant. A sample from the PCR plate was diluted 3X in dilution buffer and a 5 µL aliquot of this dilution was used to determine initial amylase activity. The PCR plate was incubated in a Tetrad PCR block at 86.5°C for 5 minutes. After incubation, detergent-enzyme mix was diluted 3X in dilution buffer and residual activity was measured. Initial (tᵢₙᵢₜᵢₐₗ) and residual (t_{residual}) amylase activity was determined by the Ceralpha α-amylase assay as described above in Section C using a 5 µL sample.

For each variant, the ratio of the residual and initial amylase activities was used to calculate the detergent stability as follows: Detergent stability = [t_{residual} value] / [tᵢₙᵢₜᵢₐₗ value].

For each sample (variants) the performance index (PI) was calculated. The performance index for detergent stability is determined by comparing the detergent stability of the variant enzyme with that of the similarly treated PcuAmy1-v1 enzyme (SEQ ID NO: 4).

### H. Performance index

The performance index (PI) compares the performance or stability of the variant and the parent enzyme (PcuAmy1-v1) at the same protein concentration. In addition, the theoretical values can be calculated, using the parameters of the Langmuir equation of the standard enzyme. A performance index (PI) that is greater than 1 (PI>1) indicates improved performance by a variant as compared to the parent (e.g., PcuAmy1-v1, SEQ ID NO: XX), while a PI of 1 (PI=1) identifies a variant that performs the same as the parent, and a PI that is less than 1 (PI<1) identifies a variant that performs worse than the parent.

### Example 2

### Generation of B. subtilis Strains Expressing PcuAmy1-v1 and Variants Thereof

In this example, the construction of *Bacillus subtilis* strains expressing PcuAmy1-v1 and variants, thereof, are described. PcuAmy1-v1 is a variant of PcuAmy1 lacking Arginine 177 and Glycine 178. PcuAmy1 is a predicted amylase from *Paenibacillus curdlanolyticus* YK9 for which the nucleotide sequence was determined by whole genome shotgun sequencing, performed by the United States Department of Energy Joint Genome Institute; Accession number AEDD01000000.

A DNA fragment (SEQ ID NO: 1, herein referred to as "PcuAmy1-v1 DNA") encoding PcuAmy1-v1 (SEQ ID NO: 4) was amplified by PCR from plasmid p2JM706, in multiple steps so as to delete the codons for Arginine 177 and Glycine 178 and remove a *Hind*III restriction site present in the mature gene. This served as template DNA for the construction of *Bacillus subtilis* strains expressing PcuAmy1-v1 and variants, thereof.

The codon-modified nucleic acid sequence encoding the mature form of PcuAmy1-v1 fused to a sequence encoding the *Bacillus licheniformis* Epr signal peptide (bold) is shown, below, as SEQ ID NO:1:

The precursor form of the PcuAmy1-v1 polypeptide produced from the pHP024T02-PcuAmy1-v1vector is shown below as SEQ ID NO: 2 (the Epr signal peptide is in bold, residues Arg-177 and Gly-178 are underlined):

The mature form of PcuAmy1 amylase is shown, below, as SEQ ID NO: 3 (residues Arg-177 and Gly-178 are underlined):

The mature form of the variant PcuAmy1 amylase produced from the pHP024T02-PcuAmy1-v1vector (*i.e.,* PcuAmy1-v1), having deletions of residues Arg-177 and Gly-178, is shown, below as SEQ ID NO: 4:

The Epr signal peptide is shown separately, below, as SEQ ID NO: 5:
MKKLWKIAVSAAMFVGFFANSPRASA

To express PcuAmy1-v1, the PcuAmy1-v1 DNA fragment was cloned into the pHP024T02 vector, a modified version of the pHPLT vector, (Solingen et al. (2001) Extremophiles 5:333-341), and fused in-frame to the Epr signal peptide using the unique *Nhe*I and *Hind*III restriction sites, resulting in plasmid pHP024T02-PcuAmy1-v1. The pHPLT expression vector contains the *B. licheniformis* LAT promoter (Plat) and additional elements from pUB110 (McKenzie et al. (1986) Plasmid 15: 93-103) including a replicase gene (reppUB), a neomycin/kanamycin resistance gene (neo) and a bleomycin resistance marker (bleo). The pHP024T02 expression vector was derived from the pHPLT vector. Site-directed mutagenesis was used to change the codon corresponding to Serine 28 of the *B. licheniformis* Epr signal peptide from 5'-TCA-3' to 5'-AGC-3' to introduce the unique *Nhe*I restriction site, creating expression vector pHPLT02. The sequence for the *B. licheniformis* Epr signal peptide was generated synthetically by GeneArt® (Life Technologies, Carlsbad, CA, USA), and cloned into the pHPLT02 vector using the unique *Nde*I and *Nhe*I restriction sites. A map of the pHP024T02 vector containing the PcuAmy1-v1 gene (pHP024T02-PcuAmy1-v1) is shown in Figure 1.

A suitable *B. subtilis* strain was transformed with pHP024T02-PcuAmy1-v1 plasmid DNA using a method known in the art (WO 02/14490). The *B. subtilis* transformants were selected on agar plates containing heart infusion agar (Difco, Catalog No. 244400) and 10 mg/L neomycin sulfate (Sigma, Catalog No. N-1876; contains 732 µg neomycin per mg). Selective growth of *B. subtilis* transformants harboring the pHP024T02- PcuAmy1-v1 plasmid was performed in shake flasks containing MBD medium (a MOPS based defined medium), 5 mM CaCl₂ and 10 mg/L neomycin. Growth resulted in the production of secreted PcuAmy1-v1 amylase with starch hydrolyzing activity.

### Example 3

### Generation of PcuAmy1-v1 Site Evaluation Libraries

The pHP024T02-PcuAmy1-v1 plasmid DNA served as template to produce site evaluation libraries (SELs; Gene Oracle, Mountain View, CA, USA) at pre-selected sites in the mature PcuAmy1-v1 (SEQ ID NO: 4), as shown in Table 3.1. The corresponding codons for each site were substituted with codons for at least 11 (out of a possible 19) different amino acid residues. The codon-mutagenized pHP024T02-PcuAmy1-v1 mixtures were used to transform competent *B. subtilis* cells as known in the art (WO 2002/014490) to generate the PcuAmy1-v1 SELs. Transformation mixtures were plated on Heart Infusion agar (HI-agar) plates containing 10 mg/L neomycin sulfate. For each library, single bacterial colonies were picked and grown in tryptone and soy-based broth (TSB) liquid medium with 10 mg/ml neomycin selection for subsequent DNA isolation and gene sequence analysis. To generate PcuAmy1-v1 and variant enzyme samples for biochemical characterization, the variantswere grown in 96-well MTPs at 37°C for 68 hours in MBD medium. A total of 7148 out of the 9082 possible variants were obtained for the 478 positions mutagenized. The positions mutagenized are shown in Table 3.1, which uses SEQ ID NO: 3 for numbering.

### Example 4

### Identification of Combinable and Productive Mutations

Performance index (PI) values were determined for all the PcuAmy1-v1 amylase SEL variants tested using the assays described in Example 1 (*i.e.,* starch hydrolysis assays, thermostability assays (at pH 5.0 and pH 5.7), CS-28 microswatch cleaning assays (at pH 8 and pH 10), detergent stability assay, Ceralpha activity assays (at pH 5.6 and 7), and protein determination assay).

Productive positions are described as those positions within a molecule that are most useful for making combinatorial variants exhibiting an improved characteristic, where each productive position allows for at least one combinable mutation. Combinable mutations are mutations at any amino acid position that can be used to make combinatorial variants. Combinable mutations improve at least one desired property of the molecule, while not significantly decreasing expression, activity, or stability. Combinable mutations were grouped as follows:
**Group A:** A mutation that produced a variant wherein the minimum performance indices (PI) relative to a defined parental protein for: (i) protein expression or activity in Ceralpha assay (at pH 5.6 and 7), and (ii) detergent stability or thermostability at pH 5.0 or pH 5.7 was greater than or equal to 0.9, and in addition had a PI for any of the other tests that was greater than or equal to 1.0.
**Group B:** A mutation that produced a variant wherein the minimum performance indices (PI) relative to a defined parental protein for: (i) protein expression or activity in Ceralpha assay (at pH 5.6 and 7), and (ii) detergent stability or thermostability at pH 5.0 or pH 5.7 was greater than or equal to 0.8, and in addition have a PI for any of the other tests that was greater than or equal to 1.2.
**Group C:** A mutation that produced a variant wherein the minimum performance indices (PI) relative to a defined parental protein for: (i) protein expression or activity in Ceralpha assay (at pH 5.6 and 7), and (ii) detergent stability or thermostability at pH 5.0 or pH 5.7 was greater than or equal to 0.5, and in addition have a PI for any of the other tests that was greater than or equal tol.5.

The properties of combinable mutations are summarized in Table 4.1.

**Table 4.1. Properties for each group of combinable mutations**

| Performance Index (PI) | | | |
|---|---|---|---|
| Group | Protein expression or Ceralpha assays | Detergent stability, or thermostability | Minimum PI in one or more tests including: cleaning or starch hydrolysis assays |
| A | ≥ 0.9 | ≥ 0.9 | X ≥ 1.0 |
| B | ≥ 0.8 | ≥ 0.8 | X ≥ 1.2 |
| C | ≥ 0.5 | ≥ 0.5 | X ≥ 1.5 |

Preferred combinable mutations are at "productive positions," as described, below. In the case of the present amylases, "activity" refers to α-amylase activity, which can be measured as described, herein.

Productive positions are amino acid positions that are tolerant to substitution with different amino acid residues, wherein the resulting variants meet a set of performance criteria for combinability, as set forth above. Productive positions can be assigned a Productivity Score as follows: Positions where less than 15% of the substitutions at a given position fall within groups A, B, or C are given a Productivity Score of "1". Positions where less than 45%, but greater than, or equal to 15% of the substitutions at a given position fall within groups A, B, or C are given a Productivity Score of "2". Positions where less than 75%, but greater than, or equal to 30% of the substitutions at a given position fall within groups A, B, or C are given a Productivity Score of "3". Positions where 50% or more of the substitutions at a given position fall within groups A, B, or C are given a Productivity Score of "4".

Suitability score refers to the ability of one or more combinable mutations to be used to make combinatorial variants, based on the performance criteria for combinability, (*i.e.,* A, B, and C, as set forth, above) in which each of the mutations fall. A higher suitability score indicates a mutation or mutations that are more suitable for use in making combinatorial variants. Suitability scores are described in Table 4.2.

**Table 4.2. Definitions of suitability scores**

| **Substitutions Occur in Group(s)** | **Suitability Score** |
|---|---|
| A, B and C | +++++ |
| A and B | ++++ |
| A or (B and C) | +++ |
| B | ++ |
| C | + |

Suitability scores of individual substitutions in PcuAmy1-v1 are shown in Table 4.3. For each PcuAmy1-v1 protein position, variants are listed according to the suitability score they received (+, ++, +++, ++++, or +++++). Position numbering is based on the mature PcuAmy1 polypeptide (SEQ ID NO: 3).

| **Table 4.3: Suitability scores of individual substitutions in PcuAmy1-v1** | | | | | |
|---|---|---|---|---|---|
| POS | VARIANTS SUITABILITY SCORE | | | | |
| | (+) | (++) | (+++) WT AA 1^{ST}* | (++++) | (+++ ++) |
| 1 | | W | G | DFHINSV | EKMR |
| 2 | HNV | M | DY | E | A |
| 3 | CR | S | N | | |
| 4 | | | GMY | DFINQSW | EHLPV |
| 5 | P | IS | T | | |
| 6 | | | ICF | | |
| 9 | DNQ | V | YM | FHTW | AGS |
| 10 | | LV | FN | M | |
| 14 | HQ | | LS | ACDEMN | RT |
| 15 | | | PDELMNRSVW | AH | |
| 16 | | AQR | N | | |
| 17 | | V | DNQ | EGSY | KR |
| 19 | | | ADFHIMNTVY | K | |
| 20 | A | G | H | T | |
| 22 | | C | NS | ADEFGIKM QW | HRT |
| 23 | C | KVW | RDEIN | AM | |
| 24 | | AT | L | IMV | |
| 25 | C | | NAFQTY | E | |
| 26 | | A | NDEGHKLRV | Y | |
| 27 | C | FW | DIN | GMQSTV | EK |
| 28 | | | AGT | RS | |
| 29 | | | Q | ADFGIMNPRVWY | E |
| 30 | | | N | ADFGHLMPRSTVWY | Q |
| 31 | WY | | L | F | |
| 32 | | | KDGIMY | AS | |
| 33 | | W | N | DEHKQSTV | A |
| 34 | | | VADFGHLQRSTWY | | |
| 35 | S | | G | | |
| 36 | | | IFMV | | |
| 37 | C | V | TADEGS | K | Q |
| 38 | CMW | | AEHRST | DN | G |
| 39 | | T | V | LM | |
| 41 | G | | ILV | AS | |
| 44 | N | T | A | S | |
| 45 | | N | YFH | | |
| 47 | | S | G | A | |
| 48 | | DEIV | G | HT | K |
| 49 | LY | V | SEM | DGNT | |
| 50 | | HMV | SDENQ | GK | |
| 51 | | S | AEQV | GKLRW | |
| 53 | | T | VEN | | |
| 57 | C | L | VS | I | |
| 60 | HN | | TAE | GV | |
| 63 | | | LI | | |
| 65 | A | | EN | | |
| 67 | K | | NADGMQSVWY | EL | |
| 68 | GT | | QAS | | |
| 69 | EGSW | | K | H | R |
| 70 | W | Y | GDEFHK | | |
| 71 | LMR | D | TAS | | |
| 72 | | R | VTY | I | |
| 73 | W | | RHM | GPS | AE |
| 74 | I | V | T | | |
| 76 | H | | Y | W | |
| 78 | C | L | TDFHIQSV | AGKMNR | E |
| 79 | | MR | KAS | | |
| 80 | | | SCW | ADEFKMPRTV | G |
| 81 | ACDFGHMRTY | V | EQ | NS | |
| 82 | | I | L | V | |
| 83 | | HT | IDQS | AGMVWY | E |
| 84 | | AIKM | SEF | GPVWY | |
| 85 | L | | ACGMV | | |
| 86 | R | | VKST | AEILMQ | |
| 87 | | | NADFGLQRVY | ST | |
| 88 | | ST | NAM | CEIRVY | |
| 89 | | | LI | V | |
| 90 | | | HKQR | | |
| 91 | | CH | AEFLMSTVWY | IK | |
| 92 | S | | KADIMNQRTY | EG | |
| 93 | | | GT | ADEQRS | |
| 94 | | | IMV | | |
| 95 | | Q | ADGLMNT | FSWY | |
| 96 | | | VLT | AI | |
| 97 | N | M | Y | AFHILV | |
| 98 | E | | GA | | |
| 100 | | | VAIS | | |
| 101 | | | VEI | | |
| 102 | | | LAST | V | Q |
| 105 | AGST | M | RKL | | |
| 107 | IKMP | | NEW | AS | G |
| 110 | | CV | AKW | DEGHLNQRSTY | |
| 111 | C | L | TIK | ADFGMNSV | E |
| 112 | F | A | E | DMN | |
| 113 | | | LCIVY | DEFGNPQS | AW |
| 114 | | I | V | | |
| 115 | | FIMTW | DEHKR | AGNS | |
| 116 | | CG | AS | | |
| 117 | | | VAST | KR | |
| 120 | AR | S | D | N | |
| 121 | | AEQR | P | FST | |
| 122 | | | NHVW | ADEFGQRS | |
| 125 | | R | ND | CWY | |
| 126 | | CW | V | AELNQRST | |
| 127 | | R | EH | AFNPQSTV | K |
| 128 | | ACFSW | TM | R | IV |
| 129 | C | EQRV | TK | AG | |
| 130 | P | | SW | ADFGHIKLRTV | NQ |
| 131 | C | | T | DFGHIMNQRVW | AEPY |
| 132 | DGV | | Y | | EHQST |
| 133 | H | | QVY | DGIMNPRS | FW |
| 134 | | | I | V | |
| 135 | | | Q | ADEGHILMNTWY | RV |
| 136 | | I | AGT | | |
| 137 | | | W | Y | |
| 138 | | | TS | | |
| 139 | | | QADGKV | FLMNRWY | H |
| 140 | | I | Y | F | H |
| 141 | | AFRY | DCGIKQSV | EMW | |
| 142 | | | FY | | |
| 143 | | | PDGHIV | AFNQSW | E |
| 144 | CILY | DHKMPTV | G | AES | NQ |
| 146 | | P | GDEFV | AHKNSWY | MQ |
| 147 | | | NDFGHKQST | APR | |
| 148 | | DE | TAHMQVWY | KNR | |
| 149 | EGMV | | YIL | | F |
| 150 | | | SG | | |
| 151 | | QY | SPV | AEGHKNRT | DF |
| 152 | | Y | F | H | W |
| 153 | | FMS | KHQR | | |
| 155 | | T | RACEGNV | F | KY |
| 156 | HP | | W | | |
| 157 | | MR | Y | DFGIPQSVW | |
| 162 | ADEGHLQ | | V | C | |
| 164 | ACLMNQ | + | WT | | |
| 166 | CFKRS | AEN | QDP | GH | T |
| 167 | C | | SVWY | DEI | AFGHLQRT |
| 168 | | A | RK | LST | |
| 169 | | | GEQSTW | AFHR | DK |
| 170 | | | LK | AEGHQVWY | DRS |
| 171 | | DE | N | AS | |
| 172 | V | AW | RH | N | |
| 173 | | | IL | | |
| 174 | CE | H | Y | FW | M |
| 175 | | | K | | E |
| 176 | | A | LI | GNQ | H |
| 179 | | C | D | AEFKLMNRSTVWY | H |
| 180 | | AMNQY | DR | ET | |
| 181 | | | K | Q | H |
| 182 | | | DV | N | AEFHIKMRSTWY |
| 189 | | | S | AEGN | D |
| 190 | | | E | Q | |
| 191 | CMR | | Y | | HW |
| 192 | | | GAHMSVY | Q | E |
| 200 | | | AT | E | |
| 202 | | FN | L | | |
| 203 | A | | D | | C |
| 204 | | | F | CM | |
| 205 | C | | NIS | W | ADEFGMVY |
| 206 | L | | H | DEWY | ACMQ |
| 207 | | D | PAHLNT | EGRS | |
| 208 | | | DFGNQVY | ILRS | E |
| 210 | | | VIKM | QR | E |
| 211 | | I | NAFKSWY | HLMQRTV | |
| 212 | | S | E | AT | |
| 213 | V | | TAENS | Y | |
| 214 | | Y | KDFGILS | AMNQ | E |
| 215 | | | TADEFGILMNQSY | V | |
| 217 | | | GAS | | |
| 218 | | F | KAW | | |
| 220 | | | FMV | L | |
| 221 | | | VAL | | |
| 222 | | | NDGHQ | T | |
| 223 | | | TGRSVY | E | |
| 224 | | T | V | GIS | L |
| 225 | | CFIV | NDESW | AGMPY | |
| 226 | Y | V | LF | | |
| 227 | | | D | N | |
| 229 | | | VA | LW | I |
| 231 | CQSV | A | LT | M | |
| 234 | EN | | VGLS | AIT | |
| 237 | L | | I | | V |
| 238 | F | | KL | | CQW |
| 239 | Y | | F | | |
| 241 | G | | FCDEY | HN | IKMRSV |
| 242 | AY | | MCQ | EL | |
| 243 | V | | R | ACFGHQSWY | DEL |
| 244 | ACEGHNS | | D | | |
| 246 | | IMY | VT | A | |
| 247 | | G | N | ADEFMSV | QRW |
| 248 | | H | NCGIKVW | AEFMQRS | D |
| 249 | | | V | GN | EIM |
| 250 | | | R | | ADEFGHIMNTW |
| 251 | | | SADEFGLMNQTV | KW | |
| 252 | N | | TAEFGHKMQV | L | |
| 253 | E | | TGIKLMNPRSVY | | |
| 254 | | | GAEHIKMPQVY | R | |
| 255 | | | KADGIMNRSTVWY | | |
| 256 | | LY | NDGS | AM | |
| 257 | | | LAIKW | M | |
| 258 | G | K | FMPSW | AHTVY | |
| 259 | | N | AGHST | | |
| 260 | I | L | V | | |
| 261 | | A | G | S | |
| 264 | | | WLM | F | |
| 265 | W | EK | HDIRV | | AGM |
| 266 | G | D | YK | NPRSTW | ELMQV |
| 267 | GHK | S | D | | |
| 268 | R | W | VL | DEFGIY | AKMNPQ |
| 269 | | | NI | GHKY | ADEQST |
| 270 | A | ST | K | V | |
| 271 | | I | L | M | |
| 272 | | | NDEHIKQ | V | |
| 273 | | | SDEY | | |
| 274 | | F | Y | | |
| 275 | FWY | | ILV | M | |
| 276 | | K | TIQWY | ACEGL | D |
| 278 | | I | TV | | |
| 279 | DQV | HT | N | AGS | EW |
| 280 | | | GANQRSY | DKM | E |
| 281 | | | TDHQS | EKNR | A |
| 282 | | NY | MQ | EHILS | |
| 283 | CW | G | SAERY | DHKMT | |
| 285 | MW | Y | F | | |
| 287 | | T | VAIS | | |
| 289 | A | | L | | |
| 291 | | G | FQ | DIN | KMVY |
| 292 | | E | RK | | |
| 293 | AEL | | F | | |
| 294 | D | N | Y | | |
| 295 | | WY | D | AEHKNQ | |
| 297 | | | SAG | | |
| 298 | | F | NHIT | Q | E |
| 299 | | | GAKMQS | E | |
| 300 | | FV | GHWY | AEKQS | N |
| 302 | | W | GHMY | DENRSV | AK |
| 303 | | | YF | | |
| 306 | | | RS | | |
| 307 | | | NEG | FHKMQRST | ADLVW |
| 309 | K | | L | | |
| 310 | | | NDGHMV | KR | E |
| 311 | | Y | NADEGHQR | | |
| 313 | | | LM | | |
| 314 | | | MATV | S | |
| 315 | | | SDEGHILRTVY | MN | |
| 316 | | | SGLT | ADHINQVW | EY |
| 317 | | FWY | NAK | DEIV | Q |
| 318 | W | | PDHS | | A |
| 319 | | F | MW | DIKQRSVY | AEGT |
| 320 | | SY | KAEFL | | |
| 322 | Y | | V | | + |
| 331 | | | Q | E | A |
| 333 | | FQY | TN | S | ADEG |
| 337 | | | QD | E | |
| 338 | | T | S | | |
| 339 | | A | TE | | |
| 340 | | | V | + | |
| 341 | | HMNS | Q | EK | |
| 342 | | | SHPQR | K | |
| 345 | | V | K | | |
| 347 | | | LI | AMQ | |
| 348 | | | AG | | |
| 356 | | T | EGNW | DS | |
| 357 | | | Q | E | |
| 359 | | LS | Y | AFHMR TV W | DN |
| 361 | K | Q | C | AITV | E |
| 362 | A | | VM | L | + |
| 364 | | | YF | L | |
| 367 | M | | Y | | |
| 368 | | | YEFIKLNQRSTVW | | AD |
| 369 | | | GA | | |
| 370 | | | TS | | |
| 371 | | | SAEGY | DT | |
| 373 | | | GADEHQRS | | |
| 374 | | DM | KAEG | N | |
| 376 | V | | SEGHQ | AIRT | N |
| 377 | | | SALY | DEFGHPQRTV | N |
| 378 | | | YEGNT | SV | AD |
| 379 | | | KAGS | | |
| 380 | | | PDGWY | AEHKMRST | N |
| 381 | | | IAEFLT | Q | |
| 382 | | | M | I | |
| 383 | | | D | E | |
| 384 | CF | P | KAGMT | INSY | DV |
| 385 | | M | L | | |
| 386 | | T | LFV | M | |
| 387 | | I | NAGHQSTY | L | |
| 388 | | | ALTV | | |
| 390 | | | KHLQRY | | |
| 391 | | | VADFHLN | KM | |
| 392 | | | YF | | |

| (+) | (++) | (+++) WT AA 1^{ST}* | (++++) | (+++++ ) | |
|---|---|---|---|---|---|
| 396 | | | TIPRSVY | EFHNQ | DLM |
| 398 | | Y | R | HN | |
| 401 | | | F | M | |
| 402 | | | D | N | |
| 403 | | | HD | | |
| 404 | CLS | GV | PW | AEMNQ | |
| 405 | | | D | N | |
| 406 | T | M | ILV | | |
| 407 | | | VI | | |
| 412 | D | AGLT | E | MQ | |
| 414 | E | | D | | |
| 415 | | | ADEGMR | KQ | |
| 416 | | | AY | DEFGIKLMNPQTV | |
| 418 | | | ASWY | EGHMRTV | KLNQ |
| 419 | | V | GH | DEFKMQRSTWY | |
| 420 | A | | S | | |
| 425 | | | LM | | |
| 426 | | | I | V | |
| 427 | | | T | DN | A |
| 430 | CH | F | PM | DESVY | AGIK |
| 431 | DN | KR | GV | S | AEPQT |
| 432 | A | | G | | |
| 433 | | | SD | HQR | |
| 435 | | D | WAFKSY | EHNQR | LM |
| 440 | | | TILMNSV | ADEH | |
| 441 | | R | SDEGHQ | | |
| 442 | Q | R | K | | HN |
| 443 | | K | A | | |
| 444 | D | HK | GN | | |
| 446 | | D | V | AEHILRST | MN |
| 448 | | | THS | R | |
| 450 | MRY | | K | HQ | |
| 451 | | L | T | | |
| 452 | AM | R | GH | Q | N |
| 453 | | | NH | | |
| 454 | C | EK | RQW | AFNSVY | L |
| 455 | | D | SEPQT | AHR | |
| 456 | | | GEN | D | |
| 457 | | | TEGNY | KSV | |
| 459 | | V | T | KQR | |
| 461 | S | E | DQ | G | |
| 462 | C | | AV | GPT | DEKMR |
| 463 | | | NAGHRSTY | EQ | |
| 465 | | | WY | | |
| 467 | RV | | NEHKLMQ | | |
| 469 | | | WADEGHNPSVY | K | |
| 471 | | A | NEGR | DKQ | |
| 472 | F | | G | DHK | AENT |
| 473 | V | | GFY | R | ADEKQS |
| 474 | | G | S | | |
| 475 | | T | V | | |
| 477 | | | VL | | |
| 480 | MV | IW | KP | EGSY | |

| | | | | | |
|---|---|---|---|---|---|
| * The wild-type amino acid residue is listed first | | | | | |

The productive positions in PcuAmy1-v1 that fall within the previously described Productivity Scores of "1," "2," "3," and "4," and the substitutions within those positions that are combinable, are listed below. Each position identified in the list, and each substitution identified in parenthesis following the numerical position identifier, represents a mutation that has been determined, based on experimental data, to contribute to the performance of an amylase variant, particularly a variant comprising more than one of the described mutations. The position numbering is based on mature PcuAmy1 polypeptide (SEQ ID NO: 3).

The productive positions in PcuAmy1-v1 that fall within the previously described Productivity Scores of "1," "2," "3," and "4," and the substitutions within those positions that are combinable, are listed below. The position numbering is based on mature PcuAmy1 polypeptide (SEQ ID NO: 3):
LIST A:
1 (G,D,E,F,H,I,K,M,N,R,S,V,W); 2 (D,A,E,H,M,N,V,Y); 3 (N,C,R,S); 4 (G,D,E,F,H,I,L,M,N,P,Q,S,V,W,Y); 5 (T,I,P,S); 6 (I,C,F); 9 (Y,A,D,F,G,H,M,N,Q,S,T,V,W); 10 (F,L,M,N,V); 14 (L,A,C,D,E,H,M,N,Q,R,S,T); 15 (P,A,D,E,H,L,M,N,R,S,V,W); 16 (N,A,Q,R); 17 (D,E,G,K,N,Q,R,S,V,Y); 19 (A,D,F,H,I,K,M,N,T,V,Y); 20 (H,A,G,T\); 22 (N,A,C,D,E,F,G,H,I,K,M,Q,R,S,T,W); 23 (R,A,C,D,E,I,K,M,N,V,W); 24 (L,A,I,M,T,V); 25 (N,A,C,E,F,Q,T,Y); 26 (N,A,D,E,G,H,K,L,R,V,Y); 27 (D,C,E,F,G,I,K,M,N,Q,S,T,V,W); 28 (A,G,R,S,T); 29 (Q,A,D,E,F,G,I,M,N,P,R,V,W,Y); 30 (N,A,D,F,G,H,L,M,P,Q,R,S,T,V,W,Y); 31 (L,F,W,Y); 32 (K,A,D,G,I,M,S,Y); 33 (N,A,D,E,H,K,Q,S,T,V,W); 34 (V,A,D,F,G,H,L,Q,R,S,T,W,Y); 35 (G,S); 36 (I,F,M,V); 37 (T,A,C,D,E,G,K,Q,S,V); 38 (A,C,D,E,G,H,M,N,R,S,T,W); 39 (V,L,M,T); 41 (I,A,G,L,S,V); 44 (A,N,S,T\); 45 (Y,F,H,N); 47 (G,A,S); 48 (G,D,E,H,I,K,T,V); 49 (S,D,E,G,L,M,N,T,V,Y); 50 (S,D,E,G,H,K,M,N,Q,V); 51 (A,E,G,K,L,Q,R,S,V,W); 53 (V,E,N,T); 57 (V,C,I,L,S); 60 (T,A,E,G,H,N,V); 63 (L,I); 65 (E,A,N); 67 (N,A,D,E,G,K,L,M,Q,S,V,W,Y); 68 (Q,A,G,S,T); 69 (K,E,G,H,R,S,W); 70 (G,D,E,F,H,K,W,Y); 71 (T,A,D,L,M,R,S); 72 (V,I,R,T,Y); 73 (R,A,E,G,H,M,P,S,W); 74 (T,I,V); 76 (Y,H,W); 78 (T,A,C,D,E,F,G,H,I,K,L,M,N,Q,R,S,V); 79 (K,A,M,R,S); 80 (S,A,C,D,E,F,G,K,M,P,R,T,V,W); 81 (E,A,C,D,F,G,H,M,N,Q,R,S,T,V,Y); 82 (L,I,V); 83 (I,A,D,E,G,H,M,Q,S,T,V,W,Y); 84 (S,A,E,F,G,I,K,M,P,V,W,Y); 85 (A,C,G,L,M,V); 86 (V,A,E,I,K,L,M,Q,R,S,T); 87 (N,A,D,F,G,L,Q,R,S,T,V,Y); 88 (N,A,C,E,I,M,R,S,T,V,Y); 89 (L,I,V); 90 (H,K,Q,R); 91 (A,C,E,F,H,I,K,L,M,S,T,V,W,Y); 92 (K,A,D,E,G,I,M,N,Q,R,S,T,Y); 93 (G,A,D,E,Q,R,S,T); 94 (I,M,V); 95 (A,D,F,G,L,M,N,Q,S,T,W,Y); 96 (V,A,I,L,T); 97 (Y,A,F,H,I,L,M,N,V); 98 (G,A,E); 100 (V,A,I,S); 101 (V,E,I); 102 (L,A,Q,S,T,V); 105 (R,A,G,K,L,M,S,T); 107 (N,A,E,G,I,K,M,P,S,W); 110 (A,C,D,E,G,H,K,L,N,Q,R,S,T,V,W,Y); 111 (T,A,C,D,E,F,G,I,K,L,M,N,S,V); 112 (E,A,D,F,M,N); 113 (L,A,C,D,E,F,G,I,N,P,Q,S,V,W,Y); 114 (V,I); 115 (D,A,E,F,G,H,I,K,M,N,R,S,T,W); 116 (A,C,G,S); 117 (V,A,K,R,S,T); 120 (D,A,N,R,S); 121 (P,A,E,F,Q,R,S,T); 122 (N,A,D,E,F,G,H,Q,R,S,V,W); 125 (N,C,D,R,W,Y); 126 (V,A,C,E,L,N,Q,R,S,T,W); 127 (E,A,F,H,K,N,P,Q,R,S,T,V); 128 (T,A,C,F,I,M,R,S,V,W); 129 (T,A,C,E,G,K,Q,R,V); 130 (S,A,D,F,G,H,I,K,L,N,P,Q,R,T,V,W); 131 (T,A,C,D,E,F,G,H,I,M,N,P,Q,R,V,W,Y); 132 (Y,D,E,G,H,Q,S,T,V); 133 (Q,D,F,G,H,I,M,N,P,R,S,V,W,Y); 134 (I,V); 135 (Q,A,D,E,G,H,I,L,M,N,R,T,V,W,Y); 136 (A,G,I,T); 137 (W,Y); 138 (T,S); 139 (Q,A,D,F,G,H,K,L,M,N,R,V,W,Y); 140 (Y,F,H,I); 141 (D,A,C,E,F,G,I,K,M,Q,R,S,V,W,Y); 142 (F,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 147 (N,A,D,F,G,H,K,P,Q,R,S,T); 148 (T,A,D,E,H,K,M,N,Q,R,V,W,Y); 149 (Y,E,F,G,I,L,M,V); 150 (S,G); 151 (S,A,D,E,F,G,H,K,N,P,Q,R,T,V,Y); 152 (F,H,W,Y); 153 (K,F,H,M,Q,R,S); 155 (R,A,C,E,F,G,K,N,T,V,Y); 156 (W,H,P); 157 (Y,D,F,G,I,M,P,Q,R,S,V,W); 162 (V,A,C,D,E,G,H,L,Q); 164 (W,A,C,I,L,M,N,Q,T); 166 (Q,A,C,D,E,F,G,H,K,N,P,R,S,T); 167 (S,A,C,D,E,F,G,H,I,L,Q,R,T,V,W,Y); 168 (R,A,K,L,S,T); 169 (G,A,D,E,F,H,K,Q,R,S,T,W); 170 (L,A,D,E,G,H,K,Q,R,S,V,W,Y); 171 (N,A,D,E,S); 172 (R,A,H,N,V,W); 173 (I,L); 174 (Y,C,E,F,H,M,W); 175 (K,E); 176 (L,A,G,H,I,N,Q); 179 (D,A,C,E,F,H,K,L,M,N,R,S,T,V,W,Y); 180 (D,A,E,M,N,Q,R,T,Y); 181 (K,H,Q); 182 (D,A,E,F,H,I,K,M,N,R,S,T,V,W,Y); 189 (S,A,D,E,G,N); 190 (E,Q); 191 (Y,C,H,M,R,W); 192 (G,A,E,H,M,Q,S,V,Y); 200 (A,E,T); 202 (L,F,N); 203 (D,A,C); 204 (F,C,M); 205 (N,A,C,D,E,F,G,I,M,S,V,W,Y); 206 (H,A,C,D,E,L,M,Q,W,Y); 207 (P,A,D,E,G,H,L,N,R,S,T); 208 (D,E,F,G,I,L,N,Q,R,S,V,Y); 210 (V,E,I,K,M,Q,R); 211 (N,A,F,H,I,K,L,M,Q,R,S,T,V,W,Y); 212 (E,A,S,T); 213 (T,A,E,N,S,V,Y); 214 (K,A,D,E,F,G,I,L,M,N,Q,S,Y); 215 (T,A,D,E,F,G,I,L,M,N,Q,S,V,Y); 217 (G,A,S); 218 (K,A,F,W); 220 (F,L,M,V); 221 (V,A,L); 222 (N,D,G,H,Q,T); 223 (T,E,G,R,S,V,Y); 224 (V,G,I,L,S,T); 225 (N,A,C,D,E,F,G,I,M,P,S,V,W,Y); 226 (L,F,V,Y); 227 (D,N); 229 (V,A,I,L,W); 231 (L,A,C,M,Q,S,T,V); 234 (V,A,E,G,I,L,N,S,T); 237 (I,L,V); 238 (K,C,F,L,Q,W); 239 (F,Y); 241 (F,C,D,E,G,H,I,K,M,N,R,S,V,Y); 242 (M,A,C,E,L,Q,Y); 243 (R,A,C,D,E,F,G,H,L,Q,S,V,W,Y); 244 (D,A,C,E,G,H,N,S); 246 (V,A,I,M,T,Y); 247 (N,A,D,E,F,G,M,Q,R,S,V,W); 248 (N,A,C,D,E,F,G,H,I,K,M,Q,R,S,V,W); 249 (V,E,G,I,M,N); 250 (R,A,D,E,F,G,H,I,M,N,T,W); 251 (S,A,D,E,F,G,K,L,M,N,Q,T,V,W); 252 (T,A,E,F,G,H,K,L,M,N,Q,V); 253 (T,E,G,I,K,L,M,N,P,R,S,V,Y); 254 (G,A,E,H,I,K,M,P,Q,R,V,Y); 255 (K,A,D,G,I,M,N,R,S,T,V,W,Y); 256 (N,A,D,G,L,M,S,Y); 257 (L,A,I,K,M,W); 258 (F,A,G,H,K,M,P,S,T,V,W,Y); 259 (A,G,H,N,S,T); 260 (V,I,L); 261 (G,A,S); 264 (W,F,L,M); 265 (H,A,D,E,G,I,K,M,R,V,W); 266 (Y,D,E,G,K,L,M,N,P,Q,R,S,T,V,W); 267 (D,G,H,K,S); 268 (V,A,D,E,F,G,I,K,L,M,N,P,Q,R,W,Y); 269 (N,A,D,E,G,H,I,K,Q,S,T,Y); 270 (K,A,S,T,V); 271 (L,I,M); 272 (N,D,E,H,I,K,Q,V); 273 (S,D,E,Y); 274 (Y,F); 275 (I,F,L,M,V,W,Y); 276 (T,A,C,D,E,G,I,K,L,Q,W,Y); 278 (T,I,V); 279 (N,A,D,E,G,H,Q,S,T,V,W); 280 (G,A,D,E,K,M,N,Q,R,S,Y); 281 (T,A,D,E,H,K,N,Q,R,S); 282 (M,E,H,I,L,N,Q,S,Y); 283 (S,A,C,D,E,G,H,K,M,R,T,W,Y); 285 (F,M,W,Y); 287 (V,A,I,S,T); 289 (L,A); 291 (F,D,G,I,K,M,N,Q,V,Y); 292 (R,E,K); 293 (F,A,E,L); 294 (Y,D,N); 295 (D,A,E,H,K,N,Q,W,Y); 297 (S,A,G); 298 (N,E,F,H,I,Q,T); 299 (G,A,E,K,M,Q,S); 300 (G,A,E,F,H,K,N,Q,S,V,W,Y); 302 (G,A,D,E,H,K,M,N,R,S,V,W,Y); 303 (Y,F); 306 (R,S); 307 (N,A,D,E,F,G,H,K,L,M,Q,R,S,T,V,W); 309 (L,K); 310 (N,D,E,G,H,K,M,R,V); 311 (N,A,D,E,G,H,Q,R,Y); 313 (L,M); 314 (M,A,S,T,V); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 318 (P,A,D,H,S,W); 319 (M,A,D,E,F,G,I,K,Q,R,S,T,V,W,Y); 320 (K,A,E,F,L,S,Y); 322 (V,I,Y); 331 (Q,A,E); 333 (T,A,D,E,F,G,N,Q,S,Y); 337 (Q,D,E); 338 (S,T); 339 (T,A,E); 340 (V,I); 341 (Q,E,H,K,M,N,S); 342 (S,H,K,P,Q,R); 345 (K,V); 347 (L,A,I,M,Q); 348 (A,G); 356 (E,D,G,N,S,T,W); 357 (Q,E); 359 (Y,A,D,F,H,L,M,N,R,S,T,V,W); 361 (C,A,E,I,K,Q,T,V); 362 (V,A,I,L,M); 364 (Y,F,L); 367 (Y,M); 368 (Y,A,D,E,F,I,K,L,N,Q,R,S,T,V,W); 369 (G,A); 370 (T,S); 371 (S,A,D,E,G,T,Y); 373 (G,A,D,E,H,Q,R,S); 374 (K,A,D,E,G,M,N); 376 (S,A,E,G,H,I,N,Q,R,T,V); 377 (S,A,D,E,F,G,H,L,N,P,Q,R,T,V,Y); 378 (Y,A,D,E,G,N,S,T,V); 379 (K,A,G,S); 380 (P,A,D,E,G,H,K,M,N,R,S,T,W,Y); 381 (I,A,E,F,L,Q,T); 382 (M,I); 383 (D,E); 384 (K,A,C,D,F,G,I,M,N,P,S,T,V,Y); 385 (L,M); 386 (L,F,M,T,V); 387 (N,A,G,H,I,L,Q,S,T,Y); 388 (A,L,T,V); 390 (K,H,L,Q,R,Y); 391 (V,A,D,F,H,K,L,M,N); 392 (Y,F); 396 (T,D,E,F,H,I,L,M,N,P,Q,R,S,V,Y); 398 (R,H,N,Y); 401 (F,M); 402 (D,N); 403 (H,D); 404 (P,A,C,E,G,L,M,N,Q,S,V,W); 405 (D,N); 406 (I,L,M,T,V); 407 (V,I); 412 (E,A,D,G,L,M,Q,T); 414 (D,E); 415 (A,D,E,G,K,M,Q,R); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 420 (S,A); 425 (L,M); 426 (I,V); 427 (T,A,D,N); 430 (P,A,C,D,E,F,G,H,I,K,M,S,V,Y); 431 (G,A,D,E,K,N,P,Q,R,S,T,V); 432 (G,A); 433 (S,D,H,Q,R); 435 (W,A,D,E,F,H,K,L,M,N,Q,R,S,Y); 440 (T,A,D,E,H,I,L,M,N,S,V); 441 (S,D,E,G,H,Q,R); 442 (K,H,N,Q,R); 443 (A,K); 444 (G,D,H,K,N); 446 (V,A,D,E,H,I,L,M,N,R,S,T); 448 (T,H,R,S); 450 (K,H,M,Q,R,Y); 451 (T,L); 452 (G,A,H,M,N,Q,R); 453 (N,H); 454 (R,A,C,E,F,K,L,N,Q,S,V,W,Y); 455 (S,A,D,E,H,P,Q,R,T); 456 (G,D,E,N); 457 (T,E,G,K,N,S,V,Y); 459 (T,K,Q,R,V); 461 (D,E,G,Q,S); 462 (A,C,D,E,G,K,M,P,R,T,V); 463 (N,A,E,G,H,Q,R,S,T,Y); 465 (W,Y); 467 (N,E,H,K,L,M,Q,R,V); 469 (W,A,D,E,G,H,K,N,P,S,V,Y); 471 (N,A,D,E,G,K,Q,R); 472 (G,A,D,E,F,H,K,N,T); 473 (G,A,D,E,F,K,Q,R,S,V,Y); 474 (S,G); 475 (V,T); 477 (V,L); and 480 (K,E,G,I,M,P,S,V,W,Y).

The productive positions in PcuAmy1-v1 that fall within the previously described Productivity Scores of "2," "3," and "4," and the substitutions within those positions that are combinable, are listed below. The position numbering is based on the mature PcuAmy1 polypeptide (SEQ ID NO: 3):
LIST B:
1 (G,D,E,F,H,I,K,M,N,R,S,V,W); 2 (D,A,E,H,M,N,V,Y); 3 (N,C,R,S); 4 (G,D,E,F,H,I,L,M,N,P,Q,S,V,W,Y); 5 (T,I,P,S); 6 (I,C,F); 9 (Y,A,D,F,G,H,M,N,Q,S,T,V,W); 10 (F,L,M,N,V); 14 (L,A,C,D,E,H,M,N,Q,R,S,T); 15 (P,A,D,E,H,L,M,N,R,S,V,W); 16 (N,A,Q,R); 17 (D,E,G,K,N,Q,R,S,V,Y); 19 (A,D,F,H,I,K,M,N,T,V,Y); 20 (H,A,G,T\); 22 (N,A,C,D,E,F,G,H,I,K,M,Q,R,S,T,W); 23 (R,A,C,D,E,I,K,M,N,V,W); 24 (L,A,I,M,T,V); 25 (N,A,C,E,F,Q,T,Y); 26 (N,A,D,E,G,H,K,L,R,V,Y); 27 (D,C,E,F,G,I,K,M,N,Q,S,T,V,W); 28 (A,G,R,S,T); 29 (Q,A,D,E,F,G,I,M,N,P,R,V,W,Y); 30 (N,A,D,F,G,H,L,M,P,Q,R,S,T,V,W,Y); 31 (L,F,W,Y); 32 (K,A,D,G,I,M,S,Y); 33 (N,A,D,E,H,K,Q,S,T,V,W); 34 (V,A,D,F,G,H,L,Q,R,S,T,W,Y); 36 (I,F,M,V); 37 (T,A,C,D,E,G,K,Q,S,V); 38 (A,C,D,E,G,H,M,N,R,S,T,W); 39 (V,L,M,T); 41 (I,A,G,L,S,V); 44 (A,N,S,T\); 45 (Y,F,H,N); 48 (G,D,E,H,I,K,T,V); 49 (S,D,E,G,L,M,N,T,V,Y); 50 (S,D,E,G,H,K,M,N,Q,V); 51 (A,E,G,K,L,Q,R,S,V,W); 53 (V,E,N,T); 57 (V,C,I,L,S); 60 (T,A,E,G,H,N,V); 67 (N,A,D,E,G,K,L,M,Q,S,V,W,Y); 68 (Q,A,G,S,T); 69 (K,E,G,H,R,S,W); 70 (G,D,E,F,H,K,W,Y); 71 (T,A,D,L,M,R,S); 72 (V,I,R,T,Y); 73 (R,A,E,G,H,M,P,S,W); 78 (T,A,C,D,E,F,G,H,I,K,L,M,N,Q,R,S,V); 79 (K,A,M,R,S); 80 (S,A,C,D,E,F,G,K,M,P,R,T,V,W); 81 (E,A,C,D,F,G,H,M,N,Q,R,S,T,V,Y); 83 (I,A,D,E,G,H,M,Q,S,T,V,W,Y); 84 (S,A,E,F,G,I,K,M,P,V,W,Y); 85 (A,C,G,L,M,V); 86 (V,A,E,I,K,L,M,Q,R,S,T); 87 (N,A,D,F,G,L,Q,R,S,T,V,Y); 88 (N,A,C,E,I,M,R,S,T,V,Y); 89 (L,I,V); 90 (H,K,Q,R); 91 (A,C,E,F,H,I,K,L,M,S,T,V,W,Y); 92 (K,A,D,E,G,I,M,N,Q,R,S,T,Y); 93 (G,A,D,E,Q,R,S,T); 95 (A,D,F,G,L,M,N,Q,S,T,W,Y); 96 (V,A,I,L,T); 97 (Y,A,F,H,I,L,M,N,V); 100 (V,A,I,S); 102 (L,A,Q,S,T,V); 105 (R,A,G,K,L,M,S,T); 107 (N,A,E,G,I,K,M,P,S,W); 110 (A,C,D,E,G,H,K,L,N,Q,R,S,T,V,W,Y); 111 (T,A,C,D,E,F,G,I,K,L,M,N,S,V); 112 (E,A,D,F,M,N); 113 (L,A,C,D,E,F,G,I,N,P,Q,S,V,W,Y); 115 (D,A,E,F,G,H,I,K,M,N,R,S,T,W); 116 (A,C,G,S); 117 (V,A,K,R,S,T); 120 (D,A,N,R,S); 121 (P,A,E,F,Q,R,S,T); 122 (N,A,D,E,F,G,H,Q,R,S,V,W); 125 (N,C,D,R,W,Y); 126 (V,A,C,E,L,N,Q,R,S,T,W); 127 (E,A,F,H,K,N,P,Q,R,S,T,V); 128 (T,A,C,F,I,M,R,S,V,W); 129 (T,A,C,E,G,K,Q,R,V); 130 (S,A,D,F,G,H,I,K,L,N,P,Q,R,T,V,W); 131 (T,A,C,D,E,F,G,H,I,M,N,P,Q,R,V,W,Y); 132 (Y,D,E,G,H,Q,S,T,V); 133 (Q,D,F,G,H,I,M,N,P,R,S,V,W,Y); 135 (Q,A,D,E,G,H,I,L,M,N,R,T,V,W,Y); 136 (A,G,I,T); 139 (Q,A,D,F,G,H,K,L,M,N,R,V,W,Y); 140 (Y,F,H,I); 141 (D,A,C,E,F,G,I,K,M,Q,R,S,V,W,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 147 (N,A,D,F,G,H,K,P,Q,R,S,T); 148 (T,A,D,E,H,K,M,N,Q,R,V,W,Y); 149 (Y,E,F,G,I,L,M,V); 151 (S,A,D,E,F,G,H,K,N,P,Q,R,T,V,Y); 152 (F,H,W,Y); 153 (K,F,H,M,Q,R,S); 155 (R,A,C,E,F,G,K,N,T,V,Y); 157 (Y,D,F,G,I,M,P,Q,R,S,V,W); 162 (V,A,C,D,E,G,H,L,Q); 164 (W,A,C,I,L,M,N,Q,T); 166 (Q,A,C,D,E,F,G,H,K,N,P,R,S,T); 167 (S,A,C,D,E,F,G,H,I,L,Q,R,T,V,W,Y); 168 (R,A,K,L,S,T); 169 (G,A,D,E,F,H,K,Q,R,S,T,W); 170 (L,A,D,E,G,H,K,Q,R,S,V,W,Y); 171 (N,A,D,E,S); 172 (R,A,H,N,V,W); 174 (Y,C,E,F,H,M,W); 176 (L,A,G,H,I,N,Q); 179 (D,A,C,E,F,H,K,L,M,N,R,S,T,V,W,Y); 180 (D,A,E,M,N,Q,R,T,Y); 182 (D,A,E,F,H,I,K,M,N,R,S,T,V,W,Y); 189 (S,A,D,E,G,N); 191 (Y,C,H,M,R,W); 192 (G,A,E,H,M,Q,S,V,Y); 204 (F,C,M); 205 (N,A,C,D,E,F,G,I,M,S,V,W,Y); 206 (H,A,C,D,E,L,M,Q,W,Y); 207 (P,A,D,E,G,H,L,N,R,S,T); 208 (D,E,F,G,I,L,N,Q,R,S,V,Y); 210 (V,E,I,K,M,Q,R); 211 (N,A,F,H,I,K,L,M,Q,R,S,T,V,W,Y); 212 (E,A,S,T); 213 (T,A,E,N,S,V,Y); 214 (K,A,D,E,F,G,I,L,M,N,Q,S,Y); 215 (T,A,D,E,F,G,I,L,M,N,Q,S,V,Y); 218 (K,A,F,W); 220 (F,L,M,V); 222 (N,D,G,H,Q,T); 223 (T,E,G,R,S,V,Y); 224 (V,G,I,L,S,T); 225 (N,A,C,D,E,F,G,I,M,P,S,V,W,Y); 226 (L,F,V,Y); 229 (V,A,I,L,W); 231 (L,A,C,M,Q,S,T,V); 234 (V,A,E,G,I,L,N,S,T); 238 (K,C,F,L,Q,W); 241 (F,C,D,E,G,H,I,K,M,N,R,S,V,Y); 242 (M,A,C,E,L,Q,Y); 243 (R,A,C,D,E,F,G,H,L,Q,S,V,W,Y); 244 (D,A,C,E,G,H,N,S); 246 (V,A,I,M,T,Y); 247 (N,A,D,E,F,G,M,Q,R,S,V,W); 248 (N,A,C,D,E,F,G,H,I,K,M,Q,R,S,V,W); 249 (V,E,G,I,M,N); 250 (R,A,D,E,F,G,H,I,M,N,T,W); 251 (S,A,D,E,F,G,K,L,M,N,Q,T,V,W); 252 (T,A,E,F,G,H,K,L,M,N,Q,V); 253 (T,E,G,I,K,L,M,N,P,R,S,V,Y); 254 (G,A,E,H,I,K,M,P,Q,R,V,Y); 255 (K,A,D,G,I,M,N,R,S,T,V,W,Y); 256 (N,A,D,G,L,M,S,Y); 257 (L,A,I,K,M,W); 258 (F,A,G,H,K,M,P,S,T,V,W,Y); 259 (A,G,H,N,S,T); 264 (W,F,L,M); 265 (H,A,D,E,G,I,K,M,R,V,W); 266 (Y,D,E,G,K,L,M,N,P,Q,R,S,T,V,W); 267 (D,G,H,K,S); 268 (V,A,D,E,F,G,I,K,L,M,N,P,Q,R,W,Y); 269 (N,A,D,E,G,H,I,K,Q,S,T,Y); 270 (K,A,S,T,V); 272 (N,D,E,H,I,K,Q,V); 273 (S,D,E,Y); 275 (I,F,L,M,V,W,Y); 276 (T,A,C,D,E,G,I,K,L,Q,W,Y); 278 (T,I,V); 279 (N,A,D,E,G,H,Q,S,T,V,W); 280 (G,A,D,E,K,M,N,Q,R,S,Y); 281 (T,A,D,E,H,K,N,Q,R,S); 282 (M,E,H,I,L,N,Q,S,Y); 283 (S,A,C,D,E,G,H,K,M,R,T,W,Y); 285 (F,M,W,Y); 287 (V,A,I,S,T); 291 (F,D,G,I,K,M,N,Q,V,Y); 292 (R,E,K); 293 (F,A,E,L); 295 (D,A,E,H,K,N,Q,W,Y); 298 (N,E,F,H,I,Q,T); 299 (G,A,E,K,M,Q,S); 300 (G,A,E,F,H,K,N,Q,S,V,W,Y); 302 (G,A,D,E,H,K,M,N,R,S,V,W,Y); 307 (N,A,D,E,F,G,H,K,L,M,Q,R,S,T,V,W); 310 (N,D,E,G,H,K,M,R,V); 311 (N,A,D,E,G,H,Q,R,Y); 314 (M,A,S,T,V); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 318 (P,A,D,H,S,W); 319 (M,A,D,E,F,G,I,K,Q,R,S,T,V,W,Y); 320 (K,A,E,F,L,S,Y); 333 (T,A,D,E,F,G,N,Q,S,Y); 341 (Q,E,H,K,M,N,S); 342 (S,H,K,P,Q,R); 347 (L,A,I,M,Q); 356 (E,D,G,N,S,T,W); 359 (Y,A,D,F,H,L,M,N,R,S,T,V,W); 361 (C,A,E,I,K,Q,T,V); 362 (V,A,I,L,M); 368 (Y,A,D,E,F,I,K,L,N,Q,R,S,T,V,W); 371 (S,A,D,E,G,T,Y); 373 (G,A,D,E,H,Q,R,S); 374 (K,A,D,E,G,M,N); 376 (S,A,E,G,H,I,N,Q,R,T,V); 377 (S,A,D,E,F,G,H,L,N,P,Q,R,T,V,Y); 378 (Y,A,D,E,G,N,S,T,V); 379 (K,A,G,S); 380 (P,A,D,E,G,H,K,M,N,R,S,T,W,Y); 381 (I,A,E,F,L,Q,T); 384 (K,A,C,D,F,G,I,M,N,P,S,T,V,Y); 386 (L,F,M,T,V); 387 (N,A,G,H,I,L,Q,S,T,Y); 388 (A,L,T,V); 390 (K,H,L,Q,R,Y); 391 (V,A,D,F,H,K,L,M,N); 396 (T,D,E,F,H,I,L,M,N,P,Q,R,S,V,Y); 398 (R,H,N,Y); 404 (P,A,C,E,G,L,M,N,Q,S,V,W); 406 (I,L,M,T,V); 412 (E,A,D,G,L,M,Q,T); 415 (A,D,E,G,K,M,Q,R); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 427 (T,A,D,N); 430 (P,A,C,D,E,F,G,H,I,K,M,S,V,Y); 431 (G,A,D,E,K,N,P,Q,R,S,T,V); 433 (S,D,H,Q,R); 435 (W,A,D,E,F,H,K,L,M,N,Q,R,S,Y); 440 (T,A,D,E,H,I,L,M,N,S,V); 441 (S,D,E,G,H,Q,R); 442 (K,H,N,Q,R); 444 (G,D,H,K,N); 446 (V,A,D,E,H,I,L,M,N,R,S,T); 448 (T,H,R,S); 450 (K,H,M,Q,R,Y); 452 (G,A,H,M,N,Q,R); 454 (R,A,C,E,F,K,L,N,Q,S,V,W,Y); 455 (S,A,D,E,H,P,Q,R,T); 456 (G,D,E,N); 457 (T,E,G,K,N,S,V,Y); 459 (T,K,Q,R,V); 461 (D,E,G,Q,S); 462 (A,C,D,E,G,K,M,P,R,T,V); 463 (N,A,E,G,H,Q,R,S,T,Y); 467 (N,E,H,K,L,M,Q,R,V); 469 (W,A,D,E,G,H,K,N,P,S,V,Y); 471 (N,A,D,E,G,K,Q,R); 472 (G,A,D,E,F,H,K,N,T); 473 (G,A,D,E,F,K,Q,R,S,V,Y); and 480 (K,E,G,I,M,P,S,V,W,Y).

The productive positions in PcuAmy1-v1 that fall within the previously described Productivity Scores of "3" and "4," and the substitutions within those positions that are combinable, are listed below. The position numbering is based on the mature PcuAmy1 polypeptide (SEQ ID NO: 3):
LIST C:
1 (G,D,E,F,H,I,K,M,N,R,S,V,W); 2 (D,A,E,H,M,N,V,Y); 4 (G,D,E,F,H,I,L,M,N,P,Q,S,V,W,Y); 9 (Y,A,D,F,G,H,M,N,Q,S,T,V,W); 14 (L,A,C,D,E,H,M,N,Q,R,S,T); 15 (P,A,D,E,H,L,M,N,R,S,V,W); 17 (D,E,G,K,N,Q,R,S,V,Y); 19 (A,D,F,H,I,K,M,N,T,V,Y); 22 (N,A,C,D,E,F,G,H,I,K,M,Q,R,S,T,W); 23 (R,A,C,D,E,I,K,M,N,V,W); 25 (N,A,C,E,F,Q,T,Y); 26 (N,A,D,E,G,H,K,L,R,V,Y); 27 (D,C,E,F,G,I,K,M,N,Q,S,T,V,W); 29 (Q,A,D,E,F,G,I,M,N,P,R,V,W,Y); 30 (N,A,D,F,G,H,L,M,P,Q,R,S,T,V,W,Y); 32 (K,A,D,G,I,M,S,Y); 33 (N,A,D,E,H,K,Q,S,T,V,W); 34 (V,A,D,F,G,H,L,Q,R,S,T,W,Y); 37 (T,A,C,D,E,G,K,Q,S,V); 38 (A,C,D,E,G,H,M,N,R,S,T,W); 49 (S,D,E,G,L,M,N,T,V,Y); 50 (S,D,E,G,H,K,M,N,Q,V); 51 (A,E,G,K,L,Q,R,S,V,W); 60 (T,A,E,G,H,N,V); 67 (N,A,D,E,G,K,L,M,Q,S,V,W,Y); 70 (G,D,E,F,H,K,W,Y); 73 (R,A,E,G,H,M,P,S,W); 78 (T,A,C,D,E,F,G,H,I,K,L,M,N,Q,R,S,V); 80 (S,A,C,D,E,F,G,K,M,P,R,T,V,W); 81 (E,A,C,D,F,G,H,M,N,Q,R,S,T,V,Y); 83 (I,A,D,E,G,H,M,Q,S,T,V,W,Y); 84 (S,A,E,F,G,I,K,M,P,V,W,Y); 86 (V,A,E,I,K,L,M,Q,R,S,T); 87 (N,A,D,F,G,L,Q,R,S,T,V,Y); 88 (N,A,C,E,I,M,R,S,T,V,Y); 91 (A,C,E,F,H,I,K,L,M,S,T,V,W,Y); 92 (K,A,D,E,G,I,M,N,Q,R,S,T,Y); 95 (A,D,F,G,L,M,N,Q,S,T,W,Y); 97 (Y,A,F,H,I,L,M,N,V); 105 (R,A,G,K,L,M,S,T); 107 (N,A,E,G,I,K,M,P,S,W); 110 (A,C,D,E,G,H,K,L,N,Q,R,S,T,V,W,Y); 111 (T,A,C,D,E,F,G,I,K,L,M,N,S,V); 113 (L,A,C,D,E,F,G,I,N,P,Q,S,V,W,Y); 115 (D,A,E,F,G,H,I,K,M,N,R,S,T,W); 121 (P,A,E,F,Q,R,S,T); 122 (N,A,D,E,F,G,H,Q,R,S,V,W); 126 (V,A,C,E,L,N,Q,R,S,T,W); 127 (E,A,F,H,K,N,P,Q,R,S,T,V); 128 (T,A,C,F,I,M,R,S,V,W); 129 (T,A,C,E,G,K,Q,R,V); 130 (S,A,D,F,G,H,I,K,L,N,P,Q,R,T,V,W); 131 (T,A,C,D,E,F,G,H,I,M,N,P,Q,R,V,W,Y); 132 (Y,D,E,G,H,Q,S,T,V); 133 (Q,D,F,G,H,I,M,N,P,R,S,V,W,Y); 135 (Q,A,D,E,G,H,I,L,M,N,R,T,V,W,Y); 139 (Q,A,D,F,G,H,K,L,M,N,R,V,W,Y); 141 (D,A,C,E,F,G,I,K,M,Q,R,S,V,W,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 147 (N,A,D,F,G,H,K,P,Q,R,S,T); 148 (T,A,D,E,H,K,M,N,Q,R,V,W,Y); 149 (Y,E,F,G,I,L,M,V); 151 (S,A,D,E,F,G,H,K,N,P,Q,R,T,V,Y); 155 (R,A,C,E,F,G,K,N,T,V,Y); 157 (Y,D,F,G,I,M,P,Q,R,S,V,W); 162 (V,A,C,D,E,G,H,L,Q); 164 (W,A,C,I,L,M,N,Q,T); 166 (Q,A,C,D,E,F,G,H,K,N,P,R,S,T); 167 (S,A,C,D,E,F,G,H,I,L,Q,R,T,V,W,Y); 169 (G,A,D,E,F,H,K,Q,R,S,T,W); 170 (L,A,D,E,G,H,K,Q,R,S,V,W,Y); 174 (Y,C,E,F,H,M,W); 179 (D,A,C,E,F,H,K,L,M,N,R,S,T,V,W,Y); 180 (D,A,E,M,N,Q,R,T,Y); 182 (D,A,E,F,H,I,K,M,N,R,S,T,V,W,Y); 189 (S,A,D,E,G,N); 192 (G,A,E,H,M,Q,S,V,Y); 205 (N,A,C,D,E,F,G,I,M,S,V,W,Y); 206 (H,A,C,D,E,L,M,Q,W,Y); 207 (P,A,D,E,G,H,L,N,R,S,T); 208 (D,E,F,G,I,L,N,Q,R,S,V,Y); 211 (N,A,F,H,I,K,L,M,Q,R,S,T,V,W,Y); 214 (K,A,D,E,F,G,I,L,M,N,Q,S,Y); 215 (T,A,D,E,F,G,I,L,M,N,Q,S,V,Y); 225 (N,A,C,D,E,F,G,I,M,P,S,V,W,Y); 234 (V,A,E,G,I,L,N,S,T); 241 (F,C,D,E,G,H,I,K,M,N,R,S,V,Y); 242 (M,A,C,E,L,Q,Y); 243 (R,A,C,D,E,F,G,H,L,Q,S,V,W,Y); 247 (N,A,D,E,F,G,M,Q,R,S,V,W); 248 (N,A,C,D,E,F,G,H,I,K,M,Q,R,S,V,W); 250 (R,A,D,E,F,G,H,I,M,N,T,W); 251 (S,A,D,E,F,G,K,L,M,N,Q,T,V,W); 252 (T,A,E,F,G,H,K,L,M,N,Q,V); 253 (T,E,G,I,K,L,M,N,P,R,S,V,Y); 254 (G,A,E,H,I,K,M,P,Q,R,V,Y); 255 (K,A,D,G,I,M,N,R,S,T,V,W,Y); 256 (N,A,D,G,L,M,S,Y); 258 (F,A,G,H,K,M,P,S,T,V,W,Y); 265 (H,A,D,E,G,I,K,M,R,V,W); 266 (Y,D,E,G,K,L,M,N,P,Q,R,S,T,V,W); 268 (V,A,D,E,F,G,I,K,L,M,N,P,Q,R,W,Y); 269 (N,A,D,E,G,H,I,K,Q,S,T,Y); 272 (N,D,E,H,I,K,Q,V); 276 (T,A,C,D,E,G,I,K,L,Q,W,Y); 279 (N,A,D,E,G,H,Q,S,T,V,W); 280 (G,A,D,E,K,M,N,Q,R,S,Y); 281 (T,A,D,E,H,K,N,Q,R,S); 282 (M,E,H,I,L,N,Q,S,Y); 283 (S,A,C,D,E,G,H,K,M,R,T,W,Y); 291 (F,D,G,I,K,M,N,Q,V,Y); 295 (D,A,E,H,K,N,Q,W,Y); 300 (G,A,E,F,H,K,N,Q,S,V,W,Y); 302 (G,A,D,E,H,K,M,N,R,S,V,W,Y); 307 (N,A,D,E,F,G,H,K,L,M,Q,R,S,T,V,W); 310 (N,D,E,G,H,K,M,R,V); 311 (N,A,D,E,G,H,Q,R,Y); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 319 (M,A,D,E,F,G,I,K,Q,R,S,T,V,W,Y); 333 (T,A,D,E,F,G,N,Q,S,Y); 359 (Y,A,D,F,H,L,M,N,R,S,T,V,W); 361 (C,A,E,I,K,Q,T,V); 368 (Y,A,D,E,F,I,K,L,N,Q,R,S,T,V,W); 371 (S,A,D,E,G,T,Y); 374 (K,A,D,E,G,M,N); 376 (S,A,E,G,H,I,N,Q,R,T,V); 377 (S,A,D,E,F,G,H,L,N,P,Q,R,T,V,Y); 378 (Y,A,D,E,G,N,S,T,V); 380 (P,A,D,E,G,H,K,M,N,R,S,T,W,Y); 381 (I,A,E,F,L,Q,T); 384 (K,A,C,D,F,G,I,M,N,P,S,T,V,Y); 387 (N,A,G,H,I,L,Q,S,T,Y); 391 (V,A,D,F,H,K,L,M,N); 396 (T,D,E,F,H,I,L,M,N,P,Q,R,S,V,Y); 404 (P,A,C,E,G,L,M,N,Q,S,V,W); 412 (E,A,D,G,L,M,Q,T); 415 (A,D,E,G,K,M,Q,R); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 430 (P,A,C,D,E,F,G,H,I,K,M,S,V,Y); 431 (G,A,D,E,K,N,P,Q,R,S,T,V); 435 (W,A,D,E,F,H,K,L,M,N,Q,R,S,Y); 440 (T,A,D,E,H,I,L,M,N,S,V); 446 (V,A,D,E,H,I,L,M,N,R,S,T); 454 (R,A,C,E,F,K,L,N,Q,S,V,W,Y); 455 (S,A,D,E,H,P,Q,R,T); 462 (A,C,D,E,G,K,M,P,R,T,V); 463 (N,A,E,G,H,Q,R,S,T,Y); 467 (N,E,H,K,L,M,Q,R,V); 469 (W,A,D,E,G,H,K,N,P,S,V,Y); 471 (N,A,D,E,G,K,Q,R); 472 (G,A,D,E,F,H,K,N,T); 473 (G,A,D,E,F,K,Q,R,S,V,Y); and 480 (K,E,G,I,M,P,S,V,W,Y).

The productive positions in PcuAmy1-v1 that fall within the previously described Productivity Scores of "4," and the substitutions within those positions that are combinable, are listed below. The position numbering is based on the mature PcuAmy1 polypeptide (SEQ ID NO: 3):
LIST D:
1 (G,D,E,F,H,I,K,M,N,R,S,V,W); 4 (G,D,E,F,H,I,L,M,N,P,Q,S,V,W,Y); 9 (Y,A,D,F,G,H,M,N,Q,S,T,V,W); 22 (N,A,C,D,E,F,G,H,I,K,M,Q,R,S,T,W); 27 (D,C,E,F,G,I,K,M,N,Q,S,T,V,W); 29 (Q,A,D,E,F,G,I,M,N,P,R,V,W,Y); 30 (N,A,D,F,G,H,L,M,P,Q,R,S,T,V,W,Y); 34 (V,A,D,F,G,H,L,Q,R,S,T,W,Y); 67 (N,A,D,E,G,K,L,M,Q,S,V,W,Y); 78 (T,A,C,D,E,F,G,H,I,K,L,M,N,Q,R,S,V); 80 (S,A,C,D,E,F,G,K,M,P,R,T,V,W); 81 (E,A,C,D,F,G,H,M,N,Q,R,S,T,V,Y); 83 (I,A,D,E,G,H,M,Q,S,T,V,W,Y); 84 (S,A,E,F,G,I,K,M,P,V,W,Y); 91 (A,C,E,F,H,I,K,L,M,S,T,V,W,Y); 110 (A,C,D,E,G,H,K,L,N,Q,R,S,T,V,W,Y); 111 (T,A,C,D,E,F,G,I,K,L,M,N,S,V); 113 (L,A,C,D,E,F,G,I,N,P,Q,S,V,W,Y); 115 (D,A,E,F,G,H,I,K,M,N,R,S,T,W); 122 (N,A,D,E,F,G,H,Q,R,S,V,W); 126 (V,A,C,E,L,N,Q,R,S,T,W); 130 (S,A,D,F,G,H,I,K,L,N,P,Q,R,T,V,W); 131 (T,A,C,D,E,F,G,H,I,M,N,P,Q,R,V,W,Y); 133 (Q,D,F,G,H,I,M,N,P,R,S,V,W,Y); 135 (Q,A,D,E,G,H,I,L,M,N,R,T,V,W,Y); 139 (Q,A,D,F,G,H,K,L,M,N,R,V,W,Y); 141 (D,A,C,E,F,G,I,K,M,Q,R,S,V,W,Y); 143 (P,A,D,E,F,G,H,I,N,Q,S,V,W); 144 (G,A,C,D,E,H,I,K,L,M,N,P,Q,S,T,V,Y); 146 (G,A,D,E,F,H,K,M,N,P,Q,S,V,W,Y); 148 (T,A,D,E,H,K,M,N,Q,R,V,W,Y); 151 (S,A,D,E,F,G,H,K,N,P,Q,R,T,V,Y); 157 (Y,D,F,G,I,M,P,Q,R,S,V,W); 166 (Q,A,C,D,E,F,G,H,K,N,P,R,S,T); 167 (S,A,C,D,E,F,G,H,I,L,Q,R,T,V,W,Y); 170 (L,A,D,E,G,H,K,Q,R,S,V,W,Y); 179 (D,A,C,E,F,H,K,L,M,N,R,S,T,V,W,Y); 182 (D,A,E,F,H,I,K,M,N,R,S,T,V,W,Y); 205 (N,A,C,D,E,F,G,I,M,S,V,W,Y); 208 (D,E,F,G,I,L,N,Q,R,S,V,Y); 211 (N,A,F,H,I,K,L,M,Q,R,S,T,V,W,Y); 214 (K,A,D,E,F,G,I,L,M,N,Q,S,Y); 215 (T,A,D,E,F,G,I,L,M,N,Q,S,V,Y); 225 (N,A,C,D,E,F,G,I,M,P,S,V,W,Y); 241 (F,C,D,E,G,H,I,K,M,N,R,S,V,Y); 243 (R,A,C,D,E,F,G,H,L,Q,S,V,W,Y); 247 (N,A,D,E,F,G,M,Q,R,S,V,W); 248 (N,A,C,D,E,F,G,H,I,K,M,Q,R,S,V,W); 250 (R,A,D,E,F,G,H,I,M,N,T,W); 253 (T,E,G,I,K,L,M,N,P,R,S,V,Y); 255 (K,A,D,G,I,M,N,R,S,T,V,W,Y); 265 (H,A,D,E,G,I,K,M,R,V,W); 266 (Y,D,E,G,K,L,M,N,P,Q,R,S,T,V,W); 268 (V,A,D,E,F,G,I,K,L,M,N,P,Q,R,W,Y); 269 (N,A,D,E,G,H,I,K,Q,S,T,Y); 276 (T,A,C,D,E,G,I,K,L,Q,W,Y); 283 (S,A,C,D,E,G,H,K,M,R,T,W,Y); 302 (G,A,D,E,H,K,M,N,R,S,V,W,Y); 307 (N,A,D,E,F,G,H,K,L,M,Q,R,S,T,V,W); 315 (S,D,E,G,H,I,L,M,N,R,T,V,Y); 316 (S,A,D,E,G,H,I,L,N,Q,T,V,W,Y); 317 (N,A,D,E,F,I,K,Q,V,W,Y); 319 (M,A,D,E,F,G,I,K,Q,R,S,T,V,W,Y); 359 (Y,A,D,F,H,L,M,N,R,S,T,V,W); 368 (Y,A,D,E,F,I,K,L,N,Q,R,S,T,V,W); 377 (S,A,D,E,F,G,H,L,N,P,Q,R,T,V,Y); 380 (P,A,D,E,G,H,K,M,N,R,S,T,W,Y); 384 (K,A,C,D,F,G,I,M,N,P,S,T,V,Y); 396 (T,D,E,F,H,I,L,M,N,P,Q,R,S,V,Y); 416 (A,D,E,F,G,I,K,L,M,N,P,Q,T,V,Y); 418 (A,E,G,H,K,L,M,N,Q,R,S,T,V,W,Y); 419 (G,D,E,F,H,K,M,Q,R,S,T,VW,Y); 430 (P,A,C,D,E,F,G,H,I,K,M,S,V,Y); 435 (W,A,D,E,F,H,K,L,M,N,Q,R,S,Y); and 454 (R,A,C,E,F,K,L,N,Q,S,V,W,Y).

### Example 5

### Protein Surface Modifications

Positions and substitutions contributing to favorable surface modifications, such as altered charge or hydrophobicity, were identified in PcuAmy1-v1. Productive positions containing all combinable substitutions were determined using the criteria for productive positions as described in Example 4. The data were further analyzed to determine substitutions that change hydrophobicity or charge, and maintain all important properties. The hydrophobicity of each substitution was determined using the methods of White and Wimley (White,S.H. and Wimley, W.C. (1999) Annu. Rev. Biophys. Biomol. Struct. 28:319-65). A subgroup of combinable positions and substitutions demonstrating favorable surface modifications in PcuAmy1-v1 is shown in Table 5.1.

Table 5.1 sets forth a subgroup of the combinable positions and substitutions that demonstrate favorable surface modifications (HYDRO stands for hydrophobic residue, and, CHARGE stands for charged residue). The position numbering is based on the mature PcuAmy1 polypeptide (SEQ ID NO: 3):

| **Table 5.1. Combinable positions and substitutions** | | | |
|---|---|---|---|
| POS | Variant | Effect | Effect |
| 1 | G001D | HYDRO | CHARGE |
| 1 | G001E | HYDRO | |
| 1 | G001H | HYDRO | CHARGE |
| 1 | G001I | HYDRO | |
| 1 | G001M | HYDRO | |
| 1 | G001R | | CHARGE |
| 1 | G001V | HYDRO | |
| 4 | G004D | HYDRO | CHARGE |
| 4 | G004E | HYDRO | |
| 4 | G004F | HYDRO | |
| 4 | G004H | HYDRO | CHARGE |
| 4 | G004I | HYDRO | |
| 4 | G004L | HYDRO | |
| 4 | G004P | HYDRO | |
| 4 | G004V | HYDRO | |
| 4 | G004W | HYDRO | |
| 4 | G004Y | HYDRO | |
| 9 | Y009A | HYDRO | |
| 9 | Y009F | HYDRO | |
| 9 | Y009G | HYDRO | |
| 9 | Y009H | HYDRO | CHARGE |
| 9 | Y009S | HYDRO | |
| 9 | Y009T | HYDRO | |
| 9 | Y009W | HYDRO | |
| 10 | F010M | HYDRO | |
| 14 | L014A | HYDRO | |
| 14 | L014D | HYDRO | CHARGE |
| 14 | L014E | HYDRO | |
| 14 | L014N | HYDRO | |
| 15 | P015R | HYDRO | CHARGE |
| 22 | N022D | HYDRO | CHARGE |
| 22 | N022E | HYDRO | |
| 22 | N022H | HYDRO | CHARGE |
| 22 | N022M | HYDRO | |
| 25 | N025E | HYDRO | |
| 26 | N026Y | HYDRO | |
| 29 | Q029D | HYDRO | CHARGE |
| 29 | Q029E | HYDRO | |
| 29 | Q029F | HYDRO | |
| 29 | Q029I | HYDRO | |
| 29 | Q029M | HYDRO | |
| 29 | Q029R | HYDRO | CHARGE |
| 29 | Q029V | HYDRO | |
| 29 | Q029W | HYDRO | |
| 29 | Q029Y | HYDRO | |
| 30 | N030D | HYDRO | CHARGE |
| 30 | N030F | HYDRO | |
| 30 | N030H | HYDRO | CHARGE |
| 30 | N030L | HYDRO | |
| 30 | N030M | HYDRO | |
| 30 | N030R | HYDRO | CHARGE |
| 30 | N030V | HYDRO | |
| 30 | N030W | HYDRO | |
| 30 | N030Y | HYDRO | |
| 32 | K032D | | CHARGE |
| 32 | K032G | HYDRO | CHARGE |
| 32 | K032S | HYDRO | CHARGE |
| 32 | K032Y | HYDRO | CHARGE |
| 33 | N033D | HYDRO | CHARGE |
| 33 | N033E | HYDRO | |
| 33 | N033H | HYDRO | CHARGE |
| 33 | N033K | HYDRO | CHARGE |
| 33 | N033V | HYDRO | |
| 34 | V034D | HYDRO | CHARGE |
| 34 | V034F | HYDRO | |
| 34 | V034G | HYDRO | |
| 34 | V034R | HYDRO | CHARGE |
| 37 | T037K | HYDRO | CHARGE |
| 41 | I041A | HYDRO | |
| 50 | S050D | HYDRO | CHARGE |
| 50 | S050E | HYDRO | |
| 67 | N067E | HYDRO | |
| 70 | G070D | HYDRO | CHARGE |
| 70 | G070H | HYDRO | CHARGE |
| 70 | G070K | HYDRO | CHARGE |
| 73 | R073A | HYDRO | CHARGE |
| 73 | R073E | HYDRO | CHARGE |
| 73 | R073G | | CHARGE |
| 73 | R073S | HYDRO | CHARGE |
| 78 | T078D | HYDRO | CHARGE |
| 78 | T078H | HYDRO | CHARGE |
| 78 | T078K | HYDRO | CHARGE |
| 78 | T078M | HYDRO | |
| 78 | T078R | HYDRO | CHARGE |
| 79 | K079A | HYDRO | CHARGE |
| 79 | K079S | HYDRO | CHARGE |
| 80 | S080D | HYDRO | CHARGE |
| 80 | S080E | HYDRO | |
| 80 | S080F | HYDRO | |
| 80 | S080K | HYDRO | CHARGE |
| 80 | S080M | HYDRO | |
| 80 | S080R | HYDRO | CHARGE |
| 80 | S080V | HYDRO | |
| 80 | S080W | HYDRO | |
| 81 | E081Q | HYDRO | |
| 83 | I083A | HYDRO | |
| 83 | I083D | HYDRO | CHARGE |
| 83 | I083G | HYDRO | |
| 83 | I083Q | HYDRO | |
| 84 | S084F | HYDRO | |
| 85 | A085M | HYDRO | |
| 87 | N087R | HYDRO | CHARGE |
| 88 | N088R | HYDRO | CHARGE |
| 90 | H090Q | HYDRO | CHARGE |
| 91 | A091I | HYDRO | |
| 91 | A091K | HYDRO | CHARGE |
| 91 | A091M | HYDRO | |
| 92 | K092G | HYDRO | CHARGE |
| 92 | K092Q | HYDRO | CHARGE |
| 92 | K092R | HYDRO | |
| 93 | G093D | HYDRO | CHARGE |
| 93 | G093E | HYDRO | |
| 93 | G093R | | CHARGE |
| 95 | A095F | HYDRO | |
| 95 | A095Y | HYDRO | |
| 97 | Y097A | HYDRO | |
| 97 | Y097F | HYDRO | |
| 97 | Y097H | HYDRO | CHARGE |
| 102 | L102T | HYDRO | |
| 110 | A110D | HYDRO | CHARGE |
| 110 | A110E | HYDRO | |
| 110 | A110H | HYDRO | CHARGE |
| 110 | A110K | HYDRO | CHARGE |
| 110 | A110L | HYDRO | |
| 110 | A110W | HYDRO | |
| 110 | A110Y | HYDRO | |
| 111 | T111D | HYDRO | CHARGE |
| 111 | T111F | HYDRO | |
| 112 | E112M | HYDRO | |
| 113 | L113A | HYDRO | |
| 113 | L113C | HYDRO | |
| 113 | L113D | HYDRO | CHARGE |
| 113 | L113E | HYDRO | |
| 113 | L113G | HYDRO | |
| 113 | L113N | HYDRO | |
| 113 | L113P | HYDRO | |
| 113 | L113Q | HYDRO | |
| 115 | D115A | HYDRO | CHARGE |
| 115 | D115H | HYDRO | CHARGE |
| 115 | D115K | | CHARGE |
| 115 | D115S | HYDRO | CHARGE |
| 117 | V117K | HYDRO | CHARGE |
| 122 | N122E | HYDRO | |
| 122 | N122F | HYDRO | |
| 122 | N122H | HYDRO | CHARGE |
| 122 | N122R | HYDRO | CHARGE |
| 122 | N122W | HYDRO | |
| 125 | N125D | HYDRO | CHARGE |
| 125 | N125Y | HYDRO | |
| 126 | V126A | HYDRO | |
| 126 | V126E | HYDRO | |
| 126 | V126N | HYDRO | |
| 126 | V126Q | HYDRO | |
| 126 | V126R | HYDRO | CHARGE |
| 126 | V126S | HYDRO | |
| 127 | E127A | HYDRO | |
| 127 | E127P | HYDRO | |
| 127 | E127Q | HYDRO | |
| 127 | E127V | HYDRO | |
| 128 | T128I | HYDRO | |
| 128 | T128M | HYDRO | |
| 128 | T128R | HYDRO | CHARGE |
| 130 | S130D | HYDRO | CHARGE |
| 130 | S130F | HYDRO | |
| 130 | S130H | HYDRO | CHARGE |
| 130 | S130I | HYDRO | |
| 130 | S130K | HYDRO | CHARGE |
| 130 | S130L | HYDRO | |
| 130 | S130R | HYDRO | CHARGE |
| 130 | S130V | HYDRO | |
| 130 | S130W | HYDRO | |
| 131 | T131E | HYDRO | |
| 131 | T131F | HYDRO | |
| 131 | T131H | HYDRO | CHARGE |
| 131 | T131I | HYDRO | |
| 131 | T131M | HYDRO | |
| 131 | T131R | HYDRO | CHARGE |
| 131 | T131W | HYDRO | |
| 131 | T131Y | HYDRO | |
| 132 | Y132E | HYDRO | |
| 132 | Y132H | HYDRO | CHARGE |
| 132 | Y132Q | HYDRO | |
| 133 | Q133D | HYDRO | CHARGE |
| 133 | Q133I | HYDRO | |
| 133 | Q133M | HYDRO | |
| 133 | Q133R | HYDRO | CHARGE |
| 133 | Q133V | HYDRO | |
| 135 | Q135D | HYDRO | CHARGE |
| 135 | Q135E | HYDRO | |
| 135 | Q135H | HYDRO | CHARGE |
| 135 | Q135L | HYDRO | |
| 135 | Q135R | HYDRO | CHARGE |
| 135 | Q135V | HYDRO | |
| 135 | Q135Y | HYDRO | |
| 139 | Q139D | HYDRO | CHARGE |
| 139 | Q139K | HYDRO | CHARGE |
| 139 | Q139L | HYDRO | |
| 139 | Q139R | HYDRO | CHARGE |
| 139 | Q139W | HYDRO | |
| 140 | Y140F | HYDRO | |
| 141 | D141G | HYDRO | CHARGE |
| 141 | D141I | HYDRO | CHARGE |
| 143 | P143E | HYDRO | |
| 143 | P143F | HYDRO | |
| 143 | P143G | HYDRO | |
| 143 | P143H | HYDRO | CHARGE |
| 143 | P143I | HYDRO | |
| 146 | G146H | HYDRO | CHARGE |
| 146 | G146K | HYDRO | CHARGE |
| 146 | G146M | HYDRO | |
| 146 | G146V | HYDRO | |
| 146 | G146W | HYDRO | |
| 146 | G146Y | HYDRO | |
| 147 | N147D | HYDRO | CHARGE |
| 147 | N147H | HYDRO | CHARGE |
| 147 | N147K | HYDRO | CHARGE |
| 148 | T148H | HYDRO | CHARGE |
| 148 | T148K | HYDRO | CHARGE |
| 149 | Y149F | HYDRO | |
| 151 | S151D | HYDRO | CHARGE |
| 151 | S151E | HYDRO | |
| 151 | S151F | HYDRO | |
| 151 | S151H | HYDRO | CHARGE |
| 151 | S151K | HYDRO | CHARGE |
| 151 | S151R | HYDRO | CHARGE |
| 151 | S151V | HYDRO | |
| 152 | F152H | HYDRO | CHARGE |
| 153 | K153R | HYDRO | |
| 155 | R155A | HYDRO | CHARGE |
| 155 | R155C | HYDRO | CHARGE |
| 155 | R155E | HYDRO | CHARGE |
| 155 | R155F | HYDRO | CHARGE |
| 155 | R155G | | CHARGE |
| 155 | R155N | HYDRO | CHARGE |
| 155 | R155Y | HYDRO | CHARGE |
| 157 | Y157D | HYDRO | CHARGE |
| 157 | Y157F | HYDRO | |
| 157 | Y157Q | HYDRO | |
| 157 | Y157S | HYDRO | |
| 157 | Y157W | HYDRO | |
| 166 | Q166D | HYDRO | CHARGE |
| 166 | Q166H | HYDRO | CHARGE |
| 167 | S167D | HYDRO | CHARGE |
| 167 | S167E | HYDRO | |
| 167 | S167F | HYDRO | |
| 167 | S167H | HYDRO | CHARGE |
| 167 | S167L | HYDRO | |
| 167 | S167R | HYDRO | CHARGE |
| 168 | R168S | HYDRO | CHARGE |
| 169 | G169E | HYDRO | |
| 169 | G169F | HYDRO | |
| 169 | G169H | HYDRO | CHARGE |
| 169 | G169K | HYDRO | CHARGE |
| 169 | G169R | | CHARGE |
| 170 | L170A | HYDRO | |
| 170 | L170D | HYDRO | CHARGE |
| 170 | L170E | HYDRO | |
| 170 | L170G | HYDRO | |
| 170 | L170H | HYDRO | CHARGE |
| 170 | L170K | HYDRO | CHARGE |
| 170 | L170Q | HYDRO | |
| 170 | L170R | HYDRO | CHARGE |
| 170 | L170S | HYDRO | |
| 174 | Y174W | HYDRO | |
| 176 | L176H | HYDRO | CHARGE |
| 176 | L176N | HYDRO | |
| 179 | D179F | HYDRO | CHARGE |
| 179 | D179H | HYDRO | CHARGE |
| 179 | D179K | | CHARGE |
| 179 | D179L | HYDRO | CHARGE |
| 179 | D179M | HYDRO | CHARGE |
| 179 | D179N | HYDRO | CHARGE |
| 179 | D179S | HYDRO | CHARGE |
| 179 | D179T | HYDRO | CHARGE |
| 179 | D179V | HYDRO | CHARGE |
| 179 | D179W | HYDRO | CHARGE |
| 179 | D179Y | HYDRO | CHARGE |
| 180 | D180T | HYDRO | CHARGE |
| 182 | D182A | HYDRO | CHARGE |
| 182 | D182F | HYDRO | CHARGE |
| 182 | D182H | HYDRO | CHARGE |
| 182 | D182I | HYDRO | CHARGE |
| 182 | D182K | | CHARGE |
| 182 | D182M | HYDRO | CHARGE |
| 182 | D182R | HYDRO | CHARGE |
| 182 | D182S | HYDRO | CHARGE |
| 182 | D182T | HYDRO | CHARGE |
| 182 | D182W | HYDRO | CHARGE |
| 182 | D182Y | HYDRO | CHARGE |
| 189 | S189D | HYDRO | CHARGE |
| 189 | S189E | HYDRO | |
| 191 | Y191H | HYDRO | CHARGE |
| 191 | Y191W | HYDRO | |
| 192 | G192H | HYDRO | CHARGE |
| 192 | G192M | HYDRO | |
| 204 | F204M | HYDRO | |
| 205 | N205D | HYDRO | CHARGE |
| 205 | N205E | HYDRO | |
| 205 | N205F | HYDRO | |
| 206 | H206A | HYDRO | CHARGE |
| 206 | H206C | HYDRO | CHARGE |
| 206 | H206D | HYDRO | CHARGE |
| 206 | H206E | | CHARGE |
| 206 | H206M | HYDRO | CHARGE |
| 206 | H206W | HYDRO | CHARGE |
| 206 | H206Y | HYDRO | CHARGE |
| 207 | P207E | HYDRO | |
| 207 | P207G | HYDRO | |
| 208 | D208F | HYDRO | CHARGE |
| 208 | D208G | HYDRO | CHARGE |
| 208 | D208L | HYDRO | CHARGE |
| 208 | D208N | HYDRO | CHARGE |
| 208 | D208Q | HYDRO | CHARGE |
| 208 | D208R | HYDRO | CHARGE |
| 208 | D208S | HYDRO | CHARGE |
| 210 | V210K | HYDRO | CHARGE |
| 210 | V210Q | HYDRO | |
| 210 | V210R | HYDRO | CHARGE |
| 211 | N211H | HYDRO | CHARGE |
| 211 | N211M | HYDRO | |
| 211 | N211R | HYDRO | CHARGE |
| 211 | N211W | HYDRO | |
| 211 | N211Y | HYDRO | |
| 212 | E212T | HYDRO | |
| 214 | K214G | HYDRO | CHARGE |
| 214 | K214M | HYDRO | CHARGE |
| 214 | K214Q | HYDRO | CHARGE |
| 214 | K214S | HYDRO | CHARGE |
| 215 | T215D | HYDRO | CHARGE |
| 215 | T215E | HYDRO | |
| 215 | T215F | HYDRO | |
| 215 | T215L | HYDRO | |
| 215 | T215M | HYDRO | |
| 215 | T215Y | HYDRO | |
| 221 | V221A | HYDRO | |
| 222 | N222D | HYDRO | CHARGE |
| 224 | V224G | HYDRO | |
| 224 | V224S | HYDRO | |
| 225 | N225D | HYDRO | CHARGE |
| 225 | N225E | HYDRO | |
| 229 | V229W | HYDRO | |
| 234 | V234A | HYDRO | |
| 238 | K238Q | HYDRO | CHARGE |
| 241 | F241R | HYDRO | CHARGE |
| 241 | F241S | HYDRO | |
| 243 | R243A | HYDRO | CHARGE |
| 243 | R243E | HYDRO | CHARGE |
| 243 | R243G | | CHARGE |
| 243 | R243L | HYDRO | CHARGE |
| 243 | R243Q | HYDRO | CHARGE |
| 243 | R243W | HYDRO | CHARGE |
| 247 | N247E | HYDRO | |
| 247 | N247M | HYDRO | |
| 247 | N247R | HYDRO | CHARGE |
| 247 | N247V | HYDRO | |
| 249 | V249E | HYDRO | |
| 249 | V249G | HYDRO | |
| 249 | V249N | HYDRO | |
| 250 | R250A | HYDRO | CHARGE |
| 250 | R250E | HYDRO | CHARGE |
| 250 | R250G | | CHARGE |
| 250 | R250N | HYDRO | CHARGE |
| 250 | R250T | HYDRO | CHARGE |
| 251 | S251E | HYDRO | |
| 252 | T252F | HYDRO | |
| 252 | T252H | HYDRO | CHARGE |
| 252 | T252K | HYDRO | CHARGE |
| 252 | T252M | HYDRO | |
| 253 | T253K | HYDRO | CHARGE |
| 253 | T253L | HYDRO | |
| 253 | T253R | HYDRO | CHARGE |
| 254 | G254E | HYDRO | |
| 254 | G254I | HYDRO | |
| 254 | G254V | HYDRO | |
| 255 | K255A | HYDRO | CHARGE |
| 255 | K255D | | CHARGE |
| 255 | K255G | HYDRO | CHARGE |
| 255 | K255I | HYDRO | CHARGE |
| 255 | K255L | 4 | |
| 255 | K255N | HYDRO | CHARGE |
| 255 | K255V | HYDRO | CHARGE |
| 255 | K255W | HYDRO | CHARGE |
| 265 | H265A | HYDRO | CHARGE |
| 265 | H265D | HYDRO | CHARGE |
| 265 | H265G | HYDRO | CHARGE |
| 266 | Y266E | HYDRO | |
| 266 | Y266N | HYDRO | |
| 266 | Y266Q | HYDRO | |
| 266 | Y266S | HYDRO | |
| 266 | Y266T | HYDRO | |
| 266 | Y266W | HYDRO | |
| 268 | V268D | HYDRO | CHARGE |
| 268 | V268E | HYDRO | |
| 268 | V268F | HYDRO | |
| 268 | V268N | HYDRO | |
| 269 | N269D | HYDRO | CHARGE |
| 269 | N269E | HYDRO | |
| 269 | N269H | HYDRO | CHARGE |
| 269 | N269I | HYDRO | |
| 269 | N269K | HYDRO | CHARGE |
| 273 | S273D | HYDRO | CHARGE |
| 276 | T276D | HYDRO | CHARGE |
| 276 | T276E | HYDRO | |
| 276 | T276L | HYDRO | |
| 276 | T276Y | HYDRO | |
| 279 | N279E | HYDRO | |
| 280 | G280K | HYDRO | CHARGE |
| 280 | G280M | HYDRO | |
| 280 | G280R | | CHARGE |
| 281 | T281K | HYDRO | CHARGE |
| 282 | M282E | HYDRO | |
| 282 | M282H | HYDRO | CHARGE |
| 283 | S283E | HYDRO | |
| 283 | S283H | HYDRO | CHARGE |
| 295 | D295H | HYDRO | CHARGE |
| 295 | D295K | | CHARGE |
| 295 | D295N | HYDRO | CHARGE |
| 298 | N298E | HYDRO | |
| 298 | N298H | HYDRO | CHARGE |
| 299 | G299K | HYDRO | CHARGE |
| 300 | G300K | HYDRO | CHARGE |
| 300 | G300Y | HYDRO | |
| 302 | G302D | HYDRO | CHARGE |
| 302 | G302E | HYDRO | |
| 302 | G302H | HYDRO | CHARGE |
| 302 | G302M | HYDRO | |
| 302 | G302R | | CHARGE |
| 303 | Y303F | HYDRO | |
| 307 | N307D | HYDRO | CHARGE |
| 307 | N307H | HYDRO | CHARGE |
| 307 | N307K | HYDRO | CHARGE |
| 307 | N307L | HYDRO | |
| 307 | N307M | HYDRO | |
| 307 | N307R | HYDRO | CHARGE |
| 307 | N307W | HYDRO | |
| 310 | N310E | HYDRO | |
| 310 | N310K | HYDRO | CHARGE |
| 310 | N310R | HYDRO | CHARGE |
| 310 | N310V | HYDRO | |
| 311 | N311D | HYDRO | CHARGE |
| 311 | N311E | HYDRO | |
| 314 | M314S | HYDRO | |
| 315 | S315E | HYDRO | |
| 315 | S315H | HYDRO | CHARGE |
| 315 | S315I | HYDRO | |
| 315 | S315V | HYDRO | |
| 316 | S316D | HYDRO | CHARGE |
| 316 | S316E | HYDRO | |
| 316 | S316H | HYDRO | CHARGE |
| 316 | S316I | HYDRO | |
| 316 | S316L | HYDRO | |
| 316 | S316V | HYDRO | |
| 316 | S316W | HYDRO | |
| 316 | S316Y | HYDRO | |
| 317 | N317K | HYDRO | CHARGE |
| 317 | N317V | HYDRO | |
| 319 | M319A | HYDRO | |
| 319 | M319D | HYDRO | CHARGE |
| 319 | M319E | HYDRO | |
| 319 | M319G | HYDRO | |
| 319 | M319K | HYDRO | CHARGE |
| 319 | M319Q | HYDRO | |
| 319 | M319R | HYDRO | CHARGE |
| 319 | M319S | HYDRO | |
| 319 | M319T | HYDRO | |
| 320 | K320E | | CHARGE |
| 320 | K320F | HYDRO | CHARGE |
| 320 | K320L | HYDRO | CHARGE |
| 333 | T333D | HYDRO | CHARGE |
| 337 | Q337D | HYDRO | CHARGE |
| 337 | Q337E | HYDRO | |
| 357 | Q357E | HYDRO | |
| 359 | Y359A | HYDRO | |
| 359 | Y359F | HYDRO | |
| 359 | Y359H | HYDRO | CHARGE |
| 359 | Y359N | HYDRO | |
| 359 | Y359R | HYDRO | CHARGE |
| 359 | Y359T | HYDRO | |
| 361 | C361I | HYDRO | |
| 368 | Y368F | HYDRO | |
| 368 | Y368K | HYDRO | CHARGE |
| 368 | Y368S | HYDRO | |
| 371 | S371E | HYDRO | |
| 371 | S371Y | HYDRO | |
| 373 | G373D | HYDRO | CHARGE |
| 373 | G373E | HYDRO | |
| 374 | K374A | HYDRO | CHARGE |
| 374 | K374G | HYDRO | CHARGE |
| 376 | S376E | HYDRO | |
| 376 | S376H | HYDRO | CHARGE |
| 376 | S376R | HYDRO | CHARGE |
| 377 | S377D | HYDRO | CHARGE |
| 377 | S377E | HYDRO | |
| 377 | S377F | HYDRO | |
| 377 | S377H | HYDRO | CHARGE |
| 377 | S377L | HYDRO | |
| 377 | S377R | HYDRO | CHARGE |
| 377 | S377V | HYDRO | |
| 377 | S377Y | HYDRO | |
| 378 | Y378E | HYDRO | |
| 380 | P380D | HYDRO | CHARGE |
| 380 | P380E | HYDRO | |
| 380 | P380G | HYDRO | |
| 380 | P380H | HYDRO | CHARGE |
| 380 | P380K | HYDRO | CHARGE |
| 380 | P380R | HYDRO | CHARGE |
| 380 | P380W | HYDRO | |
| 381 | I381Q | HYDRO | |
| 381 | I381T | HYDRO | |
| 384 | K384A | HYDRO | CHARGE |
| 384 | K384D | | CHARGE |
| 384 | K384G | HYDRO | CHARGE |
| 384 | K384I | HYDRO | CHARGE |
| 384 | K384N | HYDRO | CHARGE |
| 384 | K384S | HYDRO | CHARGE |
| 384 | K384T | HYDRO | CHARGE |
| 384 | K384V | HYDRO | CHARGE |
| 396 | T396D | HYDRO | CHARGE |
| 396 | T396E | HYDRO | |
| 396 | T396F | HYDRO | |
| 396 | T396H | HYDRO | CHARGE |
| 396 | T396I | HYDRO | |
| 396 | T396L | HYDRO | |
| 396 | T396M | HYDRO | |
| 396 | T396R | HYDRO | CHARGE |
| 396 | T396Y | HYDRO | |
| 402 | D402N | HYDRO | CHARGE |
| 403 | H403D | HYDRO | CHARGE |
| 404 | P404E | HYDRO | |
| 412 | E412M | HYDRO | |
| 412 | E412Q | HYDRO | |
| 415 | A415D | HYDRO | CHARGE |
| 416 | A416D | HYDRO | CHARGE |
| 416 | A416E | HYDRO | |
| 416 | A416F | HYDRO | |
| 416 | A416I | HYDRO | |
| 416 | A416K | HYDRO | CHARGE |
| 416 | A416L | HYDRO | |
| 416 | A416M | HYDRO | |
| 416 | A416V | HYDRO | |
| 418 | A418E | HYDRO | |
| 418 | A418H | HYDRO | CHARGE |
| 418 | A418K | HYDRO | CHARGE |
| 418 | A418L | HYDRO | |
| 418 | A418R | HYDRO | CHARGE |
| 418 | A418V | HYDRO | |
| 418 | A418Y | HYDRO | |
| 419 | G419D | HYDRO | CHARGE |
| 419 | G419E | HYDRO | |
| 419 | G419F | HYDRO | |
| 419 | G419K | HYDRO | CHARGE |
| 419 | G419M | HYDRO | |
| 419 | G419Y | HYDRO | |
| 430 | P430D | HYDRO | CHARGE |
| 430 | P430E | HYDRO | |
| 430 | P430G | HYDRO | |
| 430 | P430K | HYDRO | CHARGE |
| 431 | G431E | HYDRO | |
| 433 | S433H | HYDRO | CHARGE |
| 433 | S433R | HYDRO | CHARGE |
| 435 | W435H | HYDRO | CHARGE |
| 435 | W435K | HYDRO | CHARGE |
| 435 | W435Q | HYDRO | |
| 435 | W435R | HYDRO | CHARGE |
| 435 | W435S | HYDRO | |
| 435 | W435Y | HYDRO | |
| 440 | T440E | HYDRO | |
| 440 | T440H | HYDRO | CHARGE |
| 440 | T440I | HYDRO | |
| 441 | S441D | HYDRO | CHARGE |
| 441 | S441E | HYDRO | |
| 441 | S441H | HYDRO | CHARGE |
| 446 | V446A | HYDRO | |
| 446 | V446H | HYDRO | CHARGE |
| 452 | G452H | HYDRO | CHARGE |
| 454 | R454A | HYDRO | CHARGE |
| 454 | R454F | HYDRO | CHARGE |
| 454 | R454N | HYDRO | CHARGE |
| 454 | R454Q | HYDRO | CHARGE |
| 454 | R454S | HYDRO | CHARGE |
| 454 | R454V | HYDRO | CHARGE |
| 454 | R454Y | HYDRO | CHARGE |
| 455 | S455E | HYDRO | |
| 457 | T457E | HYDRO | |
| 457 | T457K | HYDRO | CHARGE |
| 459 | T459K | HYDRO | CHARGE |
| 459 | T459R | HYDRO | CHARGE |
| 461 | D461G | HYDRO | CHARGE |
| 461 | D461Q | HYDRO | CHARGE |
| 462 | A462D | HYDRO | CHARGE |
| 462 | A462E | HYDRO | |
| 462 | A462K | HYDRO | CHARGE |
| 462 | A462M | HYDRO | |
| 462 | A462V | HYDRO | |
| 463 | N463E | HYDRO | |
| 463 | N463H | HYDRO | CHARGE |
| 463 | N463R | HYDRO | CHARGE |
| 469 | W469A | HYDRO | |
| 469 | W469N | HYDRO | |
| 469 | W469Y | HYDRO | |
| 471 | N471D | HYDRO | CHARGE |
| 471 | N471E | HYDRO | |
| 471 | N471K | HYDRO | CHARGE |
| 472 | G472D | HYDRO | CHARGE |
| 472 | G472E | HYDRO | |
| 472 | G472H | HYDRO | CHARGE |
| 472 | G472K | HYDRO | CHARGE |
| 473 | G473D | HYDRO | CHARGE |
| 473 | G473E | HYDRO | |
| 473 | G473K | HYDRO | CHARGE |
| 473 | G473R | | CHARGE |
| 480 | K480E | | CHARGE |

The productive positions in PcuAmy1-v1 suitable for charge or hydrophobicity modifications are listed below. The position numbering is based on the mature PcuAmy1 polypeptide (SEQ ID NO: 3):
LIST E:
1, 4, 9, 10, 14, 15, 22, 25, 26, 29, 30, 32, 33, 34, 37, 41, 50, 67, 70, 73, 78, 79, 80, 81, 83, 84, 85, 87, 88, 90, 91, 92, 93, 95, 97, 102, 110, 111, 112, 113, 115, 117, 122, 125, 126, 127, 128, 130, 131, 132, 133, 135, 139, 140, 141, 143, 146, 147, 148, 149, 151, 152, 153, 155, 157, 166, 167, 168, 169, 170, 174, 176, 179, 180, 182, 189, 191, 192, 204, 205, 206, 207, 208, 210, 211, 212, 214, 215, 221, 222, 224, 225, 229, 234, 238, 241, 243, 247, 249, 250, 251, 252, 253, 254, 255, 265, 266, 268, 269, 273, 276, 279, 280, 281, 282, 283, 295, 298, 299, 300, 302, 303, 307, 310, 311, 314, 315, 316, 317, 319, 320, 333, 337, 357, 359, 361, 368, 371, 373, 374, 376, 377, 378, 380, 381, 384, 396, 402, 403, 404, 412, 415, 416, 418, 419, 430, 431, 433, 435, 440, 441, 446, 452, 454, 455, 457, 459, 461, 462, 463, 469, 471, 472, 473, and 480.

The variants of PcuAmy1-v1 suitable for charge or hydrophobicity modifications are listed below. The position numbering is based on the mature PcuAmy1 polypeptide (SEQ ID NO: 3):
LIST F:
G1D, G1E, G1H, G1I, G1M, G1R, G1V, G4D, G4E, G4F, G4H, G4I, G4L, G4P, G4V, G4W, G4Y, Y9A, Y9F, Y9G, Y9H, Y9S, Y9T, Y9W, F10M, L14A, L14D, L14E, L14N, P15R, N22D, N22E, N22H, N22M, N25E, N26Y, Q29D, Q29E, Q29F, Q29I, Q29M, Q29R, Q29V, Q29W, Q29Y, N30D, N30F, N30H, N30L, N30M, N30R, N30V, N30W, N30Y, K32D, K32G, K32S, K32Y, N33D, N33E, N33H, N33K, N33V, V34D, V34F, V34G, V34R, T37K, I41A, S50D, S50E, N67E, G70D, G70H, G70K, R73A, R73E, R73G, R73S, T78D, T78H, T78K, T78M, T78R, K79A, K79S, S80D, S80E, S80F, S80K, S80M, S80R, S80V, S80W, E81Q, I83A, I83D, I83G, I83Q, S84F, A85M, N87R, N88R, H90Q, A91I, A91K, A91M, K92G, K92Q, K92R, G93D, G93E, G93R, A95F, A95Y, Y97A, Y97F, Y97H, L102T, A110D, A110E, A110H, A110K, A110L, A110W, A110Y, T111D, T111F, E112M, L113A, L113C, L113D, L113E, L113G, L113N, L113P, L113Q, D115A, D115H, D115K, D115S, V117K, N122E, N122F, N122H, N122R, N122W, N125D, N125Y, V126A, V126E, V126N, V126Q, V126R, V126S, E127A, E127P, E127Q, E127V, T128I, T128M, T128R, S130D, S130F, S130H, S130I, S130K, S130L, S130R, S130V, S130W, T131E, T131F, T131H, T131I, T131M, T131R, T131W, T131Y, Y132E, Y132H, Y132Q, Q133D, Q133I, Q133M, Q133R, Q133V, Q135D, Q135E, Q135H, Q135L, Q135R, Q135V, Q135Y, Q139D, Q139K, Q139L, Q139R, Q139W, Y140F, D141G, D141I, P143E, P143F, P143G, P143H, P143I, G146H, G146K, G146M, G146V, G146W, G146Y, N147D, N147H, N147K, T148H, T148K, Y149F, S151D, S151E, S151F, S151H, S151K, S151R, S151V, F152H, K153R, R155A, R155C, R155E, R155F, R155G, R155N, R155Y, Y157D, Y157F, Y157Q, Y157S, Y157W, Q166D, Q166H, S167D, S167E, S167F, S167H, S167L, S167R, R168S, G169E, G169F, G169H, G169K, G169R, L170A, L170D, L170E, L170G, L170H, L170K, L170Q, L170R, L170S, Y174W, L176H, L176N, D179F, D179H, D179K, D179L, D179M, D179N, D179S, D179T, D179V, D179W, D179Y, D180T, D182A, D182F, D182H, D182I, D182K, D182M, D182R, D182S, D182T, D182W, D182Y, S189D, S189E, Y191H, Y191W, G192H, G192M, F204M, N205D, N205E, N205F, H206A, H206C, H206D, H206E, H206M, H206W, H206Y, P207E, P207G, D208F, D208G, D208L, D208N, D208Q, D208R, D208S, V210K, V210Q, V210R, N211H, N211M, N211R, N211W, N211Y, E212T, K214G, K214M, K214Q, K214S, T215D, T215E, T215F, T215L, T215M, T215Y, V221A, N222D, V224G, V224S, N225D, N225E, V229W, V234A, K238Q, F241R, F241S, R243A, R243E, R243G, R243L, R243Q, R243W, N247E, N247M, N247R, N247V, V249E, V249G, V249N, R250A, R250E, R250G, R250N, R250T, S251E, T252F, T252H, T252K, T252M, T253K, T253L, T253R, G254E, G254I, G254V, K255A, K255D, K255G, K255I, K255L, K255N, K255V, K255W, H265A, H265D, H265G, Y266E, Y266N, Y266Q, Y266S, Y266T, Y266W, V268D, V268E, V268F, V268N, N269D, N269E, N269H, N269I, N269K, S273D, T276D, T276E, T276L, T276Y, N279E, G280K, G280M, G280R, T281K, M282E, M282H, S283E, S283H, D295H, D295K, D295N, N298E, N298H, G299K, G300K, G300Y, G302D, G302E, G302H, G302M, G302R, Y303F, N307D, N307H, N307K, N307L, N307M, N307R, N307W, N310E, N310K, N310R, N310V, N311D, N311E, M314S, S315E, S315H, S315I, S315V, S316D, S316E, S316H, S316I, S316L, S316V, S316W, S316Y, N317K, N317V, M319A, M319D, M319E, M319G, M319K, M319Q, M319R, M319S, M319T, K320E, K320F, K320L, T333D, Q337D, Q337E, Q357E, Y359A, Y359F, Y359H, Y359N, Y359R, Y359T, C361I, Y368F, Y368K, Y368S, S371E, S371Y, G373D, G373E, K374A, K374G, S376E, S376H, S376R, S377D, S377E, S377F, S377H, S377L, S377R, S377V, S377Y, Y378E, P380D, P380E, P380G, P380H, P380K, P380R, P380W, I381Q, I381T, K384A, K384D, K384G, K384I, K384N, K384S, K384T, K384V, T396D, T396E, T396F, T396H, T396I, T396L, T396M, T396R, T396Y, D402N, H403D, P404E, E412M, E412Q, A415D, A416D, A416E, A416F, A416I, A416K, A416L, A416M, A416V, A418E, A418H, A418K, A418L, A418R, A418V, A418Y, G419D, G419E, G419F, G419K, G419M, G419Y, P430D, P430E, P430G, P430K, G431E, S433H, S433R, W435H, W435K, W435Q, W435R, W435S, W435Y, T440E, T440H, T440I, S441D, S441E, S441H, V446A, V446H, G452H, R454A, R454F, R454N, R454Q, R454S, R454V, R454Y, S455E, T457E, T457K, T459K, T459R, D461G, D461Q, A462D, A462E, A462K, A462M, A462V, N463E, N463H, N463R, W469A, W469N, W469Y, N471D, N471E, N471K, G472D, G472E, G472H, G472K, G473D, G473E, G473K, G473R, K480E, K480S, and K480Y.

## Claims

1. A method of preparing a variant α-amylase polypeptide from a parental α-amylase polypeptide, comprising introducing at least one combinable mutation into said parental α-amylase polypeptide at a productive amino acid position; wherein:
(a) the combinable mutation produces a variant amylase wherein the minimum performance indices (PI) relative to the parental amylase for:
(i) protein expression,
(ii) activity, and
(iii) detergent stability or thermostability are either
(aa) greater than or equal to 0.9, and the PI for any one of (i), (ii), or (iii) is greater than 1.0,
(ab) greater than or equal to 0.8, and the PI for any one of (i), (ii), or (iii) is greater than or equal to 1.2, or
(ac) greater than or equal to 0.5, and the PI for any one of (i), (ii), or (iii) is greater than or equal to 1.5;
wherein protein expression, activity, detergent stability and thermostability are each determined using an assay described in Example 1;
(b) the productive position is an amino acid position that can be substituted with a plurality of different amino acid residues, each of which substitutions result in a variant α-amylase that meets the requirements of (a),
(c) the productive position is T333, using SEQ ID NO: 3 for numbering, and
(d) the combinable mutation is T333F, T333Q, T333Y, T333N, T333S, T333A, T333D, T333E or T333G,
wherein the parental α-amylase and the variant α-amylase have at least 80% amino acid sequence identity to the amino acid sequence of SEQ ID NO: 3 or SEQ ID NO: 4.

2. The method of claim 1, comprising introducing a plurality of combinable mutations into said parental α-amylase, wherein each said combinable substitution is at a productive position selected from 472, 280, 191, 1, 2, 3, 4, 5, 6, 9, 10, 14, 15, 16, 17, 19, 20, 22, 23, 24, 25, 26, 27, 28, 29, 30, 31, 32, 33, 34, 35, 36, 37, 38, 39, 41, 44, 45, 47, 48, 49, 50, 51, 53, 57, 60, 63, 65, 67, 68, 69, 70, 71, 72, 73, 74, 76, 78, 79, 80, 81, 82, 83, 84, 85, 86, 87, 88, 89, 90, 91, 92, 93, 94, 95, 96, 97, 98, 100, 101, 102, 105, 107, 110, 111, 112, 113, 114, 115, 116, 117, 120, 121, 122, 125, 126, 127, 128, 129, 130, 131, 132, 133, 134, 135, 136, 137, 138, 139, 140, 141, 142, 143, 144, 146, 147, 148, 149, 150, 151, 152, 153, 155, 156, 157, 162, 164, 166, 167, 168, 169, 170, 171, 172, 173, 174, 175, 176, 179, 180, 181, 182, 189, 190, 192, 200, 202, 203, 204, 205, 206, 207, 208, 210, 211, 212, 213, 214, 215, 217, 218, 220, 221, 222, 223, 224, 225, 226, 227, 229, 231, 234, 237, 238, 239, 241, 242, 243, 244, 246, 247, 248, 249, 250, 251, 252, 253, 254, 255, 256, 257, 258, 259, 260, 261, 264, 265, 266, 267, 268, 269, 270, 271, 272, 273, 274, 275, 276, 278, 279, 281, 282, 283, 285, 287, 289, 291, 292, 293, 294, 295, 297, 298, 299, 300, 302, 303, 306, 307, 309, 310, 311, 313, 314, 315, 316, 317, 318, 319, 320, 322, 331, 337, 338, 339, 340, 341, 342, 345, 347, 348, 356, 357, 359, 361, 362, 364, 367, 368, 369, 370, 371, 373, 374, 376, 377, 378, 379, 380, 381, 382, 383, 384, 385, 386, 387, 388, 390, 391, 392, 396, 398, 401, 402, 403, 404, 405, 406, 407, 412, 414, 415, 416, 418, 419, 420, 425, 426, 427, 430, 431, 432, 433, 435, 440, 441, 442, 443, 444, 446, 448, 450, 451, 452, 453, 454, 455, 456, 457, 459, 461, 462, 463, 465, 467, 469, 471, 473, 474, 475, 477, and 480, using SEQ ID NO: 3 for numbering.

3. The method of claim 1 or claim 2, further comprising introducing a deletion corresponding to a residue selected from the group consisting of Arg-177, Gly-178, Asp-179, and Gly-180, using SEQ ID NO: 3 for numbering.

4. The method of claim 3, comprising introducing deletions corresponding to residues Arg-177 and Gly-178, using SEQ ID NO: 3 for numbering.

5. The method of any of the preceding claims, wherein the parental α-amylase and the variant α-amylase have at least 90% or at least 95% amino acid sequence identity to the amino acid sequence of SEQ ID NO: 3 or SEQ ID NO: 4.

6. A method according to any of the preceding claims, comprising formulating the variant α-amylase into a composition.

7. The method of claim 6, wherein the composition further comprises a surfactant.

8. The method of claim 6 or claim 7, wherein the composition is a detergent composition, a composition for liquefying starch, a composition for saccharifying a composition comprising starch, a composition for SSF post liquefaction, a composition for direct SSF without prior liquefaction, a composition for producing a fermented beverage, a composition for producing a baked food product, a composition for textile desizing, or is a composition effective for removing starchy stains from laundry, dishes, or textiles.

9. The method of any of claims 6 to 8, wherein the composition is selected from the group consisting of a laundry detergent, a laundry detergent additive, a manual or automatic dishwashing detergent, or a dishwashing machine cleaning composition.

10. The method of any of claims 6 to 9, wherein the composition further comprises one or more additional enzymes selected from the group consisting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase and laccase.

## Patentansprüche

1. Verfahren zum Herstellen eines varianten α-Amylase-Polypeptids aus einem elterlichen α-Amylase-Polypeptid, umfassend Einführen mindestens einer kombinierbaren Mutation in das elterliche α-Amylase-Polypeptid an einer produktiven Aminosäureposition; wobei:
(a) die kombinierbare Mutation eine variante Amylase erzeugt, bei der die minimalen Leistungsindizes (PI) relativ zur elterlichen Amylase für:
(i) Proteinexpression,
(ii) Aktivität und
(iii) Detergensstabilität und Thermostabilität entweder
(aa) größer als oder gleich 0,9 sind und der PI für jede der (i), (ii) oder (iii) größer als 1,0 ist,
(ab) größer als oder gleich 0,8 sind und der PI für jede der (i), (ii) oder (iii) größer als oder gleich 1,2 ist, oder
(ac) größer als oder gleich 0,5 sind und der PI für jede der (i), (ii) oder (iii) größer als oder gleich 1,5 ist;
wobei Proteinexpression, Aktivität, Detergensstabilität und Thermostabilität jeweils unter Verwendung eines in Beispiel 1 beschriebenen Assays bestimmt werden;
(b) die produktive Position eine Aminosäureposition ist, die mit einer Vielzahl von unterschiedlichen Aminosäureresten substituiert sein kann, von denen jede Substitution in einer varianten α-Amylase resultiert, die den Anforderungen von (a) genügt,
(c) die produktive Position T333 ist, indem SEQ ID NO: 3 zur Nummerierung verwendet wird, und
(d) die kombinierbare Mutation T333F, T333Q, T333Y, T333N, T333S, T333A, T333D, T333E oder T333G ist,
wobei die elterliche α-Amylase und die variante α-Amylase über mindestens 80% Aminsosäuresequenz-Identität zu der SEQ ID NO: 3 oder SEQ ID NO: 4 verfügen.

2. Verfahren nach Anspruch 1, umfassend das Einführen einer Vielzahl kombinierbarer Mutationen in die elterliche α-Amylase, wobei sich jede der kombinierbaren Substitutionen an einer produktiven Position befindet, ausgewählt aus: 472, 280, 191, 1, 2, 3, 4, 5, 6, 9, 10, 14, 15, 16, 17, 19, 20, 22, 23, 24, 25, 26, 27, 28, 29, 30, 31, 32, 33, 34, 35, 36, 37, 38, 39, 41, 44, 45, 47, 48, 49, 50, 51, 53, 57, 60, 63, 65, 67, 68, 69, 70, 71, 72, 73, 74, 76, 78, 79, 80, 81, 82, 83, 84, 85, 86, 87, 88, 89, 90, 91, 92, 93, 94, 95, 96, 97, 98, 100, 101, 102, 105, 107, 110, 111, 112, 113, 114, 115, 116, 117, 120, 121, 122, 125, 126, 127, 128, 129, 130, 131, 132, 133, 134, 135, 136, 137, 138, 139, 140, 141, 142, 143, 144, 146, 147, 148, 149, 150, 151, 152, 153, 155, 156, 157, 162, 164, 166, 167, 168, 169, 170, 171, 172, 173, 174, 175, 176, 179, 180, 181, 182, 189, 190, 192, 200, 202, 203, 204, 205, 206, 207, 208, 210, 211, 212, 213, 214, 215, 217, 218, 220, 221, 222, 223, 224, 225, 226, 227, 229, 231, 234, 237, 238, 239, 241, 242, 243, 244, 246, 247, 248, 249, 250, 251, 252, 253, 254, 255, 256, 257, 258, 259, 260, 261, 264, 265, 266, 267, 268, 269, 270, 271, 272, 273, 274, 275, 276, 278, 279, 281, 282, 283, 285, 287, 289, 291, 292, 293, 294, 295, 297, 298, 299, 300, 302, 303, 306, 307, 309, 310, 311, 313, 314, 315, 316, 317, 318, 319, 320, 322, 331, 337, 338, 339, 340, 341, 342, 345, 347, 348, 356, 357, 359, 361, 362, 364, 367, 368, 369, 370, 371, 373, 374, 376, 377, 378, 379, 380, 381, 382, 383, 384, 385, 386, 387, 388, 390, 391, 392, 396, 398, 401, 402, 403, 404, 405, 406, 407, 412, 414, 415, 416, 418, 419, 420, 425, 426, 427, 430, 431, 432, 433, 435, 440, 441, 442, 443, 444, 446, 448, 450, 451, 452, 453, 454, 455, 456, 457, 459, 461, 462, 463, 465, 467, 469, 471, 473, 474, 475, 477, und 480, indem SEQ ID NO: 3 zur Nummerierung verwendet wird.

3. Verfahren nach einem der Ansprüche 1 oder 2, ferner umfassend Einführen einer Deletion, die mit einem Rest korrespondiert, der ausgewählt ist aus der Gruppe bestehend aus Arg-177, Gly-178, Asp-179 und Gly-180, indem SEQ ID NO: 3 zur Nummerierung verwendet wird.

4. Verfahren nach Anspruch 3, ferner umfassend Einführen von Deletionen, die mit den Resten Arg-177 und Gly-178 korrespondieren, indem SEQ ID NO: 3 zur Nummerierung verwendet wird.

5. Verfahren nach einem der vorgenannten Ansprüche, wobei die elterliche α-Amylase und die die variante α-Amylase über mindestens 90%, vorzugsweise mindestens 95% Aminsosäuresequenz-Identität zu der Aminsosäuresequenz von SEQ ID NO: 3 oder SEQ ID NO: 4 verfügen.

6. Verfahren nach einem der vorgenannten Ansprüche, umfassend Formulieren der varianten α-Amylase zu einer Zusammensetzung.

7. Verfahren nach Anspruch 6, wobei die Zusammensetzung ferner ein Tensid umfasst.

8. Verfahren nach Anspruch 6 oder Anspruch 7, wobei die Zusammensetzung eine Detergens-Zusammensetzung, eine Zusammensetzung zum Verflüssigen von Stärke, eine Zusammensetzung zum Verzuckern einer Stärke umfassenden Zusammensetzung, eine Zusammensetzung für SSF-Nachverflüssigung, eine Zusammensetzung zur direkten SSF ohne vorherige Verflüssigung, eine Zusammensetzung zum Herstellen eines fermentierten Getränks, eine Zusammensetzung zum Herstellen eines gebackenen Nahrungsmittelprodukts, eine Zusammensetzung zum Entschlichten eines Textils oder eine Zusammensetzung ist, die zum Entfernen von stärkehaltigen Flecken aus Wäsche, Geschirr oder Textilien wirksam ist.

9. Verfahren nach einem der Ansprüche 6 bis 8, wobei die Zusammensetzung ausgewählt ist aus der Gruppe bestehend aus einem Waschmittel, einem Waschmittelzusatz, einem Spülmittel zum manuellen oder automatischen Geschirrspülen oder einer Reinigungszusammensetzung für Geschirrspülmaschinen.

10. Verfahren nach einem der Ansprüche 6 bis 8, wobei die Zusammensetzung ferner umfasst: ein oder mehrere zusätzliche Enzyme, die ausgewählt sind aus der Gruppe bestehend aus Protease, Hemicellulase, Cellulase, Peroxidase, lipolytischem Enzym, metallolipolytischem Enzym, Xylanase, Lipase, Phospholipase, Esterase, Perhydrolase, Cutinase, Pektinase, Pectatlyase, Mannanase, Keratinase, Reduktase, Oxidase, Phenoloxidase, Lipoxygenase, Ligninase, Pullulanase, Tannase, Pentosanase, Malanase, β-Glucanase, Arabinosidase, Hyaluronidase, Chondroitinase und Laccase.

## Revendications

1. Procédé pour la préparation d'un polypeptide variant d'a-amylase à partir d'un polypeptide d'a-amylase parent, comprenant l'introduction d'au moins une mutation combinable dans ledit polypeptide d'a-amylase parent à une position d'acide aminé productive; dans lequel:
(a) la mutation combinable produit une amylase variante dans laquelle les indices de performance (PI) minimums relativement à l'amylase parente pour:
(i) l'expression d'une protéine,
(ii) l'activité, et
(iii) la stabilité à un détergent ou la thermostabilité sont:
(aa) soit supérieurs ou égaux à 0,9 et le PI pour l'un quelconque de (i), (ii) ou (iii) est supérieur à 1,0,
(ab) soit supérieurs ou égaux à 0,8 et le PI pour l'un quelconque de (i), (ii) ou (iii) est supérieur ou égal à 1,2, ou
(ac) soit supérieurs ou égaux à 0,5 et le PI pour l'un quelconque de (i), (ii) ou (iii) est supérieur ou égal à 1,5;
dans laquelle l'expression d'une protéine, l'activité, la stabilité à un détergent et la thermostabilité sont chacune déterminées en utilisant un essai décrit dans l'Exemple 1;
(b) la position productive est une position d'acide aminé qui peut être substituée avec une pluralité de différents résidus d'acides aminés, dont chaque substitution conduit à une α-amylase variante qui satisfait les exigences de (a),
(c) la position productive est T333, en utilisant la SEQ ID NO: 3 pour la numérotation, et
(d) la mutation combinable est T333F, T333Q, T333Y, T333N, T333S, T333A, T333D, T333E ou T333G,
dans laquelle l'a-amylase parente et l'a-amylase variante ont au moins 80% d'identité de séquence d'acides aminés avec la séquence d'acides aminés de la SEQ ID NO: 3 ou la SEQ ID NO: 4.

2. Procédé selon la revendication 1, comprenant l'introduction d'une pluralité de mutations combinables dans ladite α-amylase parente, dans lequel chacune desdites substitutions combinables est à une position productive choisie parmi 472, 280, 191, 1, 2, 3, 4, 5, 6, 9, 10, 14, 15, 16, 17, 19, 20, 22, 23, 24, 25, 26, 27, 28, 29, 30, 31, 32, 33, 34, 35, 36, 37, 38, 39, 41, 44, 45, 47, 48, 49, 50, 51, 53, 57, 60, 63, 65, 67, 68, 69, 70, 71, 72, 73, 74, 76, 78, 79, 80, 81, 82, 83, 84, 85, 86, 87, 88, 89, 90, 91, 92, 93, 94, 95, 96, 97, 98, 100, 101, 102, 105, 107, 110, 111, 112, 113, 114, 115, 116, 117, 120, 121, 122, 125, 126, 127, 128, 129, 130, 131, 132, 133, 134, 135, 136, 137, 138, 139, 140, 141, 142, 143, 144, 146, 147, 148, 149, 150, 151, 152, 153, 155, 156, 157, 162, 164, 166, 167, 168, 169, 170, 171, 172, 173, 174, 175, 176, 179, 180, 181, 182, 189, 190, 192, 200, 202, 203, 204, 205, 206, 207, 208, 210, 211, 212, 213, 214, 215, 217, 218, 220, 221, 222, 223, 224, 225, 226, 227, 229, 231, 234, 237, 238, 239, 241, 242, 243, 244, 246, 247, 248, 249, 250, 251, 252, 253, 254, 255, 256, 257, 258, 259, 260, 261, 264, 265, 266, 267, 268, 269, 270, 271, 272, 273, 274, 275, 276, 278, 279, 281, 282, 283, 285, 287, 289, 291, 292, 293, 294, 295, 297, 298, 299, 300, 302, 303, 306, 307, 309, 310, 311, 313, 314, 315, 316, 317, 318, 319, 320, 322, 331, 337, 338, 339, 340, 341, 342, 345, 347, 348, 356, 357, 359, 361, 362, 364, 367, 368, 369, 370, 371, 373, 374, 376, 377, 378, 379, 380, 381, 382, 383, 384, 385, 386, 387, 388, 390, 391, 392, 396, 398, 401, 402, 403, 404, 405, 406, 407, 412, 414, 415, 416, 418, 419, 420, 425, 426, 427, 430, 431, 432, 433, 435, 440, 441, 442, 443, 444, 446, 448, 450, 451, 452, 453, 454, 455, 456, 457, 459, 461, 462, 463, 465, 467, 469, 471, 473, 474, 475, 477 et 480, en utilisant la SEQ ID NO: 3 pour la numérotation.

3. Procédé selon l'une quelconque de la revendication 1 ou de la revendication 2, comprenant en outre l'introduction d'une délétion correspondant à un résidu choisi dans le groupe constitué de Arg-177, Gly-178, Asp-179 et Gly-180, en utilisant la SEQ ID NO: 3 pour la numérotation.

4. Procédé selon la revendication 3, comprenant l'introduction de délétions correspondant aux résidus Arg-177 et Gly-178, en utilisant la SEQ ID NO: 3 pour la numérotation.

5. Procédé selon l'une quelconque des revendications précédentes, dans lequel l'a-amylase parente et l'a-amylase variante ont au moins 90%, de préférence au moins 95%, d'identité de séquence d'acides aminés avec la séquence d'acides aminés de la SEQ ID NO: 3 ou la SEQ ID NO: 4.

6. Procédé selon l'une quelconque des revendications précédentes, comprenant la formulation de l'a-amylase variante en une composition.

7. Procédé selon la revendication 6, dans lequel la composition comprend en outre un agent tensioactif.

8. Procédé selon la revendication 6 ou la revendication 7, dans lequel la composition est une composition de détergent, une composition pour la liquéfaction d'amidon, une composition pour la saccharification d'une composition comprenant de l'amidon, une composition pour une SFF après liquéfaction, une composition pour une SSF directe sans liquéfaction préalable, une composition pour la production d'une boisson fermentée, une composition pour la production d'un produit alimentaire cuit au four, une composition pour le désencollage d'un textile ou est une composition efficace pour le retrait de taches d'amidon provenant de blanchisserie, de vaisselle ou de textiles.

9. Procédé selon l'une quelconque des revendications 6 à 8, dans laquelle la composition est choisie dans le groupe constitué d'un détergent pour blanchisserie, d'un additif de détergent pour blanchisserie, d'un détergent pour lavage de vaisselle manuel ou automatique ou d'une composition de nettoyage de lave-vaisselle.

10. Procédé selon l'une quelconque des revendications 6 à 8, dans laquelle la composition comprend en outre une ou plusieurs enzymes supplémentaires choisies dans le groupe constitué de protéase, hémicellulase, cellulase, peroxydase, enzyme lipolytique, enzyme métallolipolytique, xylanase, lipase, phospholipase, estérase, perhydrolase, cutinase, pectinase, pectate lyase, mannanase, kératinase, réductase, oxydase, phénoloxydase, lipoxygénase, ligninase, pullulanase, tannase, pentosanase, malanase, β-glucanase, arabinosidase, hyaluronidase, chondroïtinase et laccase.
